# EUROPEAN PATENT SPECIFICATION

(11) **EP 1 026 239 B1**
(45) Date of publication and mention of the grant of the patent: **02.03.2011**
(21) Application number: 00103589.8
(22) Date of filing: 16.05.1990
(51) Int. Cl.: C12N 15/10, C07K 16/00

(54) **A new method for tapping the immunological repertoire**
Neues Verfahren zur Erschliessung des immunologischen Repertoirs
Nouveau procédé d'exploitation du répertoire immunologique

(30) Priority: 16.05.1989 US 353235; 16.05.1989 US 352927; 17.05.1989 US 353241; 17.05.1989 US 352884; 04.12.1989 US 446332; 20.03.1990 US 496522
(43) Date of publication of application: 09.08.2000
(62) Divisional of application: 90909224.9
(73) Proprietor: THE SCRIPPS RESEARCH INSTITUTE, La Jolla, CA 92037 (US); STRATAGENE, La Jolla California 92037 (US); Medical Research Council, 20 Park Crescent London W1B 1AL (GB)
(72) Inventor: Lerner, Richard A., La Jolla, CA 92037 (US); Sorge, Joseph A., La Jolla, CA 92037 (US)
(74) Representative: Brasnett, Adrian Hugh

(56) References cited:
- WO-A-88/06630
- WO-A-88/09344
- WO-A-89/01526
- US-A- 4 642 334
- US-A- 4 800 159
- ORLANDI R ET AL: "CLONING IMMUNOGLOBULIN VARIABLE DOMAINS FOR EXPRESSION BY THE POLYMERASE CHAIN REACTION" PROCEEDINGS OF THE NATIONAL ACADEMY OF SCIENCES OF USA, NATIONAL ACADEMY OF SCIENCE. WASHINGTON, US, vol. 86, no. 10, 1 May 1989 (1989-05-01), pages 3833-3837, XP000026475 ISSN: 0027-8424
- LARRICK J W ET AL: "RAPID CLONING OF REARRANGED IMMUNOGLOBULIN GENES FROM HUMAN HYBRIDOMA CELLS USING MIXED PRIMERS AND THE POLYMERASE CHAIN REACTION" BIOCHEMICAL AND BIOPHYSICAL RESEARCH COMMUNICATIONS, ACADEMIC PRESS INC. ORLANDO, FL, US, vol. 160, no. 3, 15 May 1989 (1989-05-15), pages 1250-1256, XP000293053 ISSN: 0006-291X
- SHORT J M ET AL: "LAMBDA ZAP: A BACTERIOPHAGE LAMBDA EXPRESSION VECTOR WITH IN VIVO EXCISION PROPERTIES" NUCLEIC ACIDS RESEARCH, OXFORD UNIVERSITY PRESS, SURREY, GB, vol. 16, no. 15, 1988, pages 7583-7600, XP002007597 ISSN: 0305-1048
- JANDA ET AL.: "Induction of an antibody that catalyzes the hydrolysis of an amide bond" SCIENCE, vol. 241, 2 September 1988 (1988-09-02), pages 1188-1191, XP008026015
- POLLACK ET AL.: "Selective chemical analysis by an antibody" SCIENCE, vol. 234, 19 December 1986 (1986-12-19), pages 1570-1573, XP008026014
- HUSE W D ET AL: "GENERATION OF A LARGE COMBINATORIAL LIBRARY OF THE IMMUNOGLOBULIN REPERTOIRE IN PHAGE LAMBDA" SCIENCE, AMERICAN ASSOCIATION FOR THE ADVANCEMENT OF SCIENCE,, US, vol. 246, 8 December 1989 (1989-12-08), pages 1275-1281, XP000083689 ISSN: 0036-8075
- SASTRY ET AL.: "Cloning of the immunological repertoire in Escherichia coli for generation of monoclonal catalytic antibodies: Construction of a heavy chain variable region-specific cDNA library" PROCEEDINGS OF THE NATIONAL ACADEMY OF SCIENCES, vol. 86, August 1989 (1989-08), pages 5728-5732, XP008026026 us

## Description

### Technical Field

The present invention relates to a method for isolating a gene coding for a receptor having a preselected activity.

### Background

Binding phenomena between ligands and receptors play many crucial roles in biological systems. Exemplary of such phenomena are the binding of oxygen molecules to deoxyhemoglobin to form oxyhemoglobin, and the binding of a substrate to an enzyme that acts upon it such as between a protein and a protease like trypsin. Still further examples of biological binding phenomena include the binding of an antigen to an antibody, and the binding of complement component C3 to the so-called CR1 receptor.

Many drugs and other therapeutic agents are also believed to be dependent upon binding phenomena. For example, opiates such as morphine re reported to bind to specific receptors in the brain. Opiate agonists and antagonists are reported to compete with drugs like morphine for those binding sites.

Ligands such as man-made drugs, like morphine and its derivatives, and those that are naturally present in biological systems such as endorphins and hormones bind to receptors that are naturally present in biological systems, and will be treated together herein. Such binding can lead to a number of the phenomena of biology, including particularly the hydrolysis of amide and ester bonds as where proteins are hydrolyzed into constituent polypeptides by an enzyme such as trypsin or papain or where a fat is cleaved into glycerine and three carboxylic acids, respectively. In addition, such binding can lead to formation of amide and ester bonds in the formation of proteins and fats, as well as to the formation of carbon to carbon bonds and carbon to nitrogen bonds.

An exemplary receptor-producing system in vertebrates is the immune system. The immune system of a mammal is one of the most versatile biological systems as probably greater than 1.0 x 10⁷ receptor specificities, in the form of antibodies, can be produced. Indeed, much of contemporary biological and medical research is directed toward tapping this repertoire. During the last decade there has been a dramatic increase in the ability to harness the output of the vast immunological repertoire. The development of the hybridoma methodology by Kohler and Milstein has made it possible to produce monoclonal antibodies, i.e., a composition of antibody molecules of a single specificity, from the repertoire of antibodies induced during an immune response.

Unfortunately, current methods for generating monoclonal antibodies are not capable of efficiently surveying the entire antibody response induced by a particular immunogen. In an individual animal there are at least 5-10,000 different B-cell clones capable of generating unique antibodies to a small relatively rigid immunogens, such as, for example dinitrophenol. Further, because of the process of somatic mutation during the generation of antibody diversity, essentially an unlimited number of unique antibody molecules may be generated. In contrast to this vast potential for different antibodies, current hybridoma methodologies typically yield only a few hundred different monoclonal antibodies per fusion.

Other difficulties in producing monoclonal antibodies with the hybridoma methodology include genetic instability and low production capacity of hybridoma cultures. One means by which the art has attempted to overcome these latter two problems has been to clone the immunoglobulin-producing genes from a particular hybridoma of interest into a prokaryotic expression system. See, for example, Robinson et al., PCT Publication No. WO 89/0099; Winter et al., European Patent Publication No. 0239400; Reading, U.S. Patent No. 4,714,681; and Cabilly et al., European Patent Publication No. 0125023.

The immunologic repertoire of vertebrates has recently been found to contain genes coding for immunoglobulins having catalytic activity. Tramontano et al., Sci., 234:1566-1570 (1986); Pollack et al., Sci., 234:1570-1573 (1986); Janda et al., Sci., 241:1188-1191 (1988) ; and Janda et al., Sci., 244:437-440 (1989). The presence of, or the ability to induce the repertoire to produce, antibodies molecules capable of a catalyzing chemical reaction, i.e., acting like enzymes, had previously been postulated almost 20 years ago by W. P. Jencks in Catalysis in Chemistry and Enzymology, McGraw-Hill, N.Y. (1969).

It is believed that one reason the art failed to isolate catalytic antibodies from the immunological repertoire earlier, and its failure to isolate many to date even after their actual discovery, is the inability to screen a large portion of the repertoire for the desired activity. Another reason is believed to be the bias of currently available screening techniques, such as the hybridoma technique, towards the production high affinity antibodies inherently designed for participation in the process of neutralization, as opposed to catalysis.

### Brief Summary of the Invention

The present invention provides a novel method for screening a larger portion of a conserved receptor coding gene repertoire for receptors having a preselected activity than has heretofore been possible, thereby overcoming the before-mentioned inadequacies of the hybridoma technique.

In one embodiment, a conserved receptor-coding gene library containing a substantial portion of the conserved receptor-coding gene repertoire is synthesized. In preferred embodiments, the conserved receptor-coding gene library contains at least about 10³, preferably at least about 10⁴ and more preferably at least about 10⁵ different receptor-coding genes.

The gene library can be synthesized by either of two methods, depending on the starting material.

Where the starting material is a plurality of receptor-coding genes, the repertoire is subjected to two distinct primer extension reactions. The first primer extension reaction uses a first polynucleotide synthesis primer capable of initiating the first reaction by hybridizing to a nucleotide sequence conserved (shared by a plurality of genes) within the repertoire. The first primer extension produces of different conserved receptor-coding homolog compliments (nucleic acid strands complementary to the genes in the repertoire).

The second primer extension reaction produces, using the complements as templates, a plurality of different conserved receptor-coding DNA homologs. The second primer extension reaction uses a second polynucleotide synthesis primer that is capable of initiating the second reaction by hybridizing to a nucleotide sequence conserved among a plurality of the compliments.

Where the starting material is a plurality of compliments of conserved receptor-coding genes, the repertoire is subjected to the above-discussed second primer extension reaction. Of course, if both a repertoire of conserved receptor-coding genes and their complements are present, both approaches can be used in combination.

A conserved receptor-coding DNA homolog, i.e., a gene coding for a receptor capable of binding the preselected ligand, is then segregated from the library to produce the isolated gene. This is typically accomplished by operatively linking for expression a plurality of the different conserved receptor-coding DNA homologs of the library to an expression vector. The receptor-expression vectors so produces are introduced into a population of compatible host cells, i.e., cells capable of expressing a gene operatively linked for expression to the vector. The transformants are cultured under conditions for expressing the receptor corded for by the receptor-coding DNA homolog. The transformants are cloned and the clones are screened for expression of a receptor that binds the preselected ligand. Any of the suitable methods well known in the art for detecting the binding of a ligand to a receptor can be used. A transformant expressing the desired activity is then segregated from the population to produce the isolated gene.

In another embodiment, the present invention contemplates a gene library comprising an isolated admixture of at least about 10³, preferably at least about 10⁴ and more preferably at least 10⁵ conserved receptor-coding DNA homologs, a plurality of which share a conserved antigenic determinant. Preferably, the homologs are present in a medium suitable for in vitro manipulation, such as water, phosphate buffered saline and the like, which maintains the biological activity of the homologs.

A receptor having a preselected activity, preferably catalytic activity, produced by a method of the present invention, preferably a monomer or dimer as described herein, is also contemplated.

### Brief Description of the Drawings

In the drawings forming a portion of this disclosure:

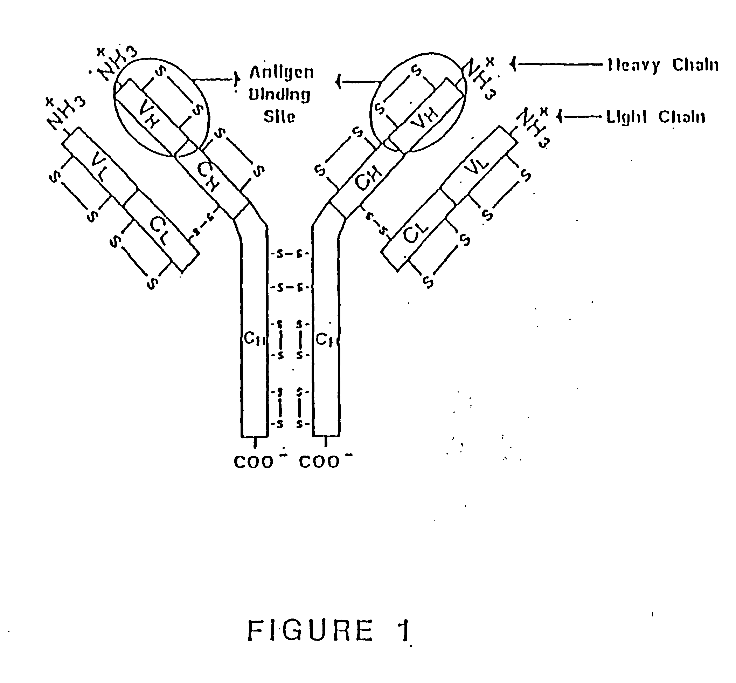

Figure 1 Illustrates a schematic diagram of the immunoglobulin molecule showing the principal structural features. The circled area on the heavy chain represents the variable region (V_{H}), a polypeptide containing a biologically active (ligand binding) portion of that region, and a gene coding for that polypeptide, are produced by the methods of the present invention. Sequences L03, L35, L47 and L48 could not be classified into any predefined subgroups.

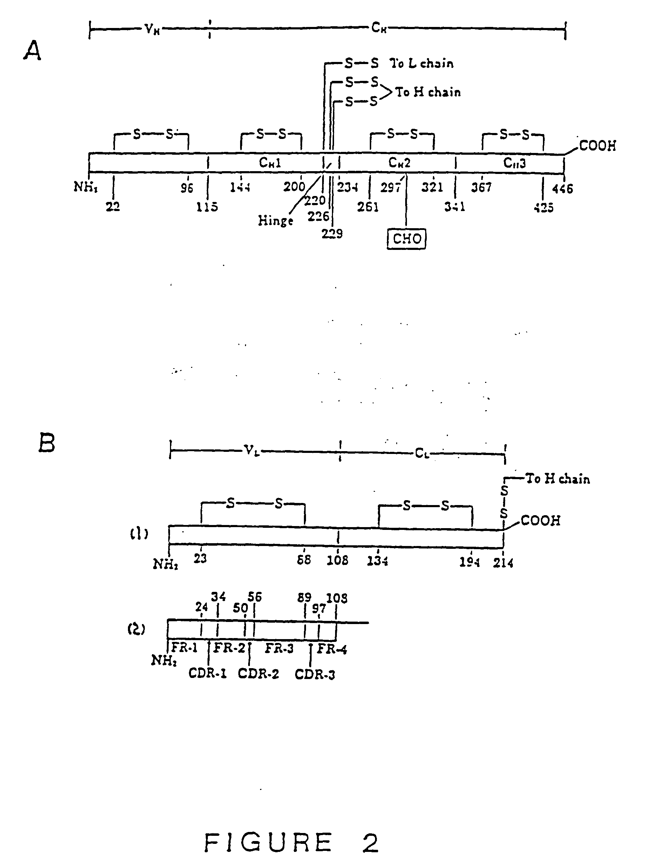

Figure 2A Diagrammatic sketch of an H chain of human IgG (IgGl subclass). Numbering is from the N-terminus on the left to the C-terminus on the right. Note the presence of four domains, each containing an intrachain disulfide bond (S-S) spanning approximately 60 amino acid residues. The symbol CHO stands for carbohydrate. The V region of the heavy (H) chain (V_{H}) resembles V_{L} in having three hypervariable CDR (not shown).
Figure 2B Diagrammatic sketch of a human K chain (Panel 1). Numbering is from the N-terminus on the left to the C-terminus on the right. Note the intrachain disulfide bond (S-S) spanning about the same number of amino acid residues in the V_{L} and C_{L} domains. Panel 2 shows the locations of the complementarity-determining regions (CDR) in the V_{L} domain. Segments outside the CDR are the framework segments (FR).

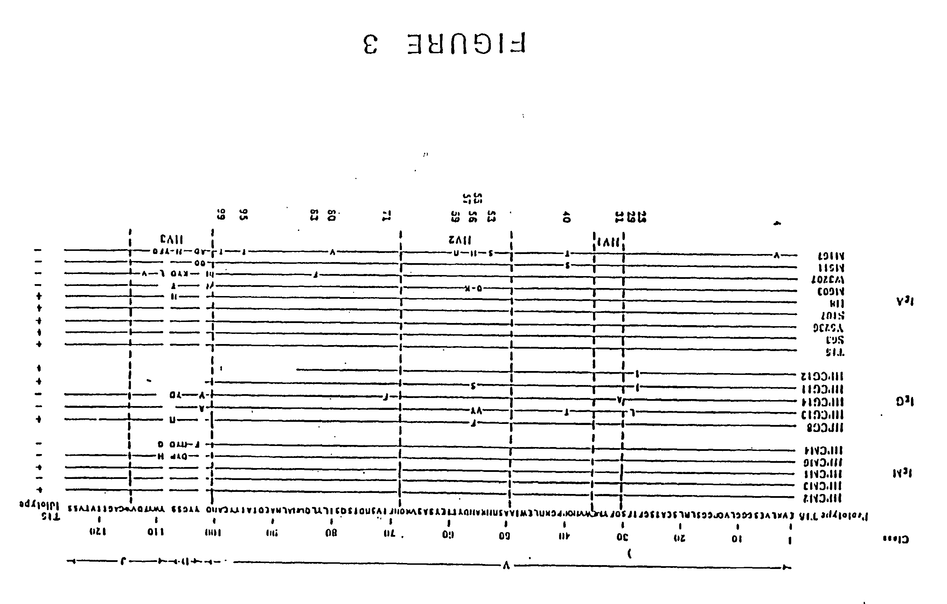

Figure 3 Amino acid sequence of the V_{H} regions of 19 mouse monoclonal antibodies with specificity for phosphorylcholine. The designation HP indicates that the protein is the product of a hybridoma. The remainder are myeloma proteins. (From Gearhart et al., Nature, 291:29, 1981.)

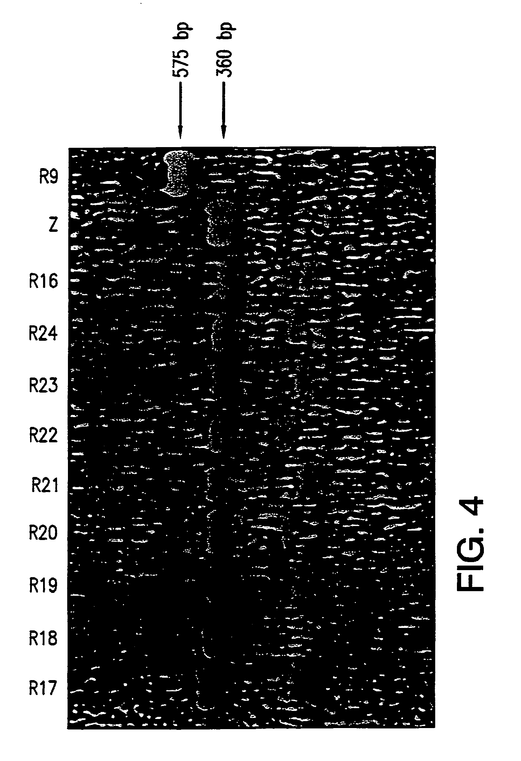

Figure 4 Illustrates the results obtained from PCR amplification of mRNA obtained from the spleen of a mouse immunized with FITC. Lanes R17-R24 correspond to amplification reactions with the unique 5' primers (2-9, Table 1) and the 3' primer (12, Table 1), R16 represents the PCR reaction with the 5' primer containing inosine (10, Table 1) and 3' primer (12, Table 1). Z and R9 are the amplification controls; control Z involves the amplification of V_{H} from a plasmid (PLR2) and R9 represents the amplification from the constant regions of spleen mRNA using primers 11 and 13 (Table 1).

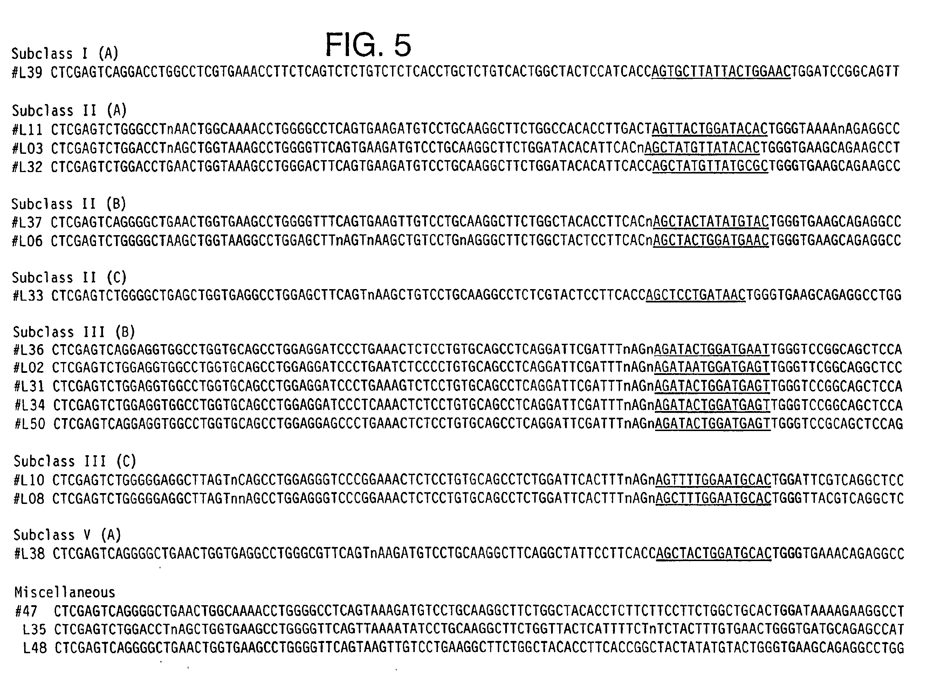

Figure 5 Nucleotide sequences are clones from the cDNA library of the PCR amplified V_{H} regions in Lambda ZAP. The N-terminal 110 bases are listed here and the underlined nucleotides represent CDR1 (complementary determining region).

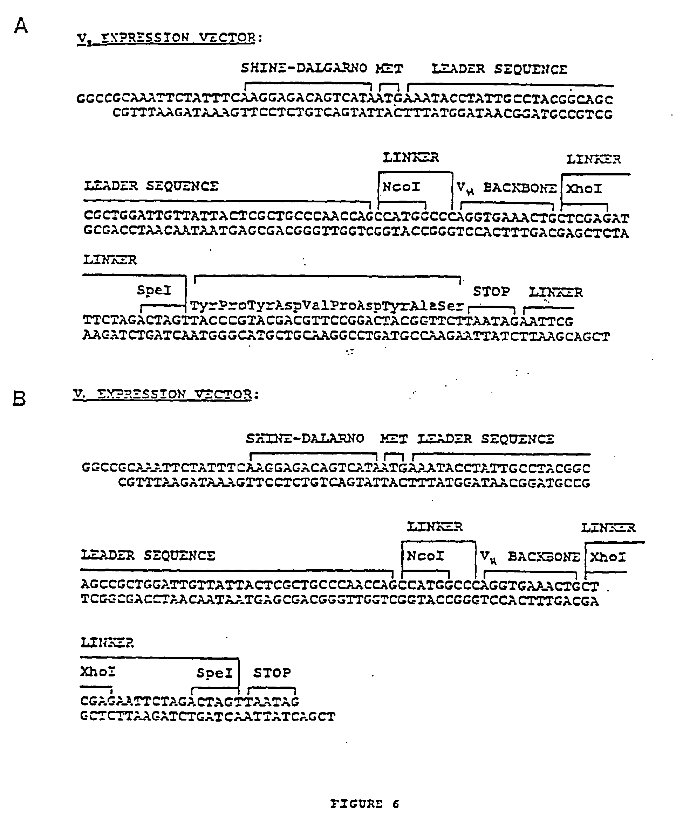

Figure 6 The sequence of the synthetic DNA insert inserted into Lambda ZAP to produce Lambda Zap II V_{H} (Panel A) and Lambda Zap V_{L} (Panel B) expression vectors. The various features required for this vector to express the V_{H} and V_{L}-coding DNA homologs include the Shine-Dalgarno ribosome binding site, a leader sequence to direct the expressed protein to the periplasm as described by Mouva et al., J. Biol. Chem., 255:27, 1980, and various restriction enzyme sites used to operatively link the V_{H} and V_{L} homologs to the expression vector. The V_{H} expression-vector sequence also contains a short nucleic acid sequence that codes for amino acids typically found in variable regions heavy chain (V_{H} Backbone). This V_{H} Backbone is just upstream and in the proper reading as the V_{H} DNA homologs that are operatively linked into the Xho I and Spe I. The V_{L} DNA homologs are operatively linked into the V_{L} sequence (Panel B) at the Nco I and Spe I restriction enzyme sites and thus the V_{H} Backbone region is deleted when the V_{L} DNA homologs are operatively linked into the V_{L} vector.

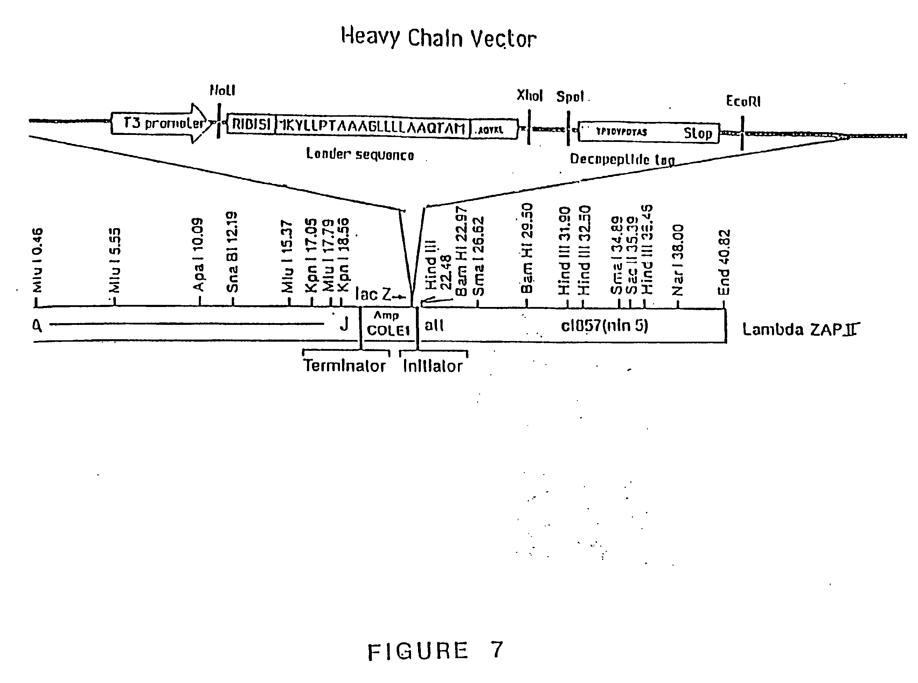

Figure 7 The major features of the bacterial expression vector Lambda Zap II V_{H} (V_{H}-expression vector) are shown. The synthetic DNA sequence from Figure 6 is shown at the top along with the T₃ polymerase promoter from Lambda Zap II. The orientation of the insert in Lambda Zap II is shown. The V_{H} DNA homologs are inserted into the Xho I and Spe I restriction enzyme sites. The V_{H} DNA are inserted into the Xho I and Spe I site and the read through transcription produces the decapeptide epitope (tag) that is located just 3' of the cloning sites.

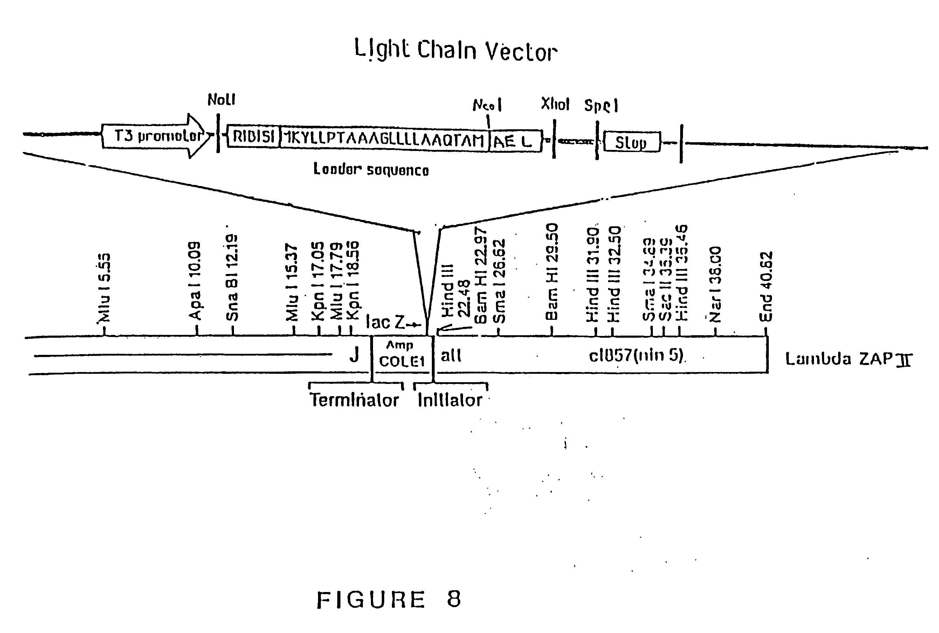

Figure 8 The major features of the bacterial expression vector Lambda Zap II V_{L} (V_{L} expression vector) are shown. The synthetic sequence shown in Figure 6 is shown at the top along with the T₃ polymerase promoter from Lambda Zap II. The orientation of the insert in Lambda Zap II is shown. The V_{L} DNA homologs are inserted into the phagemid that is produced by the in vivo excision protocol described by Short et al., Nucleic Acids Res., 16:7583-7600, 1988. The V_{L} DNA homologs are inserted into the Nco I and Spe I cloning sites of the phagemid.

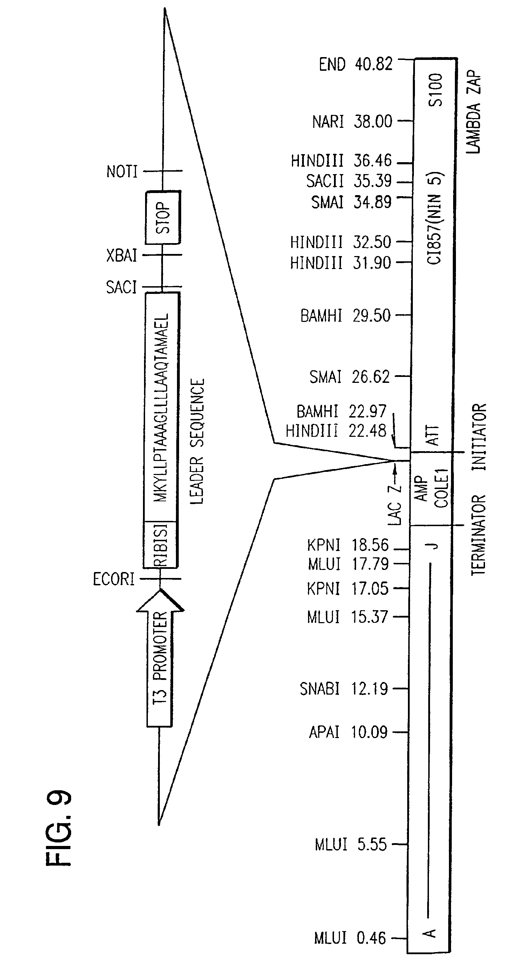

Figures 9 A modified bacterial expression vector Lambda Zap II V_{L}II. This vector is constructed by inserting this synthetic DNA sequence, 
TGAATTCTAAACTAGTCGCCAAGGAGACAGTCATAATGAA
TCGAACTTAAGATTTGATCAGCGGTTCCTCTGTCAGTATTACTT

ATACCTATTGCCTACGGCAGCCGCTGGATTGTTATTACTCGCTG
TATGGATAACGGATGCCGTCGGCGACCTAACAATAATGAGCGAC

CCCAACCAGCCATGGCCGAGCTCGTCAGTTCTAGAGTTAAGCGGCCG
GGGTTGGTCGGTACCGGCTCGAGCAGTCAAGATCTCAATTCGCCGGCAGCT
 into Lambda Zap II that has been digested with the restriction enzymes Sac I and Xho I. This sequence contains the Shine-Dalgarno sequence (Ribosome binding site), the leader sequence to direct the expressed protein to the periplasm and the appropriate nucleic acid sequence to allow the V_{L} DNA homologs to be operatively linked into the SacI and Xba I restriction enzyme sites provided by this vector.

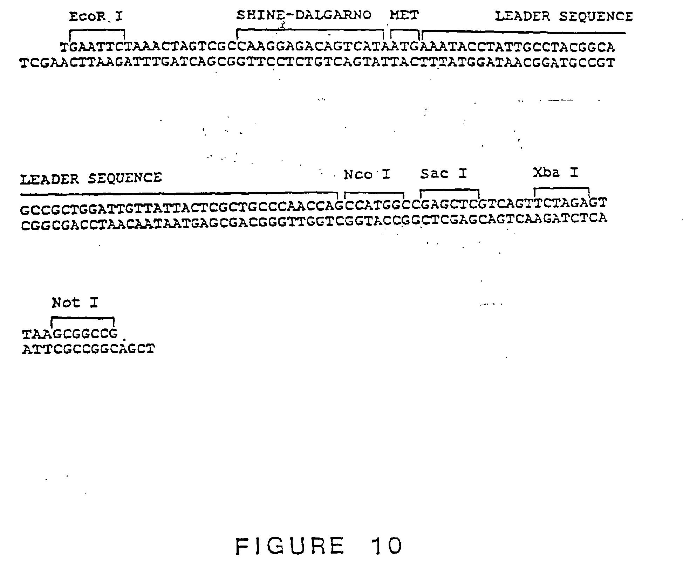

Figure 10 The sequence of the synthetic DNA segment inserted into Lambda Zap II to produce the lambda V_{L}II-expression vector. The various features and restriction endonuclease recognition sites are shown.

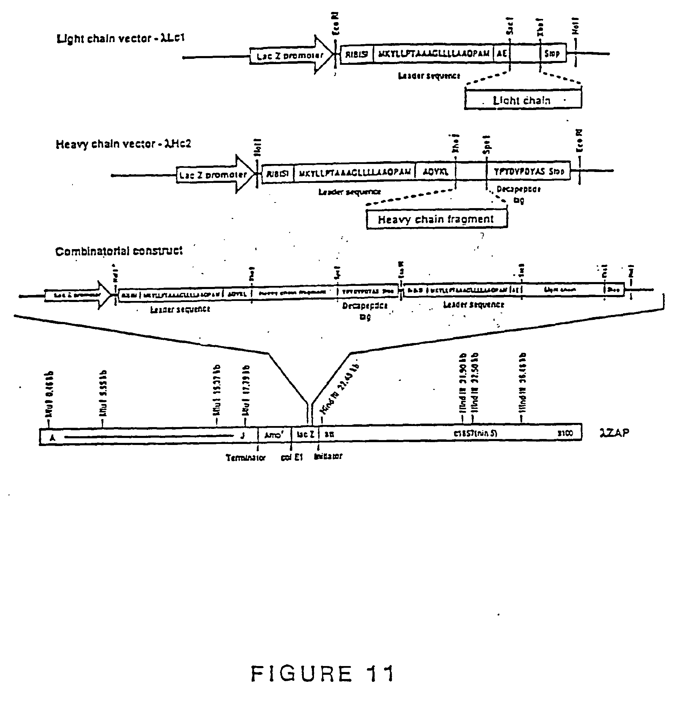

Figure 11 The vectors for expressing V_{H} and V_{L} separately and in combination are shown. The various essential components of these vectors are shown. The light chain vector or V_{L} expression vector can be combined with the V_{H} expression vector to produce a combinatorial vector containing both V_{H} and V_{L} operatively linked for expression to the same promoter.

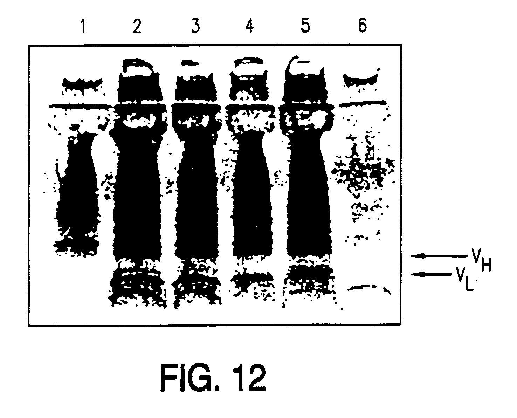

Figure 12 The labelled proteins immunoprecipitated from E. coli containing a V_{H} and a V_{L} DNA homolog are shown. In lane 1, the background proteins immunoprecipitated from E. coli that do not contain a V_{H} or V_{L} DNA homolog are shown. Lane 2 contains the V_{H} protein immunoprecipitated from E. coli containing only a V_{H} DNA homolog. In lanes 3 and 4, the co-migration of a V_{H} protein a V_{L} protein immunoprecipitated from E. coli containing both a V_{H} and a V_{L} DNA homolog is shown. In lane 5 the presence of V_{H} protein and V_{L} protein expressed from the V_{H} and V_{L} DNA homologs is demonstrated by the two distinguishable protein species. Lane 5 contains the background proteins immunoprecipitated by anti-E. coli antibodies present in mouse ascites fluid.

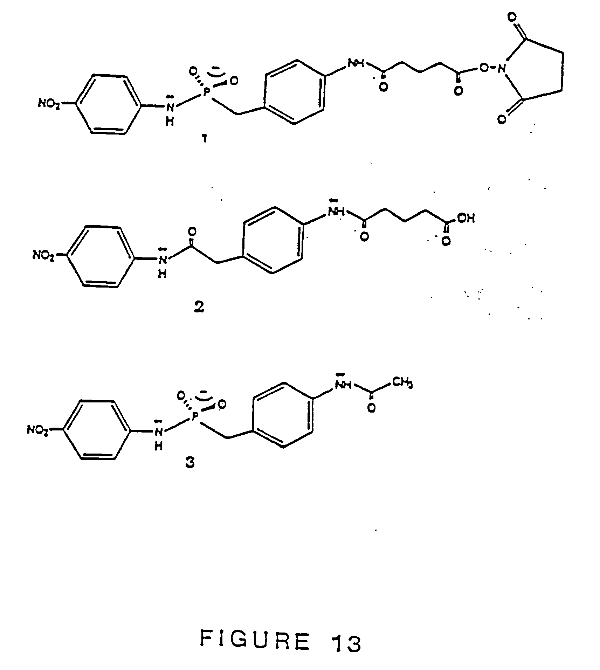

Figure 13 The transition state analogue (formula 1) which induces antibodies for hydrolyzing carboxamide substrate (formula 2). The compound of formula 1 containing a glutaryl spacer and a N-hydroxysuccinimide-linker appendage is the form used to couple the hapten (formula 1) to protein carriers KLH and BSA, while the compound of formula 3 is the inhibitor. The phosphonamidate functionality is a mimic of the stereo-electronic features of the transition state for hydrolysis of the amide bond.

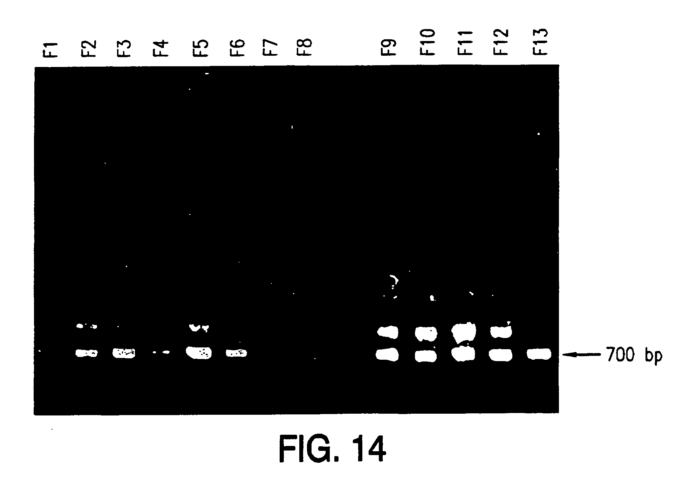

Figure 14 PCR amplification of Fd and kappa regions from the spleen mRNA of a mouse immunized with NPN is illustrated. Amplification was performed as described in Example 18 using RNA cDNA hybrids obtained by the reverse transcription of the mRNA with primer specific for amplification of light chain sequences (Table 2) or heavy chain sequences (Table 1). Lanes F1-F8 represent the product of heavy chain amplification reactions with one of each of the eight 5' primers (primers 2-9, Table 1) and the unique 3' primer (primer 15, Table 2). Light chain (k) amplifications with the 5' primers (primers 3-6, and 12, respectively, Table 2) and the appropriate 3' primer (primer 13, Table 2) are shown in lanes F9-F13. A band of 700 bps is seen in all lanes indicating the successful amplification of Fd and k regions.

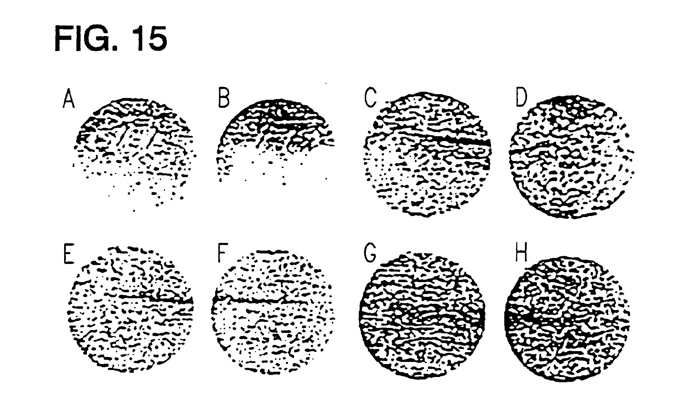

Figure 15 The screening of phage libraries for antigen binding is depicted according to Example 18C. Duplicate plaque lifts of Fab (filters A,B), heavy chain (filters E,F) and light chain (filters G,H) expression libraries were screened against ¹²⁵I-labelled BSA conjugated with NPN at a density of approximately 30,000 plaques per plate. Filters C and D illustrate the duplicate secondary screening of a cored positive from a primary filter A (arrows) as discussed in the text.
   Screening employed standard plaque lift methods. XL1 Blue cells infected with phage were incubated on 150mm plates for 4h at 37°, protein expression induced by overlay with nitrocellulose filters soaked in 10mM isopropyl thiogalactoside (IPTG) and the plates incubated at 25° for 8h. Duplicate filters were obtained during a second incubation employing the same conditions. Filters were then blocked in a solution of 1% BSA in PBS for 1h before incubation with rocking at 25° for 1h with a solution of ¹²⁵I-labelled BSA conjugated to NPN (2 x 10⁶ cpm ml⁻¹; BSA concentration at 0^{.}1 M; approximately 15 NPN per BSA molecule) in 1% BSA/PBS. Background was reduced by pre-centrifugation of stock radiolabelled BSA solution at 100,000 g for 15 min and pre-incubation of solutions with plaque lifts from plates containing bacteria infected with a phage having no insert. After labeling, filters were washed repeatedly with PBS/0.05% Tween 20 before development of autoradiographs overnight.

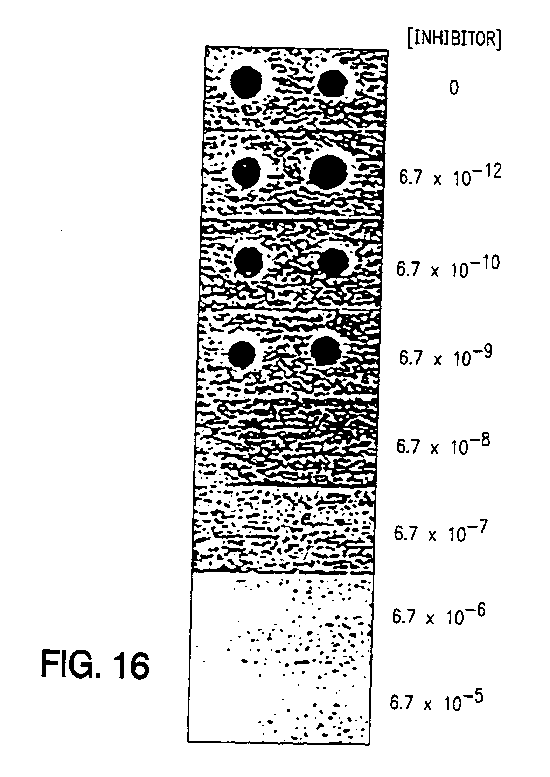

Figure 16 The specificity of antigen binding as shown by competitive inhibition is illustrated according to Example 18C. Filter lifts from positive plaques were exposed to ¹²⁵I-BSA-NPN in the presence of increasing concentrations of the inhibitor NPN.
   In this study a number of phages correlated with NPN binding as in Figure 15 were spotted (about 100 particles per spot) directly onto a bacterial lawns. The plate was then overlaid with an IPTG-soaked filter and incubated for 19h at 25°. The filter were then blocked in 1% BSA in PBS prior to incubation in ¹²⁵I-BSA-NPN as described previously in Figure 15 except with the inclusion of varying amounts of NPN in the labeling solution. Other conditions and procedures were as in Figure 15. The results for a phage of moderate affinity are shown in duplicate in the figure. Similar results were obtained for four other phages with some differences in the effective inhibitor concentration ranges.

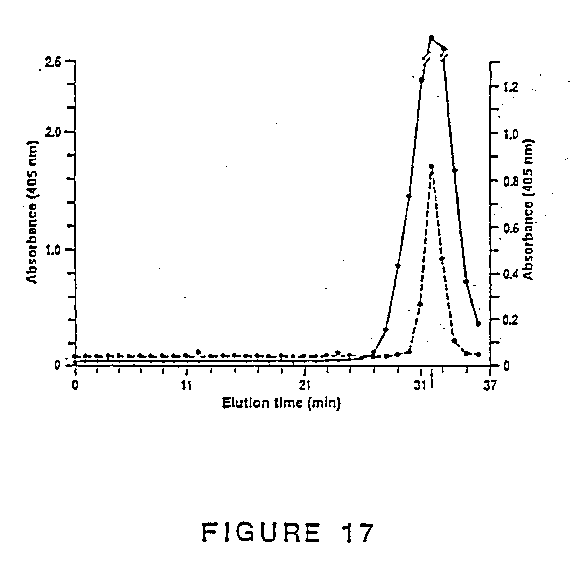

Figure 17 The characterization of an antigen binding protein is illustrated according to Example 18D. The concentrated partially purified bacterial supernate of an NPN-binding clone was separated by gel filtration and aliquots from each fraction applied to microtitre plates coated with BSA-NPN. Addition of either anti-decapeptide (---) or anti-kappa chain (——) antibodies conjugated with alkaline phosphatase was followed by color development. The arrow indicates the position of elution of a known Fab fragment. The results show that antigen binding is a property of 50 kD protein containing both heavy and light chains.
   Single plaques of two NPN-positive clones (Figure 15) were picked and the plasmid containing the heavy and light chain inserts excised (19). 500 ml cultures in L-broth were inoculated with 3 ml of a saturated culture containing the excised plasmids and incubated for 4h at 37 . Proteins synthesis was induced by the addition of IPTG to a final concentration of 1mM and the cultures incubated for 10h at 25°. 200 ml of cells supernate were concentrated to 2 ml and applied to a TSK-G4000 column. 50 µl aliquots from the eluted fractions were assayed by ELISA.
   For ELISA analysis, microtitre plates were coated with BSA-NPN at 1 ug/ml, 50 µl samples mixed with 50 µl PBS-Tween 20 (0.05%)-BSA (0.1%) added and the plates incubated for 2h at 25°. After washing with PBS-Tween 20-BSA, 50 µl of appropriate concentrations of a rabbit anti-decapeptide antibody (20) and a goat anti-mouse kappa light chain (Southern Biotech) antibody conjugated with alkaline phosphatase were added and incubated for 2h at 25. After further washing, 50 µl of p-nitrophenyl phosphate (1 mg/ml in 0.1M tris pH 9.5 containing 50 mM MgC12) were added and the plates incubated for 15-30 min before reading the OD at 405 nm.

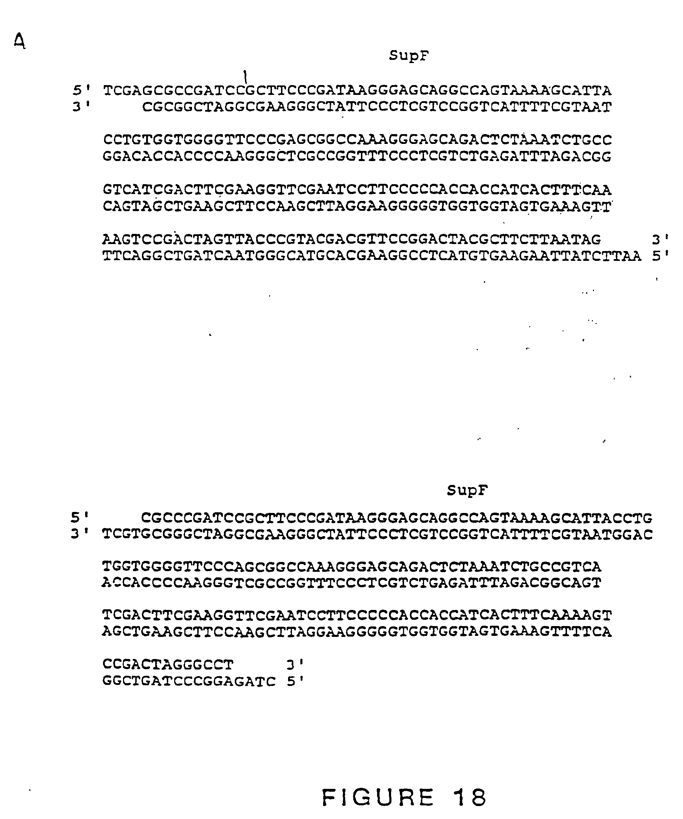

Figure 18 The sequence of the synthetic DNA insert inserted into Lambda Zap II V_{H} to produce the selectable V_{H} expression vector (panel A) and Lambda Zap II V_{L} II according to Example 17 to produce the selectable V_{L} expression vector (panel B).
Figure 19
   (A) The major features of the selectable V_{L} expression vector are shown in panel A. The feature of the synthetic DNA sequence from Figure 18A is shown at the top along with the T₃ polymerase promoter from Lambda Zap II. The orientation of the insert in Lambda Zap II is shown. The V_{H} DNA homologs are inserted into the Xho I and Spe I restriction enzyme sites. The V_{H} DNA homologs are inserted into the Xho I and Spe I site and the read through transcription produces the decapeptide epitope (tag) that is located just 3' of the cloning sites.
   (B) The major features of the bacterial expression vector Lambda Zap II V_{H} (V_{H}-expression vector) are shown. the synthetic DNA sequence from Figure 6 is shown at the top along with the T₃ polymerase promoter from Lambda Zap II. The orientation of the insert in Lambda Zap II is shown. The V_{H} DNA homologs are inserted into the Xho I and Spe I restriction enzyme sites. The V_{H} DNA are inserted into the Xho I and Spe I site and the read through transcription produces the decapeptide epitope (tag) that is located just 3' of the cloning sites.

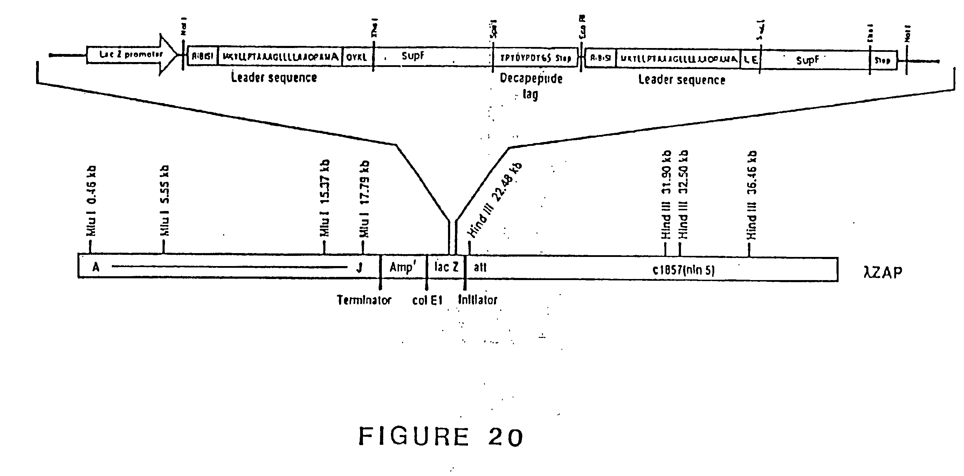

Figure 20 One of the vectors for expression V_{H} and V_{L} in combination are shown. The various essential components of these vectors are shown. The selectable marker (sup F) is shown.

### Detailed Description of the Invention

### A. Definitions

Nucleotide: a monomeric unit of DNA or RNA consisting of a sugar moiety (pentose), a phosphate, and a nitrogenous heterocyclic base. The base is linked to the sugar moiety via the glycosidic carbon (1' carbon of the pentose) and that combination of base and sugar is a nucleoside. When the nucleoside contains a phosphate group bonded to the 3' or 5' position of the pentose it is referred to as a nucleotide.

Base Pair (bp): a partnership of adenine (A) with thymine (T), or of cytosine (C) with guanine (G) in a double stranded DNA molecule. In RNA, uracil (U) is substituted for thymine.

Nucleic Acid: a polymer of nucleotides, either single or double stranded.

Gene: a nucleic acid whose nucleotide sequence codes for a RNA or polypeptide. A gene can be either RNA or DNA.

Complementary Bases: nucleotides that normally pair up when DNA or RNA adopts a double stranded configuration.

Complementary Nucleotide Sequence: a sequence of nucleotides in a single-stranded molecule of DNA or RNA that is sufficiently complementary to that on another single strand to specifically hybridize to it with consequent hydrogen bonding.

Conserved: a nucleotide sequence is conserved with respect to a preselected (reference) sequence if it non-randomly hybridizes to an exact complement of the preselected sequence.

Hybridization: the pairing of substantially complementary nucleotide sequences (strands of nucleic acid) to form a duplex or heteroduplex by the establishment of hydrogen bonds between complementary base pairs. It is a specific, i.e. non-random, interaction between two complementary polynucleotide that can be competitively inhibited.

Nucleotide Analog: a purine or pyrimidine nucleotide that differs structurally from a, T, G, C, or U, but is sufficiently similar to substitute for the normal nucleotide in a nucleic acid molecule.

DNA Homolog: is a nucleic acid having a preselected conserved nucleotide sequence and a sequence coding for a receptor capable of binding a preselected ligand.

Antibody: The term antibody in its various grammatical forms is used herein to refer to immunoglobulin molecules and immunologically active portions of immunoglobulin molecules, i.e., molecules that contain an antibody combining site or paratope. Exemplary antibody molecules are intact immunoglobulin molecules, substantially intact immunoglobulin molecules and portions of an immunoglobulin molecule, including those portions known in the art as Fab, Fab', F(ab')₂ and F(v).

Antibody Combining Site: An antibody combining site is that structural portion of an antibody molecule comprised of a heavy and light chain variable and hypervariable regions that specifically binds (immunoreacts with) an antigen. The term immunoreact in its various forms means specific binding between an antigenic determinant-containing molecule and a molecule containing an antibody combining site such as a whole antibody molecule or a portion thereof.

Monoclonal Antibody: The phrase monoclonal antibody in its various grammatical forms refers to a population of antibody molecules that contains only one species of antibody combining site capable of immunoreacting with a particular antigen. A monoclonal antibody thus typically displays a single binding affinity for any antigen with which it immunoreacts. A monoclonal antibody may therefore contain an antibody molecule having a plurality of antibody combining sites, each immunospecific for a different antigen, e.g., a bispecific monoclonal antibody.

### B. Methods

The present invention contemplates a method of isolating from a repertoire of conserved genes a gene coding for a receptor having a preselected activity, preferably a catalytic activity. The receptor can be a polypeptide, an RNA molecule, such as a transfer RNA, an RNA displaying enzymatic activity, and the like. Preferably, the receptor will be a polypeptide capable of binding a ligand, such as an enzyme, antibody molecule or immunologically active portion thereof, cellular receptor, or cellular adhesion protein coded for by one of the members of a family of conserved genes, i.e., genes containing a conserved nucleotide sequence of at least about 10 nucleotides in length.

Exemplary conserved gene families are those coding for immunoglobulins, major histocompatibility complex antigens of class I or II, lymphocyte receptors, integrins and the like.

A gene can be identified as belonging to a repertoire of conserved genes using several methods. For example, an isolated gene may be used as a hybridization probe under low stringency conditions to detect other members of the repertoire of conserved genes present, in genomic DNA using the methods described by Southern, J. Mol. Biol., 98:503 (1975). If the gene used as a hybridization probe hybridizes to multiple restriction endonuclease fragments that gene is a member of a repertoire of conserved genes.

### Immunoglobulins

The immunoglobulins, or antibody molecules, are a large family of molecules that include several types of molecules, such as IgD, IgG, IgA, IgM and IgE. The antibody molecule is typically comprised of two heavy (H) and light (L) chains with both a variable (V) and constant (C) region present on each chain. Several different regions of an immunoglobulin contain conserved sequences useful for isolating an immunoglobulin repertoire. Extensive amino acid and nucleic acid sequence data displaying exemplary conserved sequences is compiled for immunoglobulin molecules by Kabat et al., in Sequences of Proteins of Immunological Interest, National Institutes of Health, Bethesda, MD, 1987.

The C region of the H chain defines the particular immunoglobulin type. Therefore the selection of conserved sequences as defined herein from the C region of the H chain results in the preparation of a repertoire of immunoglobulin genes having members of the immunoglobulin type of the selected C region.

The V region of the H or L chain typically comprises four framework (FR) regions each containing relatively lower degrees of variability that includes lengths of conserved sequences. The use of conserved sequences from the FR1 and FR4 (J region) framework regions of the V_{H} chain is a preferred exemplary embodiment and is described herein in the Examples. Framework regions are typically conserved across several or all immunoglobulin types and thus conserved sequences contained therein are particularly suited for preparing repertoires having several immunoglobulin types.

### Major Histocompatibility Complex

The major histocompatibility complex (MHC) is a large genetic locus that encodes an extensive family of proteins that include several classes of molecules referred to as class I, class II or class III MHC molecules. Paul et al., in Fundamental Immunology, Raven Press, NY, pp. 303-378 (1984).

Class I MHC molecules are a polymorphic group of transplantation antigens representing a conserved family in which the antigen is comprised of a heavy chain and a non-MHC encoded light chain. The heavy chain includes several regions, termed the N, C1, C2, membrane and cytoplasmic regions. Conserved sequences useful in the present invention are found primarily in the N, C1 and C2 regions and are identified as continuous sequences of "invariant residues" in Kabat et al., supra.

Class II MHC molecules comprise a conserved family of polymorphic antigens that participate in immune responsiveness and are comprised of an alpha and a beta chain. The genes coding for the alpha and beta chain each include several regions that contain conserved sequences suitable for producing MHC class II alpha or beta chain repertoires. Exemplary conserved nucleotide sequences include those coding for amino acid residues 26-30 of the A1 region, residues 161-170 of the A2 region and residues 195-206 of the membrane region, all of the alpha chain. Conserved sequences are also present in the B1, B2 and membrane regions of the beta chain at nucleotide sequences coding for amino acid residues 41-45, 150-162 and 200-209, respectively.

### Lymphocyte Receptors and cell Surface Antigens

Lymphocytes contain several families of proteins on their cell surfaces including the T-cell receptor, Thy-1 antigen and numerous T-cell surface antigens including the antigens defined by the monoclonal antibodies 0KT4 (leu3), OKUT5/8 (leu2), OKUT3, OKUT1 (leul), OKT 11 (leu5) OKT6 and OKT9. Paul, supra at pp. 458-479.

The T-cell receptor is a term used for a family of antigen binding molecules found on the surface of T-cells. The T-cell receptor as a family exhibits polymorphic binding specificity similar to immunoglobulins in its diversity. The mature T-cell receptor is comprised of alpha and beta chains each having a variable (V) and constant (C) region. The similarities that the T-cell receptor has to immunoglobulins in genetic organization and function shows that T-cell receptor contains regions of conserved sequence. Lai et al., Nature, 331:543-546 (1988).

Exemplary conserved sequences include those coding for amino acid residues 84-90 of alpha chain, amino acid residues 107-115 of beta chain, and amino acid residues 91-95 and 111-116 of the gamma chain. Kabat et al., supra, p. 279.

### Integrins And Adhesions

Adhesive proteins involved in cell attachment are members of a large family of related proteins termed integrins. Integrins are heterodimers comprised of a beta and an alpha subunit. Members of the integrin family include the cell surface glycoproteins platelet receptor GpIIb-IIIa, vitronectin, receptor (VnR) fibronectin receptor (FnR) and the leukocyte adhesion receptors LFA-1, Mac-1, Mo-1 and 60.3. Roushahti et al., Science, 238:491-497 (1987). Nucleic acid and protein sequence data demonstrates regions of conserved sequences exist in the members of these families particularly between the beta chain of GpIIb-IIIa VnR and FnR, and between the alpha subunit of VnR, Mac-1, LFA-1, Fnr and GpIIb-IIIa. Suzuki et al., Proc. Natl. Acad. Sci. USA, 83:8614-8618, 1986; Ginsberg et al., J. Biol. Chem., 262:5437-5440, 1987.

The following discussion illustrates the method of the present invention applied to isolating a conserved receptor-coding gene from the immunoglobulin gene repertoire. This discussion is not to be taken as limiting, but rather as illustrating application of principles that can be used to isolate a gene from any family of conserved genes coding for functionally related receptors.

Generally, the method combines the following elements:
1. Isolating nucleic acids containing a substantial portion of the immunological repertoire.
2. Preparing polynucleotide primers for cloning polynucleotide segments containing immunoglobulin V_{H} and/or V_{L} region genes.
3. Preparing a gene library containing a plurality of different V_{H} and V_{L} genes from the repertoire.
4. Expressing the V_{H} and/or V_{L} polypeptides in a suitable host, including prokaryotic and eukaryotic hosts, either separately or in the same cell, and either on the same or different expression vectors.
5. Screening the expressed polypeptides for the preselected activity, and segregating a V_{H}- and/or V_{L}-coding gene identified by the screening process.

A receptor produced by the present invention assumes a conformation having a binding site specific for as evidenced by its ability to be competitively inhibited, a preselected or predetermined ligand such as an antigen, enzymatic substrate and the like. In one embodiment, a receptor of this invention is a ligand binding polypeptide that forms an antigen binding site which specifically binds to a preselected antigen to form a complex having a sufficiently strong binding between the antigen and the binding site for the complex to be isolated. When the receptor is an antigen binding polypeptide its affinity or avidity is generally greater than 10⁵- M⁻¹ more usually greater than 10⁶ and preferably greater than 10⁸ M⁻¹.

In another embodiment, a receptor of the subject invention binds a substrate and catalyzes the formation of a product from the substrate. While the topology of the ligand binding site of a catalytic receptor is probably more important for its preselected activity than its affinity (association constant or pKa) for the substrate, the subject catalytic receptors have an association constant for the preselected substrate generally greater than 10³ M⁻¹, more usually greater than 10⁵ M⁻¹ or 10⁶ M⁻¹ and preferably greater than 10⁷ M⁻¹.

Preferably the receptor produced by the subject invention is heterodimeric and is therefore normally comprised of two different polypeptide chains, which together assume a conformation having a binding affinity, or association constant for the preselected ligand that is different, preferably higher, than the affinity or association constant of either of the polypeptides alone, i.e., as monomers. One or both of the different polypeptide chains is derived from the variable region of the light and heavy chains of an immunoglobulin. Typically, polypeptides comprising the light (V_{L}) and heavy (V_{H}) variable regions are employed together for binding the preselected ligand.

A receptor produced by the subject invention can be active in monomeric as well as multimeric forms, either homomeric or heteromeric, preferably heterodimeric. For example, V_{H} and V_{L} ligand binding polypeptide produced by the present invention can be advantageously combined in the heterodimer to modulate the activity of either or to produce an activity unique to the heterodimer. The individual ligand binding polypeptides will be referred to as V_{H} and V_{L} and the heterodimer will be referred to as a Fv.

However, it should be understood that a V_{H} binding polypeptide may contain in addition to the V_{H}, substantially all or a portion of the heavy chain constant region. A V_{L} binding polypeptide may contain, in addition to the V_{L}, substantially all or a portion of the light chain constant region. A heterodimer comprised of a V_{H} binding polypeptide containing a portion of the heavy chain constant region and a V_{L} binding containing substantially all of the light chain constant region is termed a Fab fragment. The production of Fab can be advantageous in some situations because the additional constant region sequences contained in a Fab as compared to a F_{V} could stabilize the V_{H} and V_{L} interaction. Such stabilization could cause the Fab to have higher affinity for antigen. In addition the Fab is more commonly used in the art and thus there are more commercial antibodies available to specifically recognize a Fab.

The individual V_{H} and V_{L} polypeptides will generally have fewer than 125 amino acid residues, more usually fewer than about 120 amino acid residues, while normally having greater than 60 amino acid residues, usually greater than about 95 amino acid residues, more usually greater than about 100 amino acid residues. Preferably, the V_{H} will be from about 110 to about 125 amino acid residues in length while V_{L} will be from about 95 to about 115 amino acid residues in length.

The amino acid residue sequences will vary widely, depending upon the particular idiotype involved. Usually, there will be at least two cysteines separated by from about 60 to 75 amino acid residues and joined by a disulfide bond. The polypeptides produced by the subject invention will normally be substantial copies of idiotypes of the variable regions of the heavy and/or light chains of immunoglobulins, but in some situations a polypeptide may contain random mutations in amino acid residue sequences in order to advantageously improve the desired activity.

In some situations, it is desirable to provide for covalent cross linking of the V_{H} and V_{L} polypeptides, which can be accomplished by providing cysteine resides at the carboxyl termini. The polypeptide will normally be prepared free of the immunoglobulin constant regions, however a small portion of the J region may be included as a result of the advantageous selection of DNA synthesis primers. The D region will normally be included in the transcript of the V_{H}.

In other situations, it is desirable to provide a peptide linker to connect the V_{L} and the V_{H} to form a single-chain antigen-binding protein comprised of a V_{H} and a V_{L}. This single-chain antigen-binding protein would be synthesized as.a single protein chain. Such single-chain antigen-binding proteins have been described by Bird et al., Science, 242:423-426 (1988). The design of suitable peptide linker regions is described in U.S. Patent No. 4, 704, 692 by Robert Landner.

Such a peptide linker could be designed as part of the nucleic acid sequences contained in the expression vector. The nucleic acid sequences coding for the peptide linker would be between the V_{H} and V_{L} DNA homologs and the restriction endonuclease sites used to operatively link the V_{H} an V_{L} DNA homologs to the expression vector.

Such a peptide linker could also be coded for nucleic acid sequences that are part of the polynucleotide primers used to prepare the various gene libraries. The nucleic acid sequence coding for the peptide linker can be made up of nucleic acids attached to one of the primers or the nucleic acid sequence coding for the peptide linker may be derived from nucleic acid sequences that are attached to several polynucleotide primers used to create the gene libraries.

Typically the C terminus region of the V_{H} and V_{L} polypeptides will have a greater variety of the sequences than the N terminus and, based on the present strategy, can be further modified to permit a variation of the normally occurring V_{H} and V_{L} chains. A synthetic polynucleotide can be employed to vary one or more amino in an hypervariable region.

### 1. Isolation Of The Repertoire

To prepare a composition of nucleic acids containing a substantial portion of the immunological gene repertoire, a source of genes coding for the V_{H} and/or V_{L} polypeptides is required. Preferably the source will be a heterogeneous population of antibody producing cells, i.e. B lymphocytes (B cells), preferably rearranged B cells such as those found in the circulation or spleen of a vertebrate. (Rearranged B cells are those in which immunoglobulin gene translocation, i.e., rearrangement, has occurred as evidenced by the presence in the cell of mRNA with the immunoglobulin gene V, D and J region transcripts adjacently located thereon.)

In some cases, it is desirable to bias the repertoire for a preselected activity, such as by using as a source of nucleic acid cells (source cells) from vertebrates in any one of various stages of age, health and immune response. For example, repeated immunization of a healthy animal prior to collecting rearranged B cells results in obtaining a repertoire enriched for genetic material producing a ligand binding polypeptide of high affinity. Conversely, collecting rearranged B cells from a healthy animal whose immune system has not been recently challenged results in producing a repertoire that is not biased towards the production of high affinity V_{H} and/or V_{L} polypeptides.

It should be noted the greater the genetic heterogeneity of the population of cells for which the nucleic acids are obtained, the greater the diversity of the immunological repertoire that will be made available for screening according to the method of the present invention. Thus, cells from different individuals, particularly those having an immunologically significant age difference, and cells from individuals of different strains, races or species can be advantageously combined to increase the heterogeneity of the repertoire.

Thus, in one preferred embodiment, the source cells are obtained from a vertebrate, preferably a mammal, which has been immunized or partially immunized with an antigenic ligand (antigen) against which activity is sought, i.e., a preselected antigen. The immunization can be carried out conventionally. Antibody titer in the animal can be monitored to determine the stage of immunization desired, which stage corresponds to the amount of enrichment or biasing of the repertoire desired. Partially immunized animals typically receive only one immunization and cells are collected therefrom shortly after a response is detected. Fully immunized animals display a peak titer, which is achieved with one or more repeated injections of the antigen into the host mammal, normally at 2 to 3 week intervals. Usually three to five days after the last challenge, the spleen is removed and the genetic repertoire of the spleenocytes, about 90% of which are rearranged B cells, is isolated using standard procedures. See, Current Protocols in Molecular Biology, Ausubel et al., eds., John Wiley & Sons, NY. Nucleic acids coding for V_{H} and V_{L} polypeptides can be derived from cells producing IgA, IgD, IgE, IgG or IgM, most preferably from IgM and IgG, producing cells.

Methods for preparing fragments of genomic DNA from which immunoglobulin variable region genes can be cloned as a diverse population are well known in the art. See for example Herrmann et al., Methods In Enzymol., 152:180-183, (1987); Frischauf, Methods In Enzymol., 152:183-190 (1987); Frischauf, Methods In Enzymol., 152:190-199 (1987); and DiLella et al., Methods In Enzymol., 152:199-212 (1987). (The teachings of the references cited herein are hereby incorporated by reference.)

The desired gene repertoire can be isolated from either genomic material containing the gene expressing the variable region or the messenger RNA (mRNA) which represents a transcript of the variable region. The difficulty in using the genomic DNA from other than non-rearranged B lymphocytes is in juxtaposing the sequences coding for the variable region, where the sequences are separated by introns. The DNA fragment (s) containing the proper exons must be isolated, the introns excised, and the exons then spliced in the proper order and in the proper orientation. For the most part, this will be difficult, so that the alternative technique employing rearranged B cells will be the method of choice because the C D and J immunoglobulin gene regions have translocated to become adjacent, so that the sequence is continuous (free of introns) for the entire variable regions.

Where mRNA is utilized the cells will be lysed under RNase inhibiting conditions. In one embodiment, the first step is to isolate the total cellular mRNA by hybridization to an oligo-dT cellulose column. The presence of mRNAs coding for the heavy and/or light chain polypeptides can then be assayed by hybridization with DNA single strands of the appropriate genes. Conveniently, the sequences coding for the constant portion of the V_{H} and V_{L} can be used as polynucleotide probes, which sequences can be obtained from available sources. See for example, Early and Hood, Genetic Engineering, Setlow and Hollaender, eds., Vol. 3, Plenum Publishing Corporation, NY, (1981), pages 157-188; and Kabat et al., Sequences of Immunological Interest, National Institutes of Health, Bethesda, MD, (1987). In preferred embodiments, the preparation containing the total cellular mRNA is first enriched for the presence of V_{H} and/or V_{L} coding mRNA. Enrichment is typically accomplished by subjecting the total mRNA preparation or partially purified mRNA product thereof to a primer extension reaction employing a polynucleotide synthesis primer of the present invention.

### 2. Preparation Of Polynucleotide Primers

The term "polynucleotide" as used herein in reference to primers, probes and nucleic acid fragments or segments to be synthesized by primer extension is defined as a molecule comprised of two or more deoxyribonucleotides or ribonucleotides, preferably more than 3. Its exact size will depend on many factors, which in turn depends on the ultimate conditions of use.

The term "primer" as used herein refers to a polynucleotide whether purified from a nucleic acid restriction digest or produced synthetically, which is capable of acting as a point of initiation of synthesis when placed under conditions in which synthesis of a primer extension product which is complementary to a nucleic acid strand is induced, i.e., in the presence of nucleotides and an agent for polymerization such as DNA polymerase, reverse transcriptase and the like, and at a suitable temperature and pH. The primer is preferably single stranded for maximum efficiency, but may alternatively be double stranded. If double stranded, the primer is first treated to separate its strands before being used to prepare extension products. Preferably, the primer is a polydeoxyribonucleotide. The primer must be sufficiently long to prime the synthesis of extension products in the presence of the agents for polymerization. The exact lengths of the primers will depend on may factors, including temperature and the source of primer. For example, depending on the complexity of the target sequence, a polynucleotide primer typically contains 15 to 25 or more nucleotides, although it can contain fewer nucleotides. Short primer molecules generally require cooler temperatures to form sufficiently stable hybrid complexes with template.

The primers used herein are selected to be "substantially" complementary to the different strands of each specific sequence to be synthesized or amplified. This means that the primer must be sufficiently complementary to non-randomly hybridize with its respective template strand. Therefore, the primer sequence may not reflect the exact sequence of the template. For example, a non-complementary nucleotide fragment can be attached to the 5' end of the primer, with the remainder of the primer sequence being substantially complementary to the strand. Such non-complementary fragments typically code for an endonuclease restriction site. Alternatively, non-complementary bases or longer sequences can be interspersed into the primer, provided the primer sequence has sufficient complementarily with the sequence of the strand to be synthesized or amplified to non-randomly hybridize therewith and thereby form an extension product under polynucleotide synthesizing conditions.

The polynucleotide primers can be prepared using any suitable method, such as, for example, the phosphotriester on phosphodiester methods see Narang et al., Meth. Enzynol., 68:90, (1979); U.S. Patent No. 4,356,270; and Brown et al., Meth. Enzymol., 68:109, (1979).

The choice of a primer's nucleotide sequence depends on factors such as the distance on the nucleic acid from the region coding for the desired receptor, its hybridization site on the nucleic acid relative to any second primer to be used, the number of genes in the repertoire it is to hybridize to, and the like.

For example, to produce V_{H}-coding DNA homologs by primer extension, the nucleotide sequence of a primer is selected to hybridize with a plurality of immunoglobulin heavy chain genes at a site substantially adjacent to the V_{H}-coding region so that a nucleotide sequence coding for a functional (capable of binding) polypeptide is obtained. To hybridize to a plurality of different V_{H}-coding nucleic acid strands, the primer must be a substantial complement of a nucleotide sequence conserved among the different strands. Such sites include nucleotide sequences in the constant region, any of the variable region framework regions, preferably the third framework region, leader region, promoter region, J region and the like.

If the V_{H}-coding and V_{L}-coding DNA homologs are to be produced by polymerase chain reaction (PCR) amplification, two primers must be used for each coding strand of nucleic acid to be amplified. The first primer becomes part of the nonsense (minus or complementary) strand and hybridizes to a nucleotide sequence conserved among V_{H} (plus) strands within the repertoire. To produce V_{H} coding DNA homologs, first primers are therefore chosen to hybridize to (i.e. be complementary to) conserved regions within the J region, CH1 region, hinge region, CH2 region, or CH3 region of immunoglobulin genes and the like. To produce a V_{L} coding DNA homolog, first primers are chosen to hybridize with (i.e. be complementary to) a conserved region within the J region or constant region of immunoglobulin light chain genes and the like. Second primers become part of the coding (plus) strand and hybridize to a nucleotide sequence conserved among minus strands. To produce the V_{H}-coding DNA homologs, second primers are therefore chosen to hybridize with a conserved nucleotide sequence at the 5' end of the V_{H}-coding immunoglobulin gene such as in that area coding for the leader or first framework region. It should be noted that in the amplification of both V_{H}- and V_{L}-coding DNA homologs the conserved 5' nucleotide sequence of the second primer can be complementary to a sequence exogenously added using terminal deoxynucleotidyl transferase as described by Loh et al., Sci. Vol 243:217-220 (1989). One or both of the first and second primers can contain a nucleotide sequence defining an endonuclease recognition site. The site can be heterologous to the immunoglobulin gene being amplified and typically appears at or near the 5' end of the primer.

Primers of the present invention may also contain a DNA-dependent RNA polymerase promoter sequence or its complement. See for example, Krieg et al.,Nucleic Acids Research, 12:7057-70 (1984); Studier et al., J. Mol. Biol., 189:113-130 (1986); and Molecular Cloning: A Laboratory Manual, Second Edition, Maniatis et al., eds., Cold Spring Harbor, NY (1989).

When a primer containing a DNA-dependent RNA polymerase promoter is used the primer is hybridized to the polynucleotide strand to be amplified and the second polynucleotide strand of the DNA-dependent RNA polymerase promoter is completed using an inducing agent such as E. coli, DNA polymerase I, or the Klenow fragment of E. coli DNA polymerase. The starting polynucleotide is amplified by alternating between the production of an RNA polynucleotide and DNA polynucleotide.

Primers may also contain a template sequence or replication initiation site for a RNA-directed RNA polymerase. Typical RNA-directed RNA polymerase include the QB replicase described by Lizardi et al., Biotechnology, 6:1197-1202 (1988).

RNA-directed polymerases produce large numbers of RNA strands from a small number of template RNA strands that contain a template sequence or replication initiation site. These polymerases typically give a one million-fold amplification of the template strand as has been described by Kramer et al., J. Mol. Biol., 89:719-736 (1974).

### 3. Preparing a Gene Library

The strategy used for cloning, i.e., substantially reproducing, the V_{H} and/or V_{L} genes contained within the isolated repertoire will depend, as is well known in the art, on the type, complexity, and purity of the nucleic acids making up the repertoire. Other factors include whether or not the genes are to be amplified and/or mutagenized.

In one strategy, the object is to clone the V_{H}- and/or V_{L}-coding genes from a repertoire comprised of polynucleotide coding strands, such as mRNA and/or the sense strand of genomic DNA. If the repertoire is in the form of double stranded genomic DNA, it is usually first denatured, typically by melting, into single strands. The repertoire is subjected to a first primary extension reaction by treating (contacting) the repertoire with a first polynucleotide synthesis primer having a preselected nucleotide sequence. The first primer is capable of initiating the first primer extension reaction by hybridizing to a nucleotide sequence, preferably at least about 10 nucleotides in length and more preferably at least about 20 nucleotides in length, conserved within the repertoire. The first primer is sometimes referred to herein as the "sense primer" because it hybridizes to the coding or sense strand of a nucleic acid. In addition, the second primer is sometimes referred to herein as the "anti-sense primer" because it hybridizes to a non-coding or anti-sense strand of a nucleic acid, i.e., a strand complementary to a coding strand.

The first primer extension is performed by mixing the first primer, preferably a predetermined amount thereof, with the nucleic acids of the repertoire, preferably a predetermined amount thereof, to form a first primer extension reaction admixture. The admixture is maintained under polynucleotide synthesizing conditions for a time period, which is typically predetermined, sufficient for the formation of a first primer extension reaction product, thereby producing a plurality of different V_{H}-coding DNA homolog complements. The complements are then subjected to a second primer extension reaction by treating them with a second polynucleotide synthesis primer having a preselected nucleotide sequence. The second primer is capable of initiating the second reaction by hybridizing to a nucleotide sequence, preferably at least about 10 nucleotides in length and more preferably at least about 20 nucleotides in length, conserved among a plurality of different V_{H}-coding gene complements such as those, for example, produced by the first primer extension reaction. This is accomplished by mixing the second primer, preferably a predetermined amount thereof, with the complement nucleic acids, preferably a predetermined amount thereof, to form a second primer extension reaction admixture. The admixture is maintained under polynucleotide synthesizing conditions for a time period, which is typically predetermined, sufficient for the formation of a first primer extension reaction product, thereby producing a gene library containing a plurality of different V_{H}-and/or V_{L}-coding DNA homologs.

A plurality of first primer and/or a plurality of second primers can be used in each amplification, or an individual pair of first and second primers can be used. In any case, the amplification products of amplifications using the same or different combinations of first and second primers can be combined to increase the diversity of the gene library.

In another strategy, the object is to clone the V_{H}- and/or V_{L}-coding genes from a repertoire by providing a polynucleotide complement of the repertoire, such as the anti-sense strand of genomic dsDNA or the polynucleotide produced by subjecting mRNA to a reverse transcriptase reaction. Methods for producing such complements are well known in the art. The complement is subjected to a primer extension reaction similar to the above-described second primer extension reaction, i.e., a primer extension reaction using a polynucleotide synthesis primer capable of hybridizing to a nucleotide sequence conserved among a plurality of different V_{H}-coding gene complements.

The primer extension reaction is performed using any suitable method. Generally it occurs in a buffered aqueous solution, preferably at a pH of 7-9, most preferably about 8. Preferably, a molar excess (for genomic nucleic acid, usually about 10⁶:1 primer:template) of the primer is admixed to the buffer containing the template strand. A large molar excess is preferred to improve the efficiency of the process.

The deoxyribonucleotide triphosphates dATP, dCTP, dGTP, and dTTP are also admixed to the primer extension (polynucleotide synthesis) reaction admixture in adequate amounts and the resulting solution is heated to about 90C - 100C for about 1 to 10 minutes, preferably from 1 to 4 minutes. After this heating period the solution is allowed to cool to room temperature, which is preferable for primer hybridization. To the cooled mixture is added an appropriate agent for inducing or catalyzing the primer extension reaction, and the reaction is allowed to occur under conditions known in the art. The synthesis reaction may occur at from room temperature up to a temperature above which the inducing agent no longer functions efficiently. Thus, for example, if DNA polymerase is used as inducing agent, the temperature is generally no greater than about 40C.

The inducing agent may be any compound or system which will function to accomplish the synthesis of primer extension products, including enzymes. Suitable enzymes for this purpose include, for example, E. coli, DNA polymerase I, Klenow fragment of E. coli DNA polymerase I, T4 DNA polymerase, other available DNA polymerases, reverse transcriptase, and other enzymes, including heat-stable enzymes, which will facilitate combination of the nucleotides in the proper manner to form the primer extension products which are complementary to each nucleic acid strand. Generally, the synthesis will be initiated at the 3' end of each primer and proceed in the 5' direction along the template strand, until synthesis terminates, producing molecules of different lengths. There may be inducing agents, however, which initiate synthesis at the 5' end and proceed in the above direction, using the same process as described above.

The inducing agent also may be a compound or system which will function to accomplish the synthesis of RNA primer extension products, including enzymes. In preferred embodiments, the inducing agent may be a DNA-dependent RNA polymerase such as T7 RNA polymerase, T3 RNA polymerase or SP6 RNA polymerase. These polymerases produce a complementary RNA polynucleotide. The high turn over rate of the RNA polymerase amplifies the starting polynucleotide as has been described by Chamberlin et al., The Enzymes, ed. P. Boyer, PP. 87-108, Academic Press, New York (1982). Another advantage of T7 RNA polymerase is that mutations can be introduced into the polynucleotide synthesis by replacing a portion of cDNA with one or more mutagenic oligodeoxynucleotides (polynucleotides) and transcribing the partially-mismatched template directly as has been previously described by Joyce et al., Nucleic Acid Research, 17:711-722 (1989). Amplification systems based on transcription have been described by Gingeras et al., in PCR Protocols, A Guide to Methods and Applications, pp 245-252, Academic Press, Inc., San Diego, CA (1990).

If the inducing agent is a DNA-dependent RNA polymerase and therefore incorporates ribonucleotide triphosphates, sufficient amounts of ATP, CTP, GTP and UTP are admixed to the primer extension reaction admixture and the resulting solution is treated as described above.

The newly synthesized strand and its complementary nucleic acid strand form a double-stranded molecule which can be used in the succeeding steps of the process.

The first and/or second primer extension reaction discussed above can advantageously be used to incorporate into the receptor a preselected epitope useful in immunologically detecting and/or isolating a receptor. This is accomplished by utilizing a first and/or second polynucleotide synthesis primer or expression vector to incorporate a predetermined amino acid residue sequence into the amino acid residue sequence of the receptor.

After producing V_{H}- and/or V_{L}-coding DNA homologs for a plurality of different V_{H}- and/or V_{L}-coding genes within the repertoire, the homologs are typically amplified. While the V_{H} and/or V_{L}-coding DNA homologs can be amplified by classic techniques such as incorporation into an autonomously replicating vector, it is preferred to first amplify the DNA homologs by subjecting them to a polymerase chain reaction (PCR) prior to inserting them into a vector. In fact, in preferred strategies, the first and/or second primer extension reactions used to produce the gene library are the first and second primer extension reactions in a polymerase chain reaction.

PCR is typically carried out by cycling i.e., simultaneously performing in one admixture, the above described first and second primer extension reactions, each cycle comprising polynucleotide synthesis followed by denaturation of the double stranded polynucleotides formed. Methods and systems for amplifying a DNA homolog are described in U.S. Patents No. 4,683,195 and No. 4,683,202, both to Mullis et al.

In preferred embodiments only one pair of first and second primers is used per amplification reaction. The amplification reaction products obtained from a plurality of different amplifications, each using a plurality of different primer pairs, are then combined.

However, the present invention also contemplates DNA homolog production via coamplification (using two pairs of primers), and multiplex amplification (using up to about 8, 9 or 10 primer pairs).

The V_{H}- and V_{L}-coding DNA homologs produced by PCR amplification are typically in double-stranded form and have contiguous or adjacent to each of their termini a nucleotide sequence defining an endonuclease restriction site. Digestion of the V_{H}- and V_{L}-coding DNA homologs having restriction sites at or near their termini with one or more appropriate endonucleases results in the production of homologs having cohesive termini of predetermined specificity.

In preferred embodiments, the PCR process is used not only to amplify the V_{H}- and/or V_{L}-coding DNA homologs of the library, but also to induce mutations within the library and thereby provide a library having a greater heterogeneity. First, it should be noted that the PCR processes itself is inherently mutagenic due to a variety of factors well known in the art. Second, in addition to the mutation inducing variations described in the above referenced U.S. Patent No. 4,683,195, other mutation inducing PCR variations can be employed. For example, the PCR reaction admixture, i.e., the combined first and second primer extension reaction admixtures, can be formed with different amounts of one or more of the nucleotides to be incorporated into the extension product. Under such conditions, the PCR reaction proceeds to produce nucleotide substitutions within the extension product as a result of the scarcity of a particular base. Similarly, approximately equal molar amounts of the nucleotides can be incorporated into the initial PCR reaction admixture in an amount to efficiently perform X number of cycles, and then cycling the admixture through a number of cycles in excess of X, such as, for instance, 2X. Alternatively, mutations can be induced during the PCR reaction by incorporating into the reaction admixture nucleotide derivatives such as inosine, not normally found in the nucleic acids of the repertoire being amplified. During subsequent in vivo amplification, the nucleotide derivative will be replaced with a substitute nucleotide thereby inducing a point mutation.

### 4. Expressing the V_{H} and/or V_{L} DNA Homologs.

The V_{H}- and/or V_{L}-coding DNA homologs contained within the library produced by the above-described method can be operatively linked to a vector for amplification and/or expression.

As used herein, the term "vector" refers to a nucleic acid molecule capable of transporting between different genetic environments another nucleic acid to which it has been operatively linked. One type of preferred vector is an episome, i.e., a nucleic acid molecule capable of extra-chromosomal replication. Preferred vectors are those capable of autonomous replication and/or expression of nucleic acids to which they are linked. Vectors capable of directing the expression of genes to which they are operatively linked are referred to herein as "expression vectors".

The choice of vector to which a V_{H}- and/or V_{L}-coding DNA homolog is operatively linked depends directly, as is well known in the art, on the functional properties desired, e.g., replication or protein expression, and the host cell to be transformed, these being limitations inherent in the art of constructing recombinant DNA molecules.

In preferred embodiments, the vector utilized includes a prokaryotic replicon i.e., a DNA sequence having the ability to direct autonomous replication and maintenance of the recombinant DNA molecule extra chromosomally in a prokaryotic host cell, such as a bacterial host cell, transformed therewith. Such replicons are well known in the art. In addition, those embodiments that include a prokaryotic replicon also include a gene whose expression confers a selective advantage, such as drug resistance, to a bacterial host transformed therewith. Typical bacterial drug resistance genes are those that confer resistance to ampicillin or tetracycline.

Those vectors that include a prokaryotic replicon can also include a prokaryotic promoter capable of directing the expression (transcription and translation) of the V_{H}- and/or V_{L}-coding homologs in a bacterial host cell, such as E. coli transformed therewith. A promoter is an expression control element formed by a DNA sequence that permits binding of RNA polymerase and transcription to occur. Promoter sequences compatible with bacterial hosts are typically provided in plasmid vectors containing convenience restriction sites for insertion of a DNA segment of the present invention. Typical of such vector plasmids are pUC8, pUC9, pBR322, and pBR329 available from BioRad Laboratories, (Richmond, CA) and pPL and pKK223 available from Pharmacia, (Piscataway, NJ).

Expression vectors compatible with eukaryotic cells, preferably those compatible with vertebrate cells, can also be used. Eukaryotic cell expression vectors are well known in the art and are available from several commercial sources. Typically, such vectors are provided containing convenient restriction sites for insertion of the desired DNA homolog. Typical of such vectors are pSVL and pKSV-10 (Pharmacia), pBPV-1/PML2d (International Biotechnologies, Inc.), and pTDT1 (ATCC, No. 31255).

In preferred embodiments, the eukaryotic cell expression vectors used include a selection marker that is effective in an eukaryotic cell, preferably a drug resistant selection marker. A preferred drug resistance marker is the gene whose expression results in neomycin resistance, i.e., the neomycin phosphotransferase (neo) gene. Southern et al., J. Mol. Appl. Genet., 1:327-341 (1982).

The use of retroviral expression vectors to express the genes of the V_{H} and/or V_{L}-coding. DNA homologs is also contemplated. As used herein, the term "retroviral expression vector" refers to a DNA molecule that includes a promoter sequences derived from the long terminal repeat (LTR) region of a retrovirus genome.

In preferred embodiments, the expression vector is typically a retroviral expression vector that is preferably replication-incompetent in eukaryotic cells. The construction and use of retroviral vectors has been described by Sorge et al., Mol. Cel. Biol., 4:1730-1737 (1984).

A variety of methods have been developed to operatively link DNA to vectors via complementary cohesive termini. For instance, complementary cohesive termini can be engineered into the V_{H}- and/or V_{L}-coding DNA homologs during the primer extension reaction by use of an appropriately designed polynucleotide synthesis primer, as previously discussed. The vector, and DNA homolog if necessary, is cleaved with a restriction endonuclease to produce termini complementary to those of the DNA homolog. The complementary cohesive termini of the vector and the DNA homolog are then operatively linked (ligated) to produce a unitary double stranded DNA molecule.

In preferred embodiments, theV_{H}-coding and V_{L}-coding DNA homologs of diverse libraries are randomly combined in vitro for polycistronic expression from individual vectors. That is, a diverse population of double stranded DNA expression vectors is produced wherein each vector expresses, under the control of a single promoter, one V_{H}-coding DNA homolog and one V_{L}-coding DNA homolog, the diversity of the population being the result of different V_{H}- and V_{L}-coding DNA homolog combinations. Random combination in vitro can be accomplished using two expression vectors distinguished from one another by the location on each of a restriction site common to both. Preferably the vectors are linear double stranded DNA, such as a Lambda Zap derived vector as described herein. In the first vector, the site is located between a promoter and a polylinker, i.e., 5' terminal (upstream relative to the direction of expression) to the polylinker but 3' terminal (downstream relative to the direction of expression). In the second vector, the polylinker is located between a promoter and the restriction site, i.e., the restriction site is located 3' terminal to the polylinker, and the polylinker is located 3' terminal to the promoter.

In preferred embodiments, each of the vectors defines a nucleotide sequence coding for a ribosome binding and a leader, the sequence being located between the promoter and the polylinker, but downstream (3' terminal) from the shared restriction site if that site is between the promoter and polylinker. Also preferred are vectors containing a stop codon downstream from the polylinker, but upstream from any shared restriction site if that site is downstream from the polylinker. The first and/or second vector can also define a nucleotide sequence coding for a peptide tag. The tag sequence is typically located downstream from the polylinker but upstream from any stop codon that may be present. In preferred embodiments, the vectors contain selectable markers such that the presence of a portion of that vector, i.e. a particular lambda arm, can be selected for or selected against. Typical selectable markers are well known to those skilled in the art. Examples of such markers are antibiotic resistance genes, genetically selectable markers, mutation suppressors such as amber suppressors and the like. The selectable markers are typically located upstream of the promoter and/or downstream of the second restriction site. In preferred embodiments, one selectable marker is located upstream of the promoter on the first vector containing the V_{H}-coding DNA homologs. A second selectable marker is located downstream of the second restriction site on the vector containing the V_{L}-coding DNA homologs. This second selectable marker may be the same or different from the first as long as when the V_{H}-coding vectors and the V_{L}-coding vectors are randomly combined via the first restriction site the resulting vectors containing both V_{H} and V_{L} and both selectable markers can be selected.

Typically the polylinker is a nucleotide sequence that defines one or more, preferably at least two, restriction sites, each unique to the vector and preferably not shared by the other vector, i.e., if it is on the first vector, it is not on the second vector. The polylinker restriction sites are oriented to permit libation of V_{H}- or V_{L}-coding DNA homologs into the vector in same reading frame as any leader, tag or stop codon sequence present.

Random combination is accomplished by ligating V_{H}-coding DNA homologs into the first vector, typically at a restriction site or sites within the polylinker. Similarly, V_{L}-coding DNA homologs are ligated into the second vector, thereby creating two diverse populations of expression vectors. It does not matter which type of DNA homolog, i.e., V_{H} or V_{L} is ligated to which vector, but it is preferred, for example, that all V_{H}-coding DNA homologs are ligated to either the first or second vector, and all of the V_{L}-coding DNA homologs are ligated to the other of the first or second vector. The members of both populations are then cleaved with an endonuclease at the shared restriction site, typically by digesting both populations with the same enzyme. The resulting product is two diverse populations of restriction fragments where the members of one have cohesive termini complementary to the cohesive termini of the members of the other. The restriction fragments of the two populations are randomly ligated to one another, i.e., a random, interpopulation ligation is performed, to produce a diverse population of vectors each having a V_{H}-coding and V_{L}-coding DNA homolog located in the same reading frame and under the control of second vector's promoter. Of course, subsequent recombinations can be effected through cleavage at the shared restriction site, which is typically reformed upon ligation of members from the two populations, followed by subsequent religations.

The resulting construct is then introduced into an appropriate host to provide amplification and/or expression of the V_{H}- and/or V_{L}-coding DNA homologs, either separately or in combination. When coexpressed within the same organism, either on the same or the different vectors, a functionally active Fv is produced. When the V_{H} and V_{L} polypeptides are expressed in different organisms, the respective polypeptides are isolated and then combined in an appropriate medium to form a Fv. Cellular hosts into which a V_{H}- and/or V_{L}-coding DNA homolog-containing construct has been introduced are referred to herein as having been "transformed" or as "transformants".

The host cell can be either prokaryotic or eukaryotic. Bacterial cells are preferred prokaryotic host cells and typically are a strain of E. coli such as, for example, the E. coli strain DH5 available from Bethesda Research Laboratories, Inc., Bethesda, MD. Preferred eukaryotic host cells include yeast and mammalian cells, preferably vertebrate cells such as those from a mouse, rat, monkey or human cell line.

Transformation of appropriate cell hosts with a recombinant DNA molecule of the present invention is accomplished by methods that typically depend on the type of vector used. With regard to transformation of prokaryotic host cells, see, for example, Cohen et al., Proc. Natl. Acad. Sci., USA, 69:2110 (1972); and Maniatis et al., Molecular Cloning: A Laboratory Manual, Cold Spring Harbor, NY (1982). With regard to the transformation of vertebrate cells with retroviral vectors containing rDNAs, see for example, Sorge et al., Mol. Cell. Biol., 4:1730-1737 (1984); Graham et al., Virol., 52:456 (1973); and Wigler et al., Proc. Natl. Acad. Sci., USA, 76:1373-1376 (1979).

### 5. Screening For Expression of V_{H} and/or V_{L} Polypeptides

Successfully transformed cells, i.e., cells containing a V_{H}- and/or V_{L}-coding DNA homolog operatively linked to a vector, can be identified by any suitable well known technique for detecting the binding of a receptor to a ligand or the presence of a polynucleotide coding for the receptor, preferably its active site. Preferred screening assays are those where the binding of ligand by the receptor produces a detectable signal, either directly or indirectly. Such signals include, for example, the production of a complex, formation of a catalytic reaction product, the release or uptake of energy, and the like. For example, cells from a population subjected to transformation with a subject rDNA can be cloned to produce monoclonal colonies. Cells form those colonies can be harvested, lysed and their DNA content examined for the presence of the rDNA using a method such as that described by Southern, J. Mol. Biol., 98:503 (1975) or Berent et al., Biotech., 3:208 (1985).

In addition to directly assaying for the presence of a V_{H}- and/or V_{L}-coding DNA homolog, successful transformation can be confirmed by well known immunological methods, especially when the V_{H} and/or V_{L} polypeptides produced contain a preselected epitope. For example, samples of cells suspected of being transformed are assayed for the presence of the preselected epitope using an antibody against the epitope.

### 6. V_{H}- And/Or V_{L}-Coding Gene Libraries

The present invention contemplates a gene library, preferably produced by a primer extension reaction or combination of primer extension reactions as described herein, containing at least about 10³, preferably at least about 10⁴ and more preferably at least about 10⁵ different V_{H}- and/or V_{L}-coding DNA homologs. The homologs are preferably in an isolated form, that is, substantially free of materials such as, for example, primer extension reaction agents and/or substrates, genomic DNA segments, and the like.

In preferred embodiments, a substantial portion of the homologs present in the library are operatively linked to a vector, preferably operatively linked for expression to an expression vector.

Preferably, the homologs are present in a medium suitable for in vitro manipulation, such as water, water containing buffering salts, and the like. The medium should be compatible with maintaining the biological activity of the homologs. In addition,the homologs should be present at a concentration sufficient to allow transformation of a host cell compatible therewith at reasonable frequencies.

It is further preferred that the homologs be present in compatible host cells transformed therewith.

### D. Expression Vectors

The present invention also contemplates various expression vectors useful in performing, inter alia, the methods of the present invention. Each of the expression vectors is a novel derivative of Lambda Zap.

### 1. Lambda Zap II

Lambda Zap II is prepared by replacing the Lambda S gene of the vector Lambda Zap with the Lambda S gene from the Lambda gt10 vector, as described in Example 6.

### 2. Lambda Zap II V_{H}

Lambda Zap II V_{H} is prepared by inserting the synthetic DNA sequences illustrated in Figure 6A into the above-described Lambda Zap II vector. The inserted nucleotide sequence advantageously provides a ribosome binding site (Shine-Dalgarno sequence) to permit proper imitation of mRNA translation into protein, and a leader sequence to efficiently direct the translated protein to the periplasm. The preparation of Lambda Zap II V_{H} is described in more detail in Example 9, and its features illustrated in Figures 6A and 7.

### 3. Lambda Zap II V_{L}

Lambda Zap II V_{L} is prepared as described in Example 12 by inserting into Lambda Zap II the synthetic DNA sequence illustrated in Figure 6B. Important features of Lambda Zap II V_{L} are illustrated in Figure 8.

### 4. Lambda Zap II V_{L} II

Lambda Zap II V_{L} II is prepared as described in Example 11 by inserting into Lambda Zap II the synthetic DNA sequence illustrated in Figure 10.

The above-described vectors are compatible with E. coli hosts, i.e., they can express for secretion into the periplasm proteins coded for by genes to which they have been operatively linked for expression.

### Examples

The following examples are intended to illustrate, but not limit, the scope of the invention.

### 1. Polynucleotide Selection

The nucleotide sequences encoding the immunoglobulin protein CDR's are highly variable. However, there are several regions of conserved sequences that flank the V_{H} domains. For instance, contain substantially conserved nucleotide sequences, i.e., sequences that will hybridize to the same primer sequence. Therefore, polynucleotide synthesis (amplification) primers that hybridize to the conserved sequences and incorporate restriction sites into the DNA homolog produced that are suitable for operatively linking the synthesized DNA fragments to a vector were constructed. More specifically, the DNA homologs were inserted into Lambda ZAP II vector (Stratagene Cloning System, La Jolla, CA) at the Xho I and EcoR I sites. For amplification of the V_{H} domains, the 3' primer (primer 12 in Table 1), was designed to be complementary to the mRNA in the J_{H} region. In all cases, the 5' primers (primers 1-10, Table 1) were chosen to be complementary to the first strand cDNA in the conserved N-terminus region (antisense strand). Initially amplification was performed with a mixture of 32 primers (primer 1, Table 1) that were degenerate at five positions. Hybridoma mRNA could be amplified with mixed primers, but initial attempts to amplify mRNA from spleen yielded variable results. Therefore, several alternatives to amplification using the mixed 5' primers were compared.

The first alternative was to construct multiple unique primers, eight of which are shown in Table 1, corresponding to individual members of the mixed primer pool. The individual primers 2-9 of Table 1 were constructed by incorporating either of the two possible nucleotides at three of the five degenerate positions.

The second alternative was to construct a primer containing inosine (primer 10, Table 1) at four of the variable positions based on the published work of Takahashi, et al., Proc. Natl. Acad. Sci. (U.S.A.), 82:1931-1935, (1985) and Ohtsuka et al., J. Biol. Chem., 260: 2605-2608, (1985). This primer has the advantage that it is not degenerate and, at the same time minimizes the negative effects of mismatches at the unconserved positions as discussed by Martin et al., Nuc. Acids Res., 13:8927 (1985). However, it was not known if the presence of inosine nucleotides would result in incorporation of unwanted sequences in the cloned V_{H} regions. Therefore, inosine was not included at the one position that remains in the amplified fragments after the cleavage of the restriction sites. As a result, inosine was not in the cloned insert.

Additional V_{H} amplification primers including the unique 3' primer were designed to be complementary to a portion of the first constant region domain of the gamma 1 heavy chain mRNA (primers 15 and 16, Table 1). These primers will produce DNA homologs containing polynucleotides coding for amino acids from the V_{H} and the first constant region domains of the heavy chain. These DNA homologs can therefore be used to produce Fab fragments rather than an F_{V}.

Additional unique 3' primers designed to hybridize to similar regions of another class of immunoglobulin heavy chain such as IgM, IgE and IgA are contemplated. Over 3' primers that hybridize to a specific region of a specific class of CH₁ constant region and are adapted for transferring the V_{H} domains amplified using this primer to an expression vector capable of expressing those V_{H} domains with a different class of heavy or light chain constant region is also contemplated.

As a control for amplification from spleen or hybridoma mRNA, a set of primers hybridizing to a highly conserved region within the constant region IgG, heavy chain gene were constructed. The 5' primer (primer 11, Table 1) is complementary to the cDNA in the C_{H}2 region whereas the 3' primer (primer 13, Table 1) is complementary to the mRNA in the C_{H}3 region. It is believed that no mismatches were present between these primers and their templates.

The nucleotide sequences encoding the V_{L} CDRs are highly variable. However, there are several regions of conserved sequences that flank the V_{L} CDR domains including the J_{L}, V_{L} framework regions and V_{L} leader/promotor. Therefore, amplification primers that hybridize to the conserved sequences and incorporate restriction sites that allowing cloning the amplified fragments into the pBluescript SK- vector cut with Nco I and Spe I were constructed. For amplification of the V_{L} CDR domains, the 3' primer (primer 14 in Table 1), was designed to be complementary to the mRNA in the J_{L} regions. The 5' primer (primer 15, Table 1) was chosen to be complementary to the first strand cDNA in the conserved N-terminus region (antisense strand).

A second set of amplification primers for amplification of the V_{L} CDR domains the 5' primers (primers 1-8 in Table 2) were designed to be complementary to the first strand cDNA in the conserved N-terminus region. These primers also introduced a Sac I restriction endonuclease site to allow the V_{L} DNA homolog to be cloned into the V_{L}II-expression vector. The 3' V_{L} amplification primer (primer 9 in Table 2) was designed to be complementary to the mRNA in the J_{L} regions and to introduce the Xba I restriction endonuclease site required to insert the V_{L} DNA homolog into the V_{L}II-expression vector (Figure a).

Additional 3' V_{L} amplification primers were designed to hybridize to the constant region of either kappa or lambda mRNA (primers 10 and 11 in Table 2). These primers allow a DNA homolog to be produced containing polynucleotide sequences coding for constant region amino acids of either kappa or lambda chain. These primers make it possible to produce an Fab fragment rather than an Fᵥ.

The primers used for amplification of kappa light chain sequences for construction of Fabs are shown at least in Table 2. Amplification with these primers was performed in 5 separate reactions, each containing one of the 5' primers (primers 3-6, and 12) and one of the 3' primers (primer 13). The remaining 3' primer (primer 9) has been used to construct Fᵥ fragments. The 5' primers contain a Sac I restriction site and the 3' primers contain a Xba I restriction site.

The primers used for amplification of heavy chain Fd fragments for construction of Fabs are shown at least in Table 1. Amplification was performed in eight separate reactions, each containing one of the 5' primers (primers 2-9) and one of the 3' primers (primer 15). The remaining 5' primers that have been used for amplification in a single reaction are either a degenerate primer (primer 1) or a primer that incorporates inosine at four degenerate positions (primer 10, Table 1, and primers 17 and 18, Table 2). The remaining 3' primer (primer 14, Table 2) has been used to construct Fᵥ fragments. Many of the 5' primers incorporate a Xho I site, and the 3' primers incorporate a Spe I restriction site.

V_{H} amplification primers designed to amplify human heavy chain variable regions are shown in Table 2. One of the 5' heavy chain primer contains inosine residues at degenerate nucleotide positions allowing a single primer to hybridize to a large number of variable region sequences. Primers designed to hybridize to the constant region sequences of various IgG mRNAs are also shown in Table 2.

V_{L} amplification primers designed to amplify human light chain variable regions of both the lambda and kappa isotypes are also shown in Table 2.

All primers and synthetic polynucleotides used herein and shown on Tables 1-4 were either purchased from Research Genetics in Huntsville, Alabama or synthesized on an Applied Biosystems DNA synthesizer, model 381A, using the manufacturer's instruction.

**TABLE 1**

| | | |
|---|---|---|
| (1) | 5'AGGT(C/G)(C/A)A(G/A)CT(G/T)CTCGAGTC(T/A)GG 3' | degenerate 5' primer for the amplification of mouse and human heavy chain variable regions (V_{H}) |
| | | |
| (2) | 5'AGGTCCAGCTGCTCGAGTCTGG 3' | Unique 5' primer for the amplification of mouse and human V_{H} |
| (3) | 5'AGGTCCAGCTGCTCGAGTCAGG 3' | " |
| (4) | 5'AGGTCCAGCTTCTCGAGTCTGG 3' | " |
| (5) | 5'AGGTCCAGCTTCTCGAGTCAGG 3' | " |
| (6) | 5'AGGTCCAACTGCTCGAGTCTGG 3' | " |
| (7) | 5'AGGTCCAACTGCTCGAGTCAGG 3' | " |
| (8) | 5'AGGTCCAACTTCTCGAGTCTGG 3' | " |
| (9) | 5'AGGTCCAACTTCTCGACTCAGG 3' | " |
| | | |
| (10) | 5'AGGTIIAICTICTCGAGTC(T/A)GG 3' | 5' degenerate primer containing inosine at 4 degenerate positions for amplification of mouse V_{H} |
| | | |
| (11) | 5'GCCCAAGGATGTGCTCACC 3' | 5' primer for amplification in the C_{H}2 region of mouse IgG1 |
| | | |
| (12) | 5'CTATTAGAATTCAACGGTAACAGTGGTGCCTTGGCCCCA 3' | 3' primer for amplification of V_{H} and introducing a 3' Eco RI site |
| | | |
| (12A) | 5'CTATTAACTAGTAACGGTAACAGTGGTGCCTTGGCCCCA 3' | 3' primer for amplification of V_{H} using 3' Spe I site |
| | | |
| (13) | 5'CTCAGTATGGTGGTTGTGC 3' | 3' primer for amplification in the C_{H}3 region of mouse IgG1 |
| | | |
| (14) | 5'GCTACTAGTTTTGATTTCCACCTTGG 3' | 3' primer for amplification of mouse kappa light chain variable regions (V_{L}) |
| | | |
| (15) | 5'CAGCCATGGCCGACATCCAGATG 3' | 5' primer for amplification of mouse kappa light chain variable regions |
| (16) | 5'AATTTTACTAGTCACCTTGGTGCTGCTGGC 3' | Unique 3' primer for amplification of V_{H} including part of the mouse gamma 1 first constant region |
| | | |
| (17) | 5'TATGCAACTAGTACAACCACAATCCCTGGGCACAATTTT 3' | Unique 3' primer for amplification of Fd including part of mouse IgG1 first constant region and hinge region |
| | | |
| (18) | 5'AGGCTTACTAGTACAATCCCTGGGCACAAT 3' | 3' primer for amplifying mouse Fd includinc part of the mouse IgG first constant reglor and part of the hinge region |

**TABLE 2**

| | | |
|---|---|---|
| (1) | 5' CCAGTTCCGAGCTCGTTGTGACTCAGGAATCT 3' | Unique 5' primer for the amplificatior of kappa light chain variable regions |
| (2) | 5' CCAGTTCCGAGCTCGTGTTGACGCAGCCGCCC 3' | " " |
| (3) | 5' CCAGTTCCGAGCTCGTGCTCACCCAGTCTCCA 3' | " " |
| (4) | 5' CCAGTTCCGAGCTCCAGATGACCCAGTCTCCA 3' | " " |
| (5) | 5' CCAGATGTGAGCTCGTGATGACCCAGACTCCA 3' | " " |
| (6) | 5' CCAGATGTGAGCTCGTCATGACCCAGTCTCCA 3' | " " |
| (7) | 5' CCAGATGTGAGCTCTTGATGACCCAAACTCAA 3' | " " |
| (8) | 5' CCAGATGTGAGCTCGTGATAACCCAGGATGAA 3' | " " |
| | | |
| (9) | 5' GCAGCATTCTAGAGTTTCAGCTCCAGCTTGCC 3' | Unique 3' primer for amplification of kappa light chain variable regions |
| | | |
| (10) | 5' CCGCCGTCTAGAACACTCATTCCTGTTGAAGCT 3' | Unique 3' primer for mouse kappa light chain amplification including the constant region |
| | | |
| (11) | 5' CCGCCGTCTAGAACATTCTGCAGGAGACAGACT 3' | Unique 3' primer for mouse lambda ligt chain amplification including the constant region |
| | | |
| (12) | 5' CCAGTTCCGAGCTCGTGATGACACAGTCTCCA 3' | Unique 5' primer for V_{L} amplification |
| | | |
| (13) | 5' GCGCCGTCTAGAATTAACACTCATTCCTGTTGAA 3' | Unique 3' primer for amplification of kappa light chain |
| | | |
| (14) | 5' CTATTAACTAGTAACGGTAACAGTGGTGCCTTGCCCCA 3' | Unique 3' primer for amplification of mouse F_{V} |
| | | |
| (15) | 5' AGGCTTACTAGTACAATCCCTGGGCACAAT 3' | Unique 3' primer for amplification of mouse IgG Fd |
| | | |
| (16) | 5' GCCGCTCTAGAACACTCATTCCTGTTGAA 3' | Unique 3' primer for amplification of mouse kappa light chain |
| (17) | 5' AGGTIIAICTICTCGAGTCTGC 3' | Degenerate 5' primer containing inosine at 4 degenerate positions for amplifying mouse V_{H} |
| | | |
| (18) | 5' AGGTIIAICTICTCGAGTCAGC 3' | " " " |
| | | |
| (19) | 5' GTGCCAGATGTGAGCTCGTGATGACCCAGTCTCCA 3' | Unique 5' primer for human and mouse kappa V_{L} amplification |
| | | |
| (20) | 5' TCCTTCTAGATTACTAACACTCTCCCCTGTTGAA 3' | Unique 3' primer for kappa V_{L} amplification |
| | | |
| (21) | 5' GCATTCTAGACTATTATGAACATTCTGTAGGGGC 3' | Unique 3' primer for human, mouse and rabbit lambda V_{L} amplification |
| | | |
| (22) | 5' CTGCACAGGGTCCTGGGCCGAGCTCGTGGTGACTCAG 3' | Unique 5' primer for human lambda V_{L} amplification |
| | | |
| (23) | 5' AGITGCAIITGCTCGAGTCTGG 3' | 5' degenerate primer for human V_{H} amplification containing inosine at 3 degenerate positions |
| | | |
| (24) | 5' GTGGGCATGTGTGAGTTGTGTCACTAGTTGGGGTTTTGAGCTC 3' | Unique 3' primer for human V_{H} amplification |
| | | |
| (25) | 5' AGCATCACTAGTACAAGATTTGGGCTC 3' amplification | Unique 3' primer for human IgG1 Fd |
| | | |
| (26) | 5' AGGTGCAGCTGCTCGAGTCTGG 3' | Unique 5' primers for amplification of human variable regions (V_{H}) |
| | | |
| (27) | 5' AGGTGCAGCTGCTCGAGTCGGG 3' | " " |
| | | |
| (28) | 5' AGGTGCAACTGCTCGAGTCTGG 3' | " " |
| | | |
| (29) | 5' AGGTGCAACTGCTCGAGTCGGG 3' | " " |
| (30) | 5' TCCTTCTAGATTACTAACACTCTCCCCTGTTGAA 3' | 3' primer in human kappa light chain constant region |
| | | |
| (31) | 5' CTGCACAGGGTCCTGGGCCGAGCTCGTGGTGACTCAG 3' | 5' primer for amplification of human lambda light chain variable regions |
| | | |
| (32) | 5' GCATTCTAGACTATTAACATTCTGTAGGGGC 3' | 3' primer in human lambda light chain constant region |
| | | |
| (33) | 5' ACCCAAGGACACCCTCATG 3' | Control primer hybridizing to the human CH₂ region |
| | | |
| (34) | 5' CTCAGTATGGTGGTTGTGC 3' | Control primer hybridizing to the human CH₃ region |
| | | |
| (35) | 5' GTCTCACTAGTCTCCACCAAGGGCCCATCGGTC 3' | 5' primer for amplifying human IgG heavy chain first constant region |
| | | |
| (36) | 5' ATATACTAGTGAGACAGTGACCAGGGTTCCTTGGCCCCA 3' | 3' primer for amplifying human heavy chain variable regions |
| | | |
| (37) | 5' ACGTCTAGATTCCACCTTGGTCCC 3' | 3' primer for amplifying human kappa chain variable regions |
| | | |
| (38) | 5' GCATACTAGTCTATTAACATTCTGTAGGGGC 3' | 5' primer for amplifying human kappa light chain constant region |
| | | |
| (39) | 5' CCGGAATTCTTATCATTTACCCGGAGA 3' | 3' primer located in the CH3 region of human IgG1 to amplify the entire heavy chain |
| | | |
| (40) | 5' TCTGCACTAGTTGGAATGGGCACATGCAG 3' | 3' primer for amplifying the Fd region of mouse IgM |

### 2. Production Of A V_{H} Coding Repertoire Enriched In FITC Binding Proteins

Fluorescein isothiocyanate (FITC) was selected as a ligand for receptor binding. It was further decided to enrich by immunization the immunological gene repertoire, i.e., V_{H}- and V_{L}-coding gene repertoires, for genes coding for anti-FITC receptors. This was accomplished by linking FITC to keyhole limpet hemocyanin (KLH) using the techniques described in Antibodies A Laboratory Manual, Harlow and Lowe, eds., Cold Spring Harbor, NY, (1988). Briefly, 10.0 milligrams (mg) of keyhole limpet hemocyanin and 0.5 mg of FITC were added to 1 ml of buffer containing 0.1 M sodium carbonate at pH 9.6 and stirred for 18 to 24 hours at 4 degrees C (4C). The unbound FITC was removed by gel filtration through Sephadex G-25.

The KLH-FITC conjugate was prepared for injection into mice by adding 100 µg of the conjugate to 250 µl of phosphate buffered saline (PBS). An equal volume of complete Freund's adjuvant was added and emulsified the entire solution for 5 minutes. A 129 G_{IX+} mouse was injected with 300 µl of the emulsion. Injections were given subcutaneously at several sites using a 21 gauge needle. A second immunization with KLH-FITC was given two weeks later. This injection was prepared as follows: fifty µg of KLH-FITC were diluted in 250 µL of PBS and an equal volume of alum was admixed to the KLH-FITC solution. The mouse was injected intraperitoneally with 500 µl of the solution using a 23 gauge needle. One month later the mice were given a final injection of 50 µg of the KLH-FITC conjugate diluted to 200 µL in PBS. This injection was given intravenously in the lateral tail vein using a 30 gauge needle. Five days after This final injection the mice were sacrificed and total cellular RNA was isolated from their spleens.

Hybridoma PCP 8D11 producing an antibody immunospecific for phosphonate ester was cultured in DMEM media (Gibco Laboratories, Grand Island, NY) containing 10 percent fetal calf serum supplemented with penicillin and streptomycin. About 5 x 10⁸ hybridoma cells were harvested and washed twice in phosphate buffered saline. Total cellular RNA was prepared from these isolated hybridoma cells.

### 3. Preparation Of A V_{H}-Coding Gene Repertoire

Total cellular RNA was prepared from the spleen of a single mouse immunized with KLH-FITC as described in Example 2 using the RNA preparation methods described by Chomczynski et al., Anal Biochem., 162:156-159 (1987)using the manufacturer's instructions and the RNA isolation kit produced by Stratagene Cloning Systems, La Jolla, CA. Briefly, immediately after removing the spleen from the immunized mouse, the tissue was homogenized in 10 ml of a denaturing solution containing 4.0 M guanine isothiocyanate, 0.25 M sodium citrate at pH 7.0, and 0.1 M 2-mercaptoethanol using a glass homogenizer. One ml of sodium acetate at a concentration of 2 M at pH 4.0 was admixed with the homogenized spleen. One ml of phenol that had been previously saturated with H₂O was also admixed to the denaturing solution containing the homogenized spleen. Two ml of a chloroform:isoamyl alcohol (24:1 v/v) mixture was added to this homogenate. The homogenate was mixed vigorously for ten seconds and maintained on ice for 15 minutes. The homogenate was then transferred to a thick-walled 50 ml polypropylene centrifuged tube (Fisher Scientific Company, Pittsburg, PA). The solution was centrifuged at 10,000 x g for 20 minutes at 4C. The upper RNA-containing aqueous layer was transferred to a fresh 50 ml polypropylene centrifuge tube and mixed with an equal volume of isopropyl alcohol. This solution was maintained at -20C for at least one hour to precipitate the RNA. The solution containing the precipitated RNA was centrifuged at 10,000 x g for twenty minutes at 4C. The pelleted total cellular RNA was collected and dissolved in 3 ml of the denaturing solution described above. Three ml of isopropyl alcohol was added to the re-suspended total cellular RNA and vigorously mixed. This solution was maintained at -20C for at least 1 hour to precipitate the RNA. The solution containing the precipitated RNA was centrifuged at 10,000 x g for ten minutes at 4C. The pelleted RNA was washed once with a solution containing 75% ethanol. The pelleted RNA was dried under vacuum for 15 minutes and then re-suspended in dimethyl pyrocarbonate (DEPC) treated (DEPC-H₂O) H₂O.

Messenger RNA (mRNA) enriched for sequences containing long poly A tracts was prepared from the total cellular RNA using methods described in Molecular Cloning A Laboratory Manual, Maniatias et al., eds., Cold Spring Harbor, NY, (1982). Briefly, one half of the total RNA isolated from a single immunized mouse spleen prepared as described above was re-suspended in one ml of DEPC-H₂O and maintained at 65C for five minutes. One ml of 2x high salt loading buffer consisting of 100 mM Tris-HCl, 1 M sodium chloride, 2.0 mM disodium ethylene diamine tetra-acetic acid (EDTA) at pH 7.5, and 0.2% sodium dodecyl sulfate (SDS) was added to the re-suspended RNA and the mixture allowed to cool to room temperature. The mixture was then applied to an oligo-dT (Collaborative Research Type 2 or Type 3) column that was previously prepared by washing the oligo-dT with a solution containing 0.1 M sodium hydroxide and 5 mM EDTA and then equilibrating the column with DEPC-H₂O. The eluate was collected in a sterile polypropylene tube and reapplied to the same column after heating the eluate for 5 minutes at 65C. The oligo dT column was then washed with 2 ml of high salt loading buffer consisting of 50 mM Tris-HCl at pH 7.5, 500 mM sodium chloride, 1 mM EDTA at pH 7.5 and 0.1% SDS. The oligo dT column was then washed with 2 ml of 1 X medium salt buffer consisting of 50 mM Tris-HCl at pH 7.5, 100 mM sodium chloride 1 mM EDTA and 0.1% SDS. The messenger RNA was eluted from the oligo dT column with 1 ml of buffer consisting of 10 mM Tris-HCl at pH 7.5, 1 mM EDTA at pH 7.5 and 0.05% SDS. The messenger RNA was purified by extracting this solution with phenol/chloroform followed by a single extraction with 100% chloroform. The messenger RNA was concentrated by ethanol precipitation and re-suspended in DEPC H₂O.

The messenger RNA isolated by the above process contains a plurality of different V_{H} coding polynucleotides, i.e., greater than about 10⁴ different V_{H}-coding genes.

### 4. Preparation Of A Single V_{H} Coding Polynucleotide

Polynucleotides coding for a single V_{H} were isolated according to Example 3 except total cellular RNA was extracted from monoclonal hybridoma cells prepared in Example 2. The polynucleotides isolated in this manner code for a single V_{H}.

### 5. DNA Homolog Preparation

In preparation for PCR amplification, mRNA prepared according to the above examples was used as a template for cDNA synthesis by a primer extension reaction. In a typical 50 µl transcription reaction, 5-10 ug of spleen or hybridoma mRNA in water was first hybridized (annealed) with 500 ng (50.0 pmol) of the 3' V_{H} primer (primer 12, Table 1), at 65C for five minutes. Subsequently, the mixture was adjusted to 1.5 mM dATP, dCTP, dGTP and dTTP, 40 mM Tris-HCl at pH 8.0, 8 mM MgCl₂, 50 mM NaCl, and 2 mM spermidine. Moloney-Murine Leukemia virus Reverse transcriptase (Stratagene Cloning Systems), 26 units, was added and the solution was maintained for 1 hour at 37C.

PCR amplification was performed in a 100 µl reaction containing the products of the reverse transcription reaction (approximately 5 ug of the cDNA/RNA hybrid), 300 ng of 3' V_{H} primer (primer 12 of Table 1), 300 ng each of the 5' V_{H} primers (primer 2-10 of Table 1) 200 mM of a mixture of dNTP's, 50 mM KCl, 10 mM Tris-HCl pH 8.3, 15 mM MgCl₂, 0.1% gelatin and 2 units of Tag DNA polymerase. The reaction mixture was overlaid with mineral oil and subjected to 40 cycles of amplification. Each amplification cycle involved denaturation at 92C for 1 minute, annealing at 52C for 2 minutes and polynucleotide synthesis by Primer extension (elongation) at 72C for 1.5 minutes. The amplified V_{H}-coding DNA homolog containing samples were extracted twice with phenol/chloroform, once with chloroform, ethanol precipitated and were stored at -70C in 10 mM Tris-HCl, (pH, 7.5) and 1 mM EDTA.

Using unique 5' primers (2-9, Table 1), efficient V_{H}-coding DNA homolog synthesis and amplification from the spleen mRNA was achieved as shown in Figure 3,- lanes R17-R24. The amplified cDNA (V_{H}-coding DNA homolog) is seen as a major band of the expected size (360 bp). The intensities of the amplified V_{H}-coding polynucleotide fragment in each reaction appear to be similar, indicating that all of these primers are about equally efficient in initiating amplification. The yield and quality of the amplification with these primers was reproducible.

The primer containing inosine also synthesized amplified V_{H}-coding DNA homologs from spleen mRNA reproducibly, leading to the production of the expected sized fragment, of an intensity similar to that of the other amplified cDNAs (Figure 4, Lane R16). This result indicated that the presence of inosine also permits efficient DNA homolog synthesis and amplification. Clearly indicating how useful such primers are in generating a plurality of V_{H}-coding DNa homologs. Amplification products obtained from the constant region primers (primers 11 and 13, Table 1) were more intense indicating that amplification was more efficient, possibly because of a higher degree of homology between the template and primers (Figure 4, Lane R9). Based on these results, a V_{H}-coding gene library was constructed from the products of eight amplifications, each performed with a different 5' primer. Equal portions of the products from each primer extension reaction were mixed and the mixed product was then used to generate a library of V_{H}-coding DNA homolog-containing vectors.

DNA homologs of the V_{L} were prepared from the purified mRNA prepared as described above. In preparation for PCR amplification, mRNA prepared according to the above examples was used as a template for cDNA synthesis. In a typical 50 µl transcription reaction, 5-10 ug of spleen or hybridoma mRNA in water was first annealed with 300 ng (50.0 pmol) of the 3' V_{L} primer (primer 14, Table 1), at 65C for five minutes. Subsequently, the mixture was adjusted to 1.5 mM dATP, dCTP, dGTP, and dTTP, 40 mM Tris-HCl at pH 8.0, 8 mM MgCl₂, 50 mM NaCl, and 2 mM spermidine. Moloney-Murine Leukemia virus reverse transcriptase (Stratagene Cloning Systems), 26 units, was added and the solution was maintained for 1 hour at 37C. The PCR amplification was performed in a 100 µl reaction containing approximately 5 ug of the cDNA/RNA hybrid produced as described above, 300 ng of the 3' V_{L} primer (primer 14 of Table 1), 300 ng of the 5' V_{L} primer (primer 15 of Table 1), 200 mM of a mixture of dNTP's, 50 mM KC1, 10 mM Tris-HCl pH 8.3, 15 mM MgCl₂, 0.1% gelatin and 2 units of Taq DNA polymerase. The reaction mixture was overlaid with mineral oil and subjected to 40 cycles of amplification. Each amplification cycle involved denaturation at 92C for 1 minute, annealing at 52C for 2 minutes and elongation at 72C for 1.5 minutes. The amplified samples were extracted twice with phenol/chloroform, once with chloroform, ethanol precipitated and were stored at -70C in 10 mM Tris-HCl at 7.5 and 1 mM EDTA.

### 6. Inserting DNA Homologs Into Vectors

In preparation for cloning a library enriched in V_{H} sequences, PCR amplified products (2.5 mg/30 µl of 150 mM NaCl, 8 mM Tris-HCl (pH 7.5), 6 mM MgSO₄, 1 mM DTT, 200 mg/ml bovine serum albumin (BSA) at 37C were digested with restriction enzymes Xho I (125 units) and EcoR I (10 U) and purified on a 1% agarose gel. In cloning experiments which required a mixture of the products of the amplification reactions, equal volumes (50 µl, 1-10 ug concentration) of each reaction mixture were combined after amplification but before restriction digestion. After gel electrophoresis of the digested PCR amplified spleen mRNA, the region of the gel containing DNA fragments of approximately 350 bps was excised, electro-eluted into a dialysis membrane, ethanol precipitated and re-suspended in 10 mM Tris-HCl pH 7.5 and 1 mM EDTA to a final concentration of 10 ng/µl. Equimolar amounts of the insert were then ligated overnight at 5C to 1 ug of Lambda ZAP^{TH} II vector (Stratagene Cloning Systems, La Jolla, CA) previously cut by EcoR I and Xho I. A portion of the ligation mixture (1 µl) was packaged for 2 hours at room temperature using Gigapack Gold packaging extract (Stratagene Cloning Systems, La Jolla, CA), and the packaged material was plated on XL1-blue host cells. The library was determined to consist of 2 x 10⁷ V_{K} homologs with less than 30% non-recombinant background.

The vector used above, Lambda Zap II is a derivative of the original Lambda Zap (ATCC # 40,298) that maintains all of the characteristics of the original Lambda Zap including 6 unique cloning sites, fusion protein expression, and the ability to rapidly excise the insert in the form of a phagemid (Bluescript SK-), but lacks the SAM 100 mutation, allowing growth on many Non-Sup F strains, including XL1-Blue. The Lambda Zap II was constructed as described in Short et al., Nucleic Acids Res., 16:7583-7600, 1988, by replacing the Lambda S gene contained in a 4254 base pair (bp) DNA fragment produced by digesting Lambda Zap with the restriction enzyme NcoI. This 4254 bp DNA fragment was replaced with the 4254 bp DNA fragment containing the Lambda S gene isolated from Lambda gt10 (ATCC # 40,179) after digesting the vector with the restriction enzyme NcoI. The 4254 bp DNA fragment isolated from lambda gt10 was ligated into the original Lambda Zap vector using T4 DNA ligase and standard protocols for such procedures described in Current Protocols in Molecular Biology, Ausubel et al., eds., John Wiley and Sons, NY, 1987.

In preparation of cloning a library enriched in V_{L} sequences, 2 ug of PCR amplified products (2.5 mg/30 µl of 150 mM NaCl, 8 mM Tris-HCl (pH 7.5), 6 mM Mg SO₄, 1 mM DTT, 200 mg/ml BSA. 37C) were digested with restriction enzymes Nco I (30 units) and Spe I (45 units). The digested PCR amplified products were purified on a 1% agarose gel using standard electro-elution technique described in Molecular Cloning A Laboratory Manual, Maniatis et al., eds., Cold Spring Harbor, NY, (1982). Briefly, after gel electro-elution of the digested PCR amplified product the region of the gel containing the V_{L}-coding DNA fragment of the appropriate size was excised, electro-elution into a dialysis membrane, ethanol precipitated and re-suspended at a final concentration of 10 ng per ml in a solution containing 10 mM Tris-HCl at pH 7.5 and 1 mM EDTA.

An equal molar amount of DNA representing a plurality of different V_{L}-coding DNA homologs was ligated to a pBluescript SK- phagemid vector that had been previously cut with Nco I and Spe I. A portion of the ligation mixture was transformed using the manufacturer's instructions into Epicuian Coli XL1-Blue competent cells (Stratagene Cloning Systems, La Jolla, CA). The transformant library was determined to consist of 1.2 x 10³ colony forming units/ug of V_{L} homologs with less than 3% non-recombinant background.

### 7. Sequencing of Plasmids from the V_{H}-Coding cDNA Library

To analyze the Lambda Zap II phage clones the clones were excised from Lambda Zap into plasmids according to the manufacture's instructions (Stratagene Cloning System, La Jolla, CA). Briefly, phage plaques were cored from the agar plates and transferred to sterile microfuge tubes containing 500 µl a buffer containing 50 mM Tris-HCl at pH 7.5, 100 mM NaCl, 10 mM MgSO₄, and 0.01% gelatin and 20 µl of chloroform.

For excisions, 200 µl of the phage stock, 200 µl of XL1-Blue cells (A₆₀₀ = 1.00) and 1 µl of R408 helper phage (1 x 10¹¹ pfu/ml) were incubated at 37C for 15 minutes. The excised plasmids were infected into XL1-Blue cells and plated onto LB plates containing ampicillin. Double stranded DNA was prepared from the phagemid containing cells according to the methods described by Holmes et al., Anal. Biochem., 114:193, (1981). Clones were first screened for DNA inserts by restriction digests with either Pvu II or Bgl I and clones containing the putative V_{H} insert were sequenced using reverse transcriptase according to the general method described by Sanger et al., Proc. Natl. Acad. Sci., USA, 74:5463-5467, (1977) and the specific modifications of this method provided in the manufacturer's instructions in the AMV reverse transcriptase ³⁵S-dATP sequencing kit from Stratagene Cloning Systems, La Jolla, CA.

### 8. Characterization Of The Cloned V_{H} Repertoire

The amplified products which had been digested with Xho I and EcoR I and cloned into Lambda ZAP, resulted in a cDNA library with 9.0 x 10⁵ pfu's. In order to confirm that the library consisted of a diverse population of V_{H}-coding DNA homologs, the N-terminal 120 bases of 18 clones, selected at random from the library, were excised and sequenced (Figure 5). To determine if the clones were of V_{H} gene origin, the cloned sequences were compared with known V_{H} sequences and V_{L} sequences. The clones exhibited from 80 to 90% homology with sequences of known heavy chain origin and little homology with sequences of light chain origin when compared with the sequences available in Sequences of Proteins of Immunological Interest by Kabot et al., 4th ed., U.S. Dept.of Health and Human Sciences, (1987). This demonstrated that the library was enriched for the desired V_{K} sequence in preference to other sequences, such as light chain sequences.

The diversity of the population was assessed by classifying the sequenced clones into predefined subgroups (Figure 5). Mouse V_{H} sequences are classified into eleven subgroups (Figure 5). Mouse V_{H} sequences are classified into eleven subgroups [I (A,B,), II (A,B,C), III (A,B,C,D,) V (A,B)] based on framework amino acid sequences described in sequences of Proteins of Immunological Interest by Kabot et al., 4th ed., U.S. Dept.of Health and Human Sciences, (1987); Dildrop, Immunology Today, 5:84, (1984); and Brodeur et al., Eur. J. Immunol., 14; 922, (1984). Classification of the sequenced clones demonstrated that the cDNA library contained V_{K} sequences of at least 7 different subgroups. Further, a pairwise comparison of the homology between the sequenced clones showed that no two sequences were identical at all positions, suggesting that the population is diverse to the extent that it is possible to characterize by sequence analysis.

Six of the clones (L 36-50, Figure 5) belong to the subclass III B and had very similar nucleotide sequences. This may reflect a preponderance of mRNA derived from one or several related variable genes in stimulated spleen, but the data does not permit ruling out the possibility of a bias in the amplification process.

### 9. V_{H}-Expression Vector Construction

The main criterion used in choosing a vector system was the necessity of generating the largest number of Fab fragments which could be screened directly. Bacteriophage lambda was selected as the expression vector for three reasons. First, in vitro packaging of phage DNA is the most efficient method of reintroducing DNA into host cells. Second, it is possible to detect protein expression at the level of single phage plaques. Finally, the screening of phage libraries typically involve less difficulty with nonspecific binding. The alternative, plasmid cloning vectors, are only advantageous in the analysis of clones after they have been identified. This advantage is not lost in the present system because of the use of lambda zap, thereby permitting a plasmid containing the heavy chain, light chain, or Fab expressing inserts to be excised.

To express the plurality of V_{K}-coding DNA homologs in an E. coli host cell, a vector was constructed that placed the V_{H}-coding DNA homologs in the proper reading frame, provided a ribosome binding site as described by Shine et al., Nature, 254:34, 1975, provided a leader sequence directing the expressed protein to the periplasmic space, provided a polynucleotide sequence that coded for a known epitope (epitope tag) and also provided a polynucleotide that coded for a spacer protein between the V_{H}-coding DNA homolog and the polynucleotide coding for the epitope tag. A synthetic DNA sequence containing all of the above polynucleotides and features was constructed by designing single stranded polynucleotide segments of 20-40 bases that would hybridize to each other and form the double stranded synthetic DNA sequence shown in Figure 6. The individual single-stranded polynucleotides (N₁-N₂) are shown in Table 3.

Polynucleotides 2, 3, 9-4', 11, 10-5', 6, 7 and 8 were kinased by adding 1 µl of each polynucleotide (0.1 ug/µl) and 20 units of T₄ polynucleotide kinase to a solution containing 70 mM Tris-HCl at pH 7.6, 10 mM MgCl₂, 5 mM DTT, 10 mM 2ME, 500 micrograms per ml of BSA. The solution was maintained at 37C for 30 minutes and the reaction stopped by maintaining the solution at 65C for 10 minutes. The two end polynucleotides 20 ng of polynucleotides N1 and polynucleotides N12, were added to the above kinasing reaction solution together with 1/10 volume of a solution containing 20.0 mM Tris-HCl at pH 7.4, 2.0 mM MgCl₂ and 50.0 mM NaCl. This solution was heated to 70C for 5 minutes and allowed to cool to room temperature, approximately 25C, over 1.5 hours in a 500 ml beaker of water. During this time period all 10 polynucleotides annealed to form the double stranded synthetic DNA insert shown in Figure 6A. The individual polynucleotides were covalently linked to each other to stabilize the synthetic DNA insert by adding 40 µl of the above reaction to a solution containing 50 mM Tris-HCl at pH 7.5, 7 mM MgCl₂, 1 mM DTT, 1 mM adenosine triphosphate (ATP) and 10 units of T4 DNA ligase. This solution was maintained at 37C for 30 minutes and then the T₄ DNA ligase was inactivated by maintaining the solution at 65C for 10 minutes. The end polynucleotides were kinased by mixing 52 µl of the above reaction, 4 µl of a solution containing 10 mM ATP and 5 units of T4 polynucleotide kinase. This solution was maintained at 37C for 30 minutes and then the T4 polynucleotide kinase was inactivated by maintaining the solution at 65C for 10 minutes. The completed synthetic DNA insert was ligated directly into a lambda Zap II vector that had been previously digested with the restriction enzymes Not I and Xho I. The ligation mixture was packaged according to the manufacture's instructions using Gigapack II Gold packing extract available from Stratagene Cloning Systems, La Jolla, CA. The packaged ligation mixture was plated on XL1 blue cells (Stratagene Cloning Systems, San Diego, CA). Individual Lambda Zap II plaques were cored and the inserts excised according to the in vivo excision protocol provided by the manufacturer, Stratagene Cloning Systems, La Jolla, CA. This in vivo excision protocol moves the cloned insert from the Lambda Zap II vector into a plasmid vector to allow easy manipulation and sequencing. The accuracy of the above cloning steps was confirmed by sequencing the insert using the Sanger dideoxide method described in by Sanger et al., Proc. Natl. Acad. Sci USA, 74:5463-5467, (1977) and using the manufacture's instructions in the AMV Reverse Transcriptase ³⁵S-ATP sequencing kit from Stratagene Cloning Systems, La Jolla, CA. The sequence of the resulting V_{K} expression vector is shown in Figure 6A and Figure 7.

**Table 3**

| | |
|---|---|
| N1) | 5' GGCCGCAAATTCTATTTCAAGGAGACAGTCAT 3' |
| N2) | 5' AATGAAATACCTATTGCCTACGGCAGCCGCTGGATT 3' |
| N3) | 5' GTTATTACTCGCTGCCCAACCAGCCATGGCCC 3' |
| N4) | 5' AGGTGAAACTGCTCGAGAATTCTAGACTAGGTTAATAG 3' |
| N5) | 5' TCGACTATTAACTAGTCTAGAATTCTCGAG 3' |
| N6) | 5' CAGTTTCACCTGGGCCATGGCTGGTTGGG 3' |
| N7) | 5' CAGCGAGTAATAACAATCCAGCGGCTGCCGTAGGCAATAG 3' |
| N8) | 5' GTATTTCATTATGACTGTCTCCTTGAAATAGAATTTGC 3' |
| N9-4) | 5' AGGTGAAACTGCTCGAGATTTCTAGACTAGTTACCCGTAC 3' |
| N11) | 5' GACGTTCCGGACTACGGTTCTTAATAGAATTCG 3' |
| N12) | 5' TCGACGAATTCTATTAAGAACCGTAGTC 3' |
| N10-5) | 5' CGGAACGTCGTACGGGTAACTAGTCTAGAAATCTCGAG 3' |

### 10. V_{L} Expression Vector Construction

To express the plurality of V_{L} coding polynucleotides in an E. coli host cell, a vector was constructed that placed the V_{L} coding polynucleotide in the proper reading frame, provided a ribosome binding site as described by Shine et al., Nature, 254:34, (1975), provided a leader sequence directing the expressed protein to the periplasmic space and also provided a polynucleotide that coded for a spacer protein between the V_{L} polynucleotide and the polynucleotide coding for the epitope tag. A synthetic DNA sequence containing all of the above polynucleotides and features was constructed by designing single stranded polynucleotide segments of 20-40 bases that would hybridize to each other and form the double stranded synthetic DNA sequence shown in Figure 6B. The individual single-stranded polynucleotides (N₁-N₈) are shown in Table 3.

Polynucleotides N2, N3, N4, N6, N7 and N8 were kinased by adding 1 µl of each polynucleotide and 20 units of T₄ polynucleotide kinase to a solution containing 70 mM Tris-HCl at pH 7.6, 10 mM MgCl₂, 5 mM DDT, 10 mM 2ME, 500 micrograms per ml of BSA. The solution was maintained at 37C for 30 minutes and the reaction stopped by maintaining the solution at 65C for 10 minutes. The two end polynucleotides 20 ng of polynucleotides N1 and polynucleotides N5 were added to the above kinasing reaction solution together with 1/10 volume of a solution containing 20.0 mM Tris-HCl at pH 7.4, 2.0 mM MgCl₂ and 50.0 mM NaCl. This solution was heated to 70 C for 5 minutes and allowed to cool to room temperature, approximately 25C, over 1.5 hours in a 500 ml beaker of water. During this time period all the polynucleotides annealed to form the double stranded synthetic DNA insert. The individual polynucleotides were covalently linked to each other to stabilize the synthetic DNA insert with adding 40 µl of the above reaction to a solution containing 50 µl Tris-HCl at pH 7.5, 7 mM MgCl₂, 1 mM DTT, 1 mM ATP and 10 units of T4 DNA ligase. This solution was maintained at 37C for 30 minutes and then the T₄ DNA ligase was inactivated by maintaining the solution at 65C for 10 minutes. The end polynucleotides were kinased by mixing 52 µl of the above reaction, 4 µl of a solution recontaining 10 mM ATP and 5 units of T4 polynucleotide kinase. This solution was maintained at 37C for 30 minutes and then the T4 polynucleotide kinase was inactivated by maintaining the solution at 65C for 10 minutes. The completed synthetic DNA insert was ligated directly into a Lambda Zap II vector that had been previously digested with the restriction enzymes Not I and Xho I. The ligation mixture was packaged according to the manufacture's instructions using Gigapack II Gold packing extract available from Stratagene Cloning Systems, La Jolla, CA. The packaged ligation mixture was plated on XL1-Blue cells (Stratagene Cloning Systems, La Jolla, CA). Individual lambda Zap II plaques were cored and the inserts excised according to the in vivo excision protocol provided by the manufacturer, Stratagene Cloning Systems, La Jolla, CA and described in Short et al., Nucleic Acids Res., 16:7583-7600, 1988. This in vivo excision protocol moves the cloned insert from the Lambda Zap II vector into a phagemid vector to allow easy manipulation and sequencing and also produces the phagemid version of the V_{L} expression vectors. The accuracy of the above cloning steps was confirmed by sequencing the insert using the Sanger dideoxide method described by Sanger et al., Proc. Natl. Acad. Aci. USA, 74:5463-5467, (1977) and using the manufacturer's instructions in the AMV reverse transcriptase ^{3S}S-dATP sequencing kit from Stratagene Cloning Systems, La Jolla, CA. The sequence of the resulting V_{L} expression vector is shown in Figure 6 and Figure 8.

The V_{L} expression vector used to construct the V_{L} library was the phagemid produced to allow the DNA of the V_{L} expression vector to be determined. The phagemid was produced, as detailed above, by the in vivo excision process from the Lambda Zap V_{L} expression vector (Figure 8). The phagemid version of this vector was used because the Nco I restriction enzyme site is unique in this version and thus could be used to operatively linked the V_{L} DNA homologs into the expression vector.

### 11. V_{L}II-Expression Vector Construction

To express the plurality of V_{L}-coding DNA homologs in an E. coli host cell, a vector was constructed that placed the V_{L}-coding DNA homologs in the proper reading frame, provided a ribosome binding site as described by Shine et al., Nature, 254:34, 1975, provided the Pel B gene leader sequence that has been previously used to successfully secrete Fab fragments in E. coli by Lei et al., J. Bac., 169:4379 (1987) and Better et al., Science, 240:1041 (1988), and also provided a polynucleotide containing a restriction endonuclease site for cloning. A synthetic DNA sequence containing all of the above polynucleotides and features was constructed by designing single stranded polynucleotide segments of 20-60 bases that would hybridize to each other and form the double stranded synthetic DNA sequence shown in Figure 10. The sequence of each individual single-stranded polynucleotides (01-08) within the double stranded synthetic DNA sequence is shown in Table 4.

Polynucleotides 02, 03, 04, 05, 06 and 07 were kinased by adding 1 µl (0.1 ug/µl) of each polynucleotide and 20 units of T₄ polynucleotide kinase to a solution containing 70 mM Tris-HCl at pH 7.6, 10 mM magnesium chloride (MgCl), 5 mM dithiothreitol (DTT), 10 mM 2-mercaptoethanol (2ME), 500 micrograms per ml of bovine serum albumin. The solution was maintained at 37C for 30 minutes and the reaction stopped by maintaining the solution at 65C for 10 minutes. The 20 ng each of the two end polynucleotides, 01 and 08, were added to the above kinasing reaction solution together with 1/10 volume of a solution containing 20.0 mM Tris-HCl at pH 7.4, 2.0 mM MgCl and 15.0 mM sodium chloride (NaCl).
This solution was heated to 70C for 5 minutes and allowed to cool to room temperature, approximately 25C, over 1.5 hours in a 500 ml beaker of water. During this time period all 8 polynucleotides annealed to form the double stranded synthetic DNA insert shown in Figure 9. The individual polynucleotides were covalently linked to each other to stabilize the synthetic DNA insert by adding 40 µl of the above reaction to a solution containing 50 ml Tris-HCl at pH 7.5, 7 ml MgCl, 1 mm DTT, 1 mm ATP and 10 units of T4 DNA ligase. This solution was maintained at 37C for 30 minutes and then the T4 DNA ligase was inactivated by maintaining the solution at 65C for 10 minutes. The end polynucleotides were kinased by mixing 52 µl of the above reaction, 4 µl of a solution containing 10 mM ATP and 5 units of T4 polynucleotide kinase. This solution was maintained at 37C for 30 minutes and then the T4 polynucleotide kinase was inactivated by maintaining the solution at 65C for 10 minutes. The completed synthetic DNA insert was ligated directly into a lambda Zap II vector that had been previously digested with the restriction enzymes Not I and Xho I. The ligation mixture was packaged according to the manufacture's instructions using Gigapack II Gold packing extract available from Stratagene Cloning Systems, La Jolla, CA. The packaged ligation mixture was plated on XL1 blue cells (Stratagene Cloning Systems, La Jolla, CA). Individual lambda Zap II plaques were cored and the inserts excised according to the in vivo excision protocol provided by the manufacturer, Stratagene Cloning Systems, La Jolla, CA. This in vivo excision protocol moves the cloned insert from the lambda Zap II vector into a plasmid vector to allow easy manipulation and sequencing. The accuracy of the above cloning steps was confirmed by sequencing the insert using the manufacture's instructions in the AMV Reverse Transcriptase ³⁵S-dATP sequencing kit from Stratagene Cloning Systems, La Jolla, CA. The sequence of the resulting V_{L}II-expression vector is shown in Figure 9 and Figure 11.

**TABLE 4**

| | |
|---|---|
| 01) | 5' 34TGAATTCTAAACTAGTCGCCAAGGAGACAGTCAT 3' |
| 02) | 5' AATGAAATACCTATTGCCTACGGCAGCCGCTGGATT 3' |
| 03) | 5' GTTATTACTCGCTGCCCAACCAGCCATGGCC 3' |
| 04) | 5' GAGCTCGTCAGTTCTAGAGTTAAGCGGCCG 3' |
| 05) | 5' GTATTTCATTATGACTGTCTCCTTGGCGACTAGTTTAGAA- TTCAAGCT 3' |
| 06) | 5' CAGCGAGTAATAACAATCCAGCGGCTGCCGTAGGCAATAG 3' |
| 07) | 5' TGACGAGCTCGGCCATGGCTGGTTGGG 3' |
| 08) | 5' TCGACGGCCGCTTAACTCTAGAAC 3' |

### 12. V_{K} + V_{L} Library Construction

To prepare an expression library enriched in V_{H} sequences, DNA homologs enriched in V_{K} sequences were prepared according to Example 6 using the same set of 5' primers but with primer 12A (Table 1) as the 3' primer. These homologs were then digested with the restriction enzymes Xho I and Spe I and purified on a 1% agarose gel using the standard electro-elution technique described in Molecular Cloning A Laboratory Manual, Maniatis et al., eds., Cold Spring Harbor, NY, (1982). These prepared V_{H} DNA homologs were then directly inserted into the V_{H} expression vector that had been previously digested with Xho I and Spe I.

The ligation mixture containing the V_{H} DNA homologs were packaged according to the manufacturers specifications using Gigapack Gold II Packing Extract (Stratagene Cloning Systems, La Jolla, CA). The expression libraries were then ready to be plated on XL-1 Blue cells.

To prepare a library enriched in V_{L} sequences, PCR amplified products enriched in V_{L} sequences were prepared according to Example 6. These V_{L} DNA homologs were digested with restriction enzymes Nco I and Spe I. The digested V_{L} DNA homologs were purified on a 1% agarose gel using standard electro-elusion techniques described in Molecular Cloning A Laboratory Manual, Maniatis et al., eds., Cold Spring Harbor, NY (1982). The prepared V_{L} DNA homologs were directly inserted into the V_{L} expression vector that had been previously digested with the restriction enzymes Nco I and Spe I. The ligation mixture containing the V_{L} DNA homologs were transformed into XL-1 blue competent cells using the manufacturer's instructions (Stratagene Cloning Systems, La Jolla, CA).

### 13. Inserting V_{L} Codinq DNA Homologs Into V_{L} Expression Vector

In preparation for cloning a library enriched in V_{L} sequences, PCR amplified products (2.5 ug/30 µl of 150 mM NaCl, 8 mM Tris-HCl (pH 7.5), 6 mM MgSO₄, 1 mM DTT, 200 ug/ml BSA at 37C were digested with restriction enzymes Sac I (125 units) and Xba I (125 units) and purified on a 1% agarose gel. In cloning experiments which required a mixture of the products of the amplification reactions, equal volumes (50 µl, 1-10 ug concentration) of each reaction mixture were combined after amplification but before restriction digestion. After gel electrophoresis of the digested PCR amplified spleen mRNA, the region of the gel containing DNA fragments of approximate 350 bps was excised, electro-eluted into a dialysis membrane, ethanol precipitated and re-suspended in a TE solution containing 10 mM Tris-HCl pH 7.5 and 1 mM EDTA to a final concentration of 50 ng/µl.

The V_{L}II-expression DNA vector was prepared for cloning by admixing 100 ug of this DNA to a solution containing 250 units each of the restriction endonucleases Sac 1 and Xba 1 (both from Boehringer Mannheim, Indianapolis, IN) and a buffer recommended by the manufacturer. This solution was maintained at 37 from 1.5 hours. The solution was heated at 65C for 15 minutes top inactivate the restriction endonucleases.
The solution was chilled to 30C and 25 units of heat-killable (HK) phosphatase (Epicenter, Madison, WI) and CaCl₂ were admixed to it according to the manufacturer's specifications. This solution was maintained at 30C for 1 hour. The DNA was purified by extracting the solution with a mixture of phenol and chloroform followed by ethanol precipitation. The V_{L}II expression vector was now ready for ligation to the V_{L} DNA homologs prepared in the above examples.

DNA homologs enriched in V_{L} sequences were prepared according to Example 5 but using a 5' light chain primer and the 3' light chain primer shown in Table 2. Individual amplification reactions were carried out using each 5' light chain primer in combination with the 3' light chain primer. These separate V_{L} homolog containing reaction mixtures were mixed and digested with the restriction endonucleases Sac 1 and Xba 1 according to Example 6. The V_{L} homologs were purified on a 1% agarose gel using the standard electro-elution technique described in Molecular Cloning A Laboratory Manual, Maniatis et al., eds., Cold Spring Harbor, NY, (1982). These prepared V_{L} DNA homologs were then directly inserted into the Sac 1 - Xba cleaved V_{L}II-expression vector that was prepared above by ligating 3 moles of V_{L} DNA homolog inserts with each mole of the V_{L}II-expression vector overnight at 5C. 3.0 x 10⁵ plague forming units were obtained after packaging the DNA with Gigapack II Bold (Stratagene Cloning Systems, La Jolla, CA) and 50% were recombinants.

### 14. Randomly Combining V_{H} and V_{L} DNA Homologs on the Same Expression Vector

The V_{L}II-expression library prepared in Example 13 was amplified and 500 ug of V_{L}II-expression library phage DNA prepared from the amplified phage stock using the procedures described in Molecular Cloning: A Laboratory Manual, Maniatis et al., eds., Cold Spring Harbor, NY (1982), 50 ug of this V_{L}II-expression library phage DNA was maintained in a solution containing 100 units of MLuI restriction endonuclease (Boehringer Mannheim, Indianapolis, IN) in 200 µl of a buffer supplied by the endonuclease manufacturer for 1.5 hours at 37C. The solution was then extracted with a mixture of phenol and chloroform. The DNA was then ethanol precipitated and re-suspended in 100 µl of water. This solution was admixed with 100 units of the restriction endonuclease EcoR I (Boehringer Mannheim, Indianapolis, IN) in a final volume of 200 µl of buffer containing the components specified by the manufacturer. This solution was maintained at 37C for 1.5 hours and the solution was then extracted with a mixture of phenol and chloroform. The DNA was ethanol precipitated and the DNA re-suspended in TE.

the V_{H} expression library prepared in Example 12 was amplified and 500 ug of V_{H} expression library phage DNA prepared using the methods detailed above. 50 ug of the V_{H} expression library phage DNA was maintained in a solution containing 100 units of Hind III restriction endonuclease (Boehringer Mannheim, Indianapolis, IN) in 200 µl of a buffer supplied by the endonuclease manufacturer for 1.5 hours at 37C. The solution was then extracted with a mixture of phenol and chloroform saturated with 0.1 M Tris-HCl at pH 7.5. The DNA was then ethanol precipitated and re-suspended in 100 µl of water. This solution was admixed with 100 units of the restriction endonuclease EcoR I (Boehringer Mannheim, Indianapolis, IN) in a final volume of 200 µl of buffer containing the components specified by the manufacturer. This solution was maintained at 37C for 1.5 hours and the solution was then extracted with a mixture of phenol and chloroform. The DNA was ethanol precipitated and the DNA re-suspended in TE.

The restriction digested V_{H} and V_{L}II-expression Libraries were ligated together. The ligation reaction consisted of 1 ug of V_{H} and 1 ug of V_{L}II phage library DNA in a 10 µl reaction using the reagents supplied in a ligation kit purchased from Stratagene Cloning Systems (La Jolla, California). After ligation for 16 hr at 4C, 1 µl of the ligated the phage DNA was packaged with Gigapack Gold II packaging extract and plated on XL 1-blue cells prepared according to the manufacturers instructions. A portion of the 3X10⁶ clones obtained were used to determine the effectiveness of the combination. The resulting V_{H} and V_{L} expression vector is shown in Figure 11.

Clones containing both V_{H} and V_{L} were excised from the phage to pBluescript using the in vitro excision protocol described by Short et al., Nucleic Acid Research, 16:7583-7600 (1988). Clones chosen for excision expressed the decapeptide tag and did not cleave X-gal in the presence of 2mM IPTGthus remaining white. Clones with these characteristics represented 30% of the library. 50% of the clones chosen for excision contained a V_{H} and V_{L} as determined by restriction analysis. Since approximately 30% of the clones in the V_{H} library expressed the decapeptide tag and 50% of the clones in the V_{L}II library contained a V_{L} sequence it was anticipated that no more than 15% of the clones in the combined library would contain both V_{H} and V_{L} clones. The actual number obtained was 15% of the library indicating that the process of combination was very efficient.

### 15. Segregating DNA Homologs For a V_{H} Antigen Binding Protein

To segregate the individual clones containing DNA homologs that code for a v_{H} antigen binding protein, the titre of the V_{H} expression library prepared according to Example 11 was determined. This library titration was performed using methods well known to one skilled in the art. Briefly, serial dilutions of the library were made into a buffer containing 100 mM NaCl, 50 mM Tris-HCl at pH 7.5 and 10 mM MgSO₄. Ten µl of each dilution was added to 200 µl of exponentially growing E. coli cells and maintained at 37C for 15 minutes to allow the phage to absorb to the bacterial cells. Three ml of top agar consisting of 5 g/L NaCl, 2 g/L of MgSO₄, 5 g/L yeast extract, 10 g/L NZ amine (casein hydrolysate) and 0.7% melted, 50C agarose. The phage, the bacteria and the top agar were mixed and then evenly distributed across the surface of a prewarmed bacterial agar plate (5 g/L NaCl, 2 g/L MgSO₄, 5 g/L yeast extract, 10 g/L NZ amine (casein hydrolysate) and 15 g/L Difco agar. The plates were maintained at 37C for 12 to 24 hours during which time period the lambda plaques developed on the bacterial lawn. The lambda plaques were counted to determined the total number of plaque forming units per ml in the original library.

The titred expression library was then plated out so that replica filters could be made from the library. The replica filters will be used to later segregate out the individual clones in the library that are expressing the antigens binding proteins of interest. Briefly, a volume of the titred library that would yield 20,000 plaques per 150 millimeter plate was added to 600 µl of exponentially growing E. coli cells and maintained at 37C for 15 minutes to allow the phage to absorb to the bacterial cells. Then 7.5 ml of top agar was admixed to the solution containing the bacterial cells and the absorbed phage and the entire mixture distributed evenly across the surface of a prewarmed bacterial agar plate. This process was repeated for a sufficient number of plates to plate out a total number of plaques at least equal to the library size. These plates were then maintained at 37 C for 5 hours. The plates were then overlaid with nitrocellulose filters that had been pretreated with a solution containing 10 mM isopropyl-beta-D-thiogalactopyranosid (IPTG) and maintained at 37C for 4 hours. The orientation of the nitrocellulose filters in relation to the plate were marked by punching a hole with a needle dipped in waterproof ink through the filter and into the bacterial plates at several locations. The nitrocellulose filters were removed with forceps and washed once in a TBST solution containing 20 mM Tris-HCL at pH 7.5, 150 mM NaCl and 0.05% monolaurate (tween-20). A second nitrocellulose filter that had also been soaked in a solution containing 10 mM IPTG was reapplied to the bacterial plates to produce duplicate filters. The filters were further washed in a fresh solution of TBST for 15 minutes. Filters were then placed in a blocking solution consisting of 20 mM Tris-HCl at pH 7.5, 150 mM NaCL and 1% BSA and agitated for 1 hour at room temperature. The nitrocellulose filters were transferred to a fresh blocking solution containing a 1 to 500 dilution of the primary antibody and gently agitated for at least 1 hour at room temperature. After the filters were agitated in the solution containing the primary antibody the filters were washed 3 to 5 times in TBST for 5 minutes each time to remove any of the residual unbound primary antibody. The filters were transferred into a solution containing fresh blocking solution and a 1 to 500 to a 1 to 1,000 dilution of alkaline phosphatase conjugated secondary antibody. The filters were gently agitated in the solution for at least 1 hour at room temperature. The filters were washed 3 to 5 times in a solution of TBST for at least 5 minutes each time to remove any residual unbound secondary antibody.
The filters were washed once in a solution containing 20 mM Tris-HCl at pH 7.5 and 150 mM NaCL. The filters were removed from this solution and the excess moisture blotted from them with filter paper. The color was developed by placing the filter in a solution containing 100 mM Tris-HCl at pH 9.5, 100 mM NaCl, 5 mM MgCl₂, 0.3. mg/ml of nitro Blue Tetrazolium (NBT) and 0.15 mg/ml of 5-bromo-4-chloro-3-indolyl-phosphate (BCIP) for at least 30 minutes at room temperature. The residual color development solution was rinsed from the filter with a solution containing 20 mM Tris-HCl at pH 7.5 and 150 mM NaCl. The filter was then placed in a stop solution consisting of 20 mM Tris-HCl at pH 2.9 and 1 mM EDTA. The development of an intense purple color indicates at positive result. The filters are used to locate the phage plaque that produced the desired protein. That phage plaque is segregated and then grown up for further analysis.

Several different combinations of primary antibodies and second antibodies were used. The first combination used a primary antibody immunospecific for a decapeptide that will be expressed only if the V_{H} antigen binding protein is expressed in the proper reading frame to allow read through translation to include the decapeptide epitope covalently attached to the V_{H} antigen binding protein. This decapeptide epitope and an antibody immunospecific for this decapeptide epitope was described by Green et al., Cell 28:477 (1982) and Niemann et al., Proc. Nat. Acad. Sci. U.S.A. 80:4949 (1983). The sequence of the decapeptide recognized is shown in Figure 11. A functional equivalent of the monoclonal antibody that is immunospecific for the decapeptide can be prepared according to the methods of Green et al. and Niemann et al. The secondary antibody used with this primary antibody was a goat anti-mouse IgG (Fisher Scientific). This antibody was immunospecific for the constant region of mouse IgG and did not recognize any portion of the variable region of heavy chain. This particular combination of primary and secondary antibodies when used according to the above protocol determined that between 25% and 30% of the clones were expressing the decapeptide and therefore these clones were assumed to also be expressing a V_{H} antigen binding protein.

In another combination the anti-decapeptide mouse monoclonal was used as the primary antibody and an affinity purified goat anti-mouse Ig, commercially available as part of the picoBlue immunoscreening kit from Stratagene Cloning System, La Jolla, CA, was use as the secondary antibody. This combination resulted in a large number of false positive clones because the secondary antibody also immunoreacted with the V_{H} of the heavy chain Therefore this antibody reacted with all clones expressing any V_{H} protein and this combination of primary and secondary antibodies did not specifically detect clones with the V_{H} polynucleotide in the proper reading frame and thus allowing expressing of the decapeptide.

Several combinations of primary and secondary antibodies are used where the primary antibody is conjugated to fluorescein isothiocyanate (FITC) and thus the immunospecificity of the antibody was not important because the antibody is conjugated to the preselected antigen (FITC) and it is that antigen that should be bound by the V_{H} antigen binding proteins produced by the clones in the expression library. After this primary antibody has bound by virtue that is FITC conjugated mouse monoclonal antibody p2 5764 (ATCC #HB-9505). The secondary antibody used with this primary antibody is a goat anti-mouse Ig⁶ (Fisher Scientific, Pittsburg, PA) conjugated to alkaline phosphatase. Using the method described in Antibodies A Laboratory Manual, Harlow and Lowe, eds., Cold Springing Harbor, NY, (1988). If a particular clone in the V_{H} expression, library, expresses a V_{H} binding protein that binds the FITC covalently coupled to the primary antibody, the secondary antibody binds specifically and when developed the alkaline phosphate causes a distinct purple color to form.

The second combination of antibodies of the type uses a primary antibody that is FITC conjugated rabbit anti-human IgG (Fisher Scientific, Pittsburg, PA). The secondary antibody used with this primary antibody is a goat anti-rabbit IgG conjugated to alkaline phosphatase using the methods described in Antibodies A Laboratory Manual, Harlow and Lane, eds., Cold Spring Harbor, NY, (1988). If a particular clone in the V_{H} expression library expresses a V_{H} binding protein that binds the FITC conjugated to the primary antibody, the secondary antibody binds specifically and when developed the alkaline phosphatase causes a distinct purple color to form.

Another primary antibody was the mouse monoclonal antibody p2 5764 (ATCC # HB-9505) conjugated to both FITC and ¹²⁵I. The antibody would be bound by any V_{H} antigen binding proteins expressed. Then because the antibody is also labeled with ¹²⁵I, an autoradiogram of the filter is made instead of using a secondary antibody that is conjugated to alkaline phosphatase. This direct production of an autoradiogram allows segregation of the clones in the library expressing a V_{H} antigen binding protein of interest.

### 16. Segregating DNA Homologs For a V_{H} and V_{L} that Form an Antigen Binding F_{V}

To segregate the individual clones containing DNA homologs that code for a V_{H} and a V_{L} that form an antigen binding F_{V} the V_{H} and V_{L} expression library was titred according to Example 15. The titred expression library was then screened for the presence of the decapeptide tag expressed with the V_{H} using the methods described in Example 15. DNA was then prepared from the clones to express the decapeptide tag. This DNA was digested with the restriction endonuclease Pvu II to determine whether these clones also contained a V_{L} DNA homolog. The slower migration of a PvuII restriction endonuclease fragment indicated that the particular clone contained both a V_{H} and a V_{L} DNA homolog.

The clones containing both a V_{H} and a V_{L} DNA homolog were analyzed to determine whether these clones produced an assembled F_{V} protein molecule from the V_{H} and V_{L} DNA homologs.

The F_{V} protein fragment produced in clones containing both V_{H} and V_{L} was visualized by immune precipitation of radiolabeled protein expressed in the clones. A 50 ml culture of LB broth (5 g/L yeast extract, 10 g/L and tryptone 10 g/L NaCl at pH 7.0) containing 100 ug/µl of ampicillin was inoculated with E. Coli harboring a plasmid contain a V_{H} and a V_{L}· The culture was maintained at 37C with shaking until the optical density measured at 550 nm was 0.5 culture then was centrifuged at 3,000 g for 10 minutes and re-suspended in 50 ml of M9 media (6 g/L Na₂HPO₄, 3 g/L KH₂PO₄, 0.5 g/L NaCl, 1 g/L NH₄Cl, 2g/L glucose, 2 mM MgSO₄ and 0.1 mMgSO₄ C_{A}Cl₂ supplemented with amino acids without methionine or cysteine. This solution was maintained at 37C for 5 minutes and then 0.5 mCi of ³⁵S as HSO₄⁻ (New England Nuclear, Boston, MA) was added and the solution was further maintained at 37C for an additional 2 hours. The solution was then centrifuged at 3000xg and the supernatant discarded. The resulting bacterial cell pellet was frozen and thawed and then re-suspended in a solution containing 40 mM Tris pH 8.0, 100 mM sucrose and 1 mM EDTA. The solution was centrifuged at 10000xg for 10 minutes and the resulting pellet discarded. The supernatant was admixed with 10 µl of anti-decapeptide monoclonal antibody and maintained for 30-90 minutes on ice. 40 µl of protein G coupled to sepharose beads (Pharmacia, Piscataway, NJ) was admixed to the solution and the added solution maintained for 30 minutes on ice to allow an immune precipitate to form. The solution was centrifuged at 10,000 xg for 10 minutes and the resulting pellet was re-suspended in 1 ml of a solution containing 100 mM Tris-HCl at pH 7.5 and centrifuged at 10,000 xg for 10 minutes. This procedure was repeated twice. The resulting immune precipitate pellet was loaded onto a PhastGel Homogenous 20 gel (Pharmacia, Piscataway, NJ) according to the manufacturer's directions. The gel was dried and used to expose X-ray film.

The resulting autoradiogram is shown in Figure 12. The presence of assembled Fᵥ molecules can be seen by the presence of V_{L} that was immunoprecipitated because it was attached to the V_{H}-decapeptide tag recognized by the precipitating antibody.

### 17. Construction of Selectable V_{H} and V_{L} Expression

### A. Construction of the Mutant S Gene Expression Plasmid

The bacteria phage lambda S gene has been shown to be directly involved in lysis as described by Reader et al., Virology, 43:607-622 (1971). The S gene encodes a 107 amino acid polypeptide that is responsible for a lethal event in the cytoplasmic membrane that allows the release of the R gene product into the periplasm of the cell where it degrades the peptidoglycan as described by Garrett et al., J. Virology, 44:886-892 (1982). The dominant S gene mutant (S₁₀₀ SAM 5) is a mutation that has been shown to interfere with the formation of the normal S protein membrane channel thus preventing cell lysis. See Raab et al., J. Mol. Biol., 199:95-105 (1988). This mutant S gene is dominant because when it is expressed, even in the presence of the wild type S protein it prevents lysis of the bacterial cell. The S₁₀₀ SAM 5 dominant mutation also contains an amber mutation and therefore requires the expression of a suppressor tRNA in the bacterial cell in order for mutant S protein to be produced. Further, this amber mutation allows the growth of bacteria containing the mutant S gene construct without lysis because without this amber suppressing tRNA no functional S gene protein is produced.

The dominant S gene from Lambda Zap Sam 5 was isolated using the polymerase chain reaction. Briefly, Lambda Zap Sam 5 DNA was isolated using the methods described in Molecular Cloning: A Laboratory Manual, Maniatis et al., eds., Cold Spring Harbor, NY (1982). Lambda Zap Sam 5 DNA, 0.1 ug, was admixed with a buffer containing 150 ng of primer RG15 (Table 5) and 150 ng of primer RG16 (Table 5), 0.25 mM each of dTTP, dCTP, dGTP, and dATP (dNTPs), 50 mM KCl, 10 mM Tris-HCl at pH 8.3, 1.5 mM MgCl₂, and 0.15% sterile gelatin. The resulting solution was heated to 91C for five minutes and then placed in a 54C water bath for five minutes. 0.5 microliters of Taq polymerase (Perkin Elmer-Cetus, Norwalk, CT) was added and the solution overlaid with a layer of mineral oil.

The solution was then placed in a DNA Thermal Cycler (Perkin-Elmer Cetus, Norwalk, CT) and subjected to the following temperature and time conditions: (1) 72C for two minutes to allow for primer extension, (2) 91C for one minute to heat denature the duplex DNA and (3) 54C for two minutes to allow the single-stranded nucleic acids to hybridize. The same solution was subjected to further cycles of steps (1), (2), and (3) for a total of thirty cycles according to the manufacturer's instructions. The cycled solution was then maintained at 72C for ten minutes and then stored at 4C until used.

The mutant S gene DNA produced by the above polymerase chain reaction was digested with the restriction endonucleases Hind III and Bgl II. Briefly, one half of the polymerase chain reaction product produced above was purified by phenol extraction followed by ethanol precipitation. The DNA was then admixed with a solution containing 100 mM NaCl, 10 mM Tris-HCl at pH 7.7, 19 mM Mg Cl₂, 1 mM DTT, 100 ug/ml BSA, 20 units of Hind III and 10 units of Bgl II. This solution was maintained at 37C for one hour. The efficiency of this restriction endonuclease digestion was determined by gel electrophoresis according to the methods described in Current Protocols in Molecular Biology, Ausubel et al., eds., John Wiley and Sons, NY (1987).

One half of the polymerase chain reaction product was digested with the restriction endonuclease Sau 3A and Bgl II. Briefly, the DNA was admixed with a buffer containing 100 mM NaCl, 10 mM Tris-HCl at pH 7.7, 10 mM MgCl₂, 1 mM DTT, 100 ug/ml bovine serum albumin (BSA), 10 units of Sau 3A, and 10 units of Bgl II (Stratagene, La Jolla, CA). This solution was maintained at 37C for one hour. The efficiency of this restriction endonuclesis digestion was determined by gel electrophoresis.

The resulting predominant, approximately 500 base pair bands were isolated and purified on agarose gels according to the procedures described in Molecular Cloning: A Laboratory Manual, Maniatis et al., eds., Cold Spring Harbor, NY (1982). The DNA was purified from the agarose slices by electro-elution according to the methods described in Molecular Cloning: A Laboratory Manual, Maniatis et al., eds., Cold Spring Harbor, NY (1982). The electro-eluted DNA was purified by phenol extraction followed by ethanol precipitation.

The mutant S gene was inserted into pBluescript KS+ (Stratagene) that had been previously digested with the restriction endonuclease Hind III and BamH I. Briefly, the pBluescript KS+ was admixed with a buffer containing 100 mM Nacl, 10 mM Tris-HCl at pH 7.7, 10 mM Mg Cl₂, 1 mM DTT, 100 ug/ml BSA, 40 units of BamH I and 40 units of Hind III (Stratagene). This solution was maintained at 37C for one hour. The pBluescript KS+ containing solution was then adjusted to pH 8.0 by adding Tris-HCl at pH 8.0 to a final concentration of 0.1 M. Five units of calf intestinal alkaline phosphatase (Stratagene) was added to this solution and the solution maintained at 37C for 30 minutes. The calf intestine alkaline phosphatase was then inactivated by maintaining the solution at 65C for 10 minutes. The pBluescript KS+ was then purified by phenol extraction followed by ethanol precipitation. The restriction endonuclease cleaned pBluescript KS+ was then re-suspended in a solution containing 10 mM Tris-HCl at pH 8.0 and 1 mM EDTA.

The mutant S gene was inserted (ligated) into the pBluescript vector prepared above by digestion with Hind III and BamH I restriction endonuclease. Briefly, 1 µl of pBluescript vector that had been previously cut with Hind III and BamH I was admixed with 1 µl of the mutant S gene insert prepared above, 1 µl of a buffer containing 0.66 M Tris-HCl at pH 7.6, 50 mM MgCl₂, 50 mM Dithiothreitol (DTT) and 1 µl of a solution containing 10 mM ATP and 0.5 µl (4 units) of T4 DNA ligase (Stratagene). This solution was maintained at 37C for one hour.

The ligation mixture was transformed in XL1 Blue cells (Stratagene) according to the manufacture's directions.

The accuracy of the above cloning steps is confirmed by DNA sequencing.

### B. Selectable V_{H}-Expression Vector Construction

To add the ability to select against expression vectors not containing V_{H}-coding DNA homologs, a suppressor tRNA gene was inserted into the V_{H}-Expression vector prepared in Example 9. The selectable V_{H}-Expression vector was prepared by inserting a synthetic DNA sequence containing the suppressor tRNA gene and DNA sequence coding for the decapeptide tag into the V_{H}-Expression vector prepared in Example 9 that had been previously cleaved with the restriction endonucleases Xho I and Eco RI.

A synthetic DNA sequence containing the suppressor tRNA gene and polynucleotide sequence coding for decapeptide tag was constructed by designing single stranded polynucleotide segments of 20-40 bases that would hybridize to each other and form the double stranded synthetic DNA sequence shown in Figure 18A. The individual single-stranded polynucleotides are shown in Table 5.

**TABLE 5**

| | |
|---|---|
| R615) | 5'-AATAAGCTTGATCTATCAGTAATCGACC-3' |
| R616) | 5'-ATTAGATCTGAATTCTGACGTCCGTTATCAG-3' |
| 926) | 5'GATCCGCTTCCCGATAAGGGAGCAGGCCAGTAAAAGCATTACCTGTGGTGGGGTTC-3'. |
| 927) | 5'-CCGAGCGGCCAAAGGGAGCAGACTCTAAATCTGCCGTCATCGACTTCGAAG-3' |
| 928) | 5'-GTTCGAATCCTTCCCCCACCACCATCACTTTCAAAAGTCCGA-3' |
| 929) | 5'-CTAGTCGGACTTTTGAAAGTGATGGTGGTGGGGGAAGGATTCGAACCTTCGAAGTC-3' |
| 930) | 5'-GATGACGGCAGATTTAGAGTCTGCTCCCTRTTGGCCGCTCGGGAACCCCACC-3' |
| 931) | 5'-ACAGGTAATGCTTTTACTGGCCTGCTCCCTTATCGGGAAGCG-3' |
| 970) | 5'-TCGAGCGCC-3' |
| 971) | 5'-GATCGGCGC-3' |
| 972) | 5'-CTAGGGCCT-3' |
| 973) | 5'-CTAGAGGCC-3' |
| 974) | 5'-CGCCC-3'. |
| 975) | 5'-GATCGGGCGAGCT-3' |
| AB23) | 5'-CTAGTTACCCGTACGACGTTCCGGACTACGCTTCTTAATAG-3' |
| AB24) | 5'-AATTCTATTAAGAAGCGTAGTCCGGAACGTCGACGGGTAA-3' |

Polynucleotides 926, 927, 928, 929, 930, 931, AB23 and 971 were kinased by adding 1.0 µl of each polynucleotide (0.1 ug/µl) and 20 units of T₄ polynucleotide kinase to a solution containing 70 mM Tris-HCl at pH 7.6, 10 mM MgCl₂, 5 mM DTT, 10 mM 2ME, 500 micrograms per ml of BSA. The solution was maintained at 37C for 30 minutes and the reaction stopped by maintaining the solution at 65C for 10 minutes.

The required polynucleotides were annealed to form the synthetic DNA sequence shown in Figure 18A. Briefly, the following solutions of polynucleotides were admixed to 1/10 volume of a solution containing 20.0 mM Tris HCl at pH 7.4, 2.0 mM MgCl₂ and 50.0 mM NaCl; 5 µl of separate, 2.5 ug/ml solutions containing the kinased polynucleotides 926, 927, 928, 929, 930 and 931; 4 µl of separate, 2.0 ug/ml solutions containing the unkinased polynucleotide AB24 and the kinased polynucleotide AB23; 2 µl of separate, 1.0 ug/ml solutions containing the kinased polynucleotide 971, and the unkinased polynucleotide 970.

This solution was heated to 70C for 5 minutes and allowed to cool to 40C over 1.5 hours in a 500 ml beaker of water. During this time period all 10 polynucleotides annealed to form the double stranded synthetic DNA insert shown in Figure 18A. The individual polynucleotides were covalently linked to each other to stabilize the synthetic DNA insert by admixing all of the above reaction (46.6 µl) to a solution containing 50 mM Tris-HCl at pH 7.5, 7 mM MgCl₂, 1 mM DDT, 1 mM adenosine triphosphate (ATP) and 10 units of T4 DNA ligase to form a ligation reaction admixture. This admixture was maintained at 37C for 1 hour and then the T4 DNA ligase was inactivated by maintaining the solution at 65C for 15 minutes. The end polynucleotides were kinased by admixing all of the above ligation reaction admixture reaction, 6 µl of a solution containing 10 mM ATP and 5 units of T4 polynucleotide kinase. This solution was maintained at 37C for 30 minutes and then the T4 polynucleotide kinase was inactivated by maintaining the solution at 65C for 10 minutes. The completed synthetic DNA insert (Figure 18A) was ready for ligation to the V_{H}-Expression vector (Figure 7) that had been previously digested with the restriction endonucleases Xho I and Eco RI.

The V_{H}-Expression vector (Figure 7) was digested with the restriction endonucleases Xho I and Eco RI, according to the manufacturers recommendations. Briefly, 50 ug the V_{H}-Expression vector (38.5 µl), 225 units of Xho I (Stratagene) and 150 units of Eco RI (Stratagene), were admixed to a universal restriction endonuclease buffer consisting of 50 mM Tris-HCl at pH 7.7, 10 mM MgCl₂, 50 mM NaCl and 100 ug/ml BSA to form a digestion admixture. The digestion admixture was maintained at 37C for 2 hours.

The digestion admixture was then adjusted to pH 8.0 by adding a solution of 1.0 m Tris-HCl at pH 8.0 to a final concentration of 0.1 M. 2.5 units of calf intestine alkaline phosphatase (Stratagene) was added to this solution and the resulting solution maintained at 37C for 30 minutes. The calf intestine alkaline phosphatase was inactivated by maintaining the solution at 65C for 10 minutes. The V_{H}-Expression vector DNA was then purified by phenol extraction followed by ethanol precipitation. The restriction endonuclease cleaved V_{H}-Expression vector DNA was then re-suspended in 50 µl of a solution containing 10 mM Tris-HCl at pH 8.0 and 1 mM EDTA.

The synthetic DNA insert prepared above was inserted into the restriction endonuclease cleaved V_{H}-Expression vector. Briefly, 1 ug of Xho I and Eco RI cleaved V_{H}-Expression vector, 2 µl of synthetic DNA insert (0.5 ug) and 0.5 µl (4 units) of T4 DNA ligase (Stratagene) was admixed to a solution containing 66 mM Tris-HCl at pH 7.6, 5.0 mM MgCl₂, 5.0 mM DTT and 1.0 mM ATP to form a ligation admixture. The ligation admixture was maintained at 37C for 2 hours. The ligation mixture was then packaged according to the manufacturer's instructions using Gigapack II Gold packing extract available from Stratagene. The packaged ligation mixture was then plated on XL1 blue cells (Stratagene).

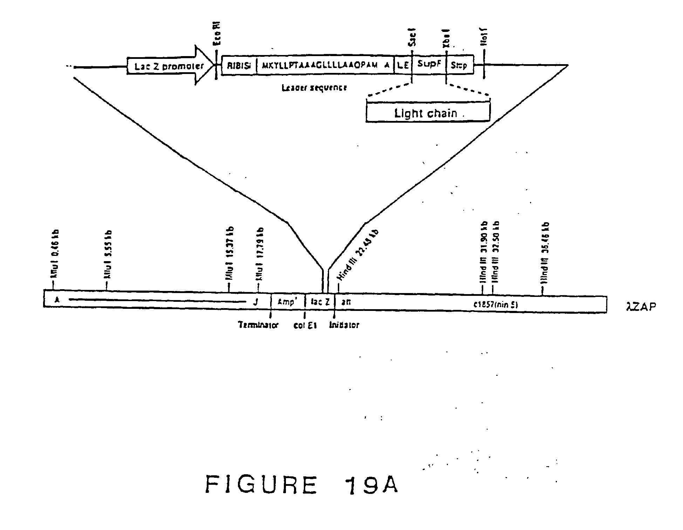

Individual lambda phage plaques were selected for DNA sequencing by selecting individual plaques that hybridized to polynucleotides contained in the synthetic DNA insert according to the method described in Current Protocols in Molecular Biology, Ausubel et al., eds., John Wiley and Sons, NY (1987). The selectable V_{H} expression vector is shown in Figure 19A.

### C. Selectable V_{L}-Expression Vector Construction

To add the ability to select against expression vectors not containing V_{L}-coding DNA homologs, a suppressor tRNA gene was inserted into the V_{L}-Expression vector prepared in Example 11. The selectable V_{L}-Expression vector was prepared by inserting a synthetic DNA sequence containing the suppressor tRNA gene into the V_{L}-Expression vector prepared in Example 11 that had been previously cleaved with the restriction endonucleases Sac I and Xba I.

A synthetic DNA sequence containing the suppressor tRNA gene was constructed by designing single stranded polynucleotide segments of 20-40 bases that would hybridize to each other and form the double stranded synthetic DNA sequence shown in Figure 18B. The individual single-stranded polynucleotides are shown in Table 5.

Polynucleotides 926, 927, 928, 929, 930, 972 and 975 were kinased by adding 1.0 µl of each polynucleotide (0.1 ug/µl) and 20 units of T₄ polynucleotide kinase to a solution containing 70 mM Tris-HCl at pH 7.6, 10 mM MgCl₂, 5 mM DTT, 10 mM 2ME, 100 micrograms per ml of BSA. The solution was maintained at 37C for 30 minutes and the reaction stopped by maintaining the solution at 65C for 10 minutes.

The required polynucleotides were annealed to form the synthetic DNA sequence shown in Figure 18B. Briefly, the following solutions of polynucleotides were admixed to 1/10 volume of a solution containing 20.0 mM Tris HCl at pH 7.4, 2.0 mM MgCl₂ and 50.0 mM NaCl ; 5 µl of separate, 2.5 ug/ml solutions containing the kinased polynucleotides 926, 927, 928, 929, 930 and 931; 2 µl of separate, 2.0 ug/ml solutions containing the unkinased polynucleotides 974 and 973, and the kinased polynucleotides 972 and 975.

This solution was heated to 70C for 5 minutes and allowed to cool to 40C over 1.5 hours in a 500 ml beaker of water. During this time period all 10 polynucleotides annealed to form the double stranded synthetic DNA insert shown in Figure 18B. The individual polynucleotides were covalently linked to each other to stabilize the synthetic DNA insert by admixing all of the above reaction (42.2 µl) to a solution containing 50 mM Tris-HCl at pH 7.5, 7 mM MgCl₂, 1 mM DDT, 1 mM adenosine triphosphate (ATP) and 10 units of T₄ DNA ligase to form a ligation reaction admixture. This admixture was maintained at 37C for 1 hour and then the T4 DNA ligase was inactivated by maintaining the solution at 65C for 15 minutes. The end polynucleotides were kinased by admixing all of the above ligation reaction admixture reaction, 6 µl of a solution containing 10 mM ATP and 5 units of T4 polynucleotide kinase. This solution was maintained at 37C for 30 minutes and then the T4 polynucleotide kinase was inactivated by maintaining the solution at 65C for 10 minutes, the completed synthetic DNA insert (Figure 18B) was ready for ligation to the V_{L}-Expression vector (Figure 9) that had been previously digested with the restriction endonucleases Sac I and Xba I.

The V_{L}-Expression vector (Figure 9) was digested with the restriction endonucleases Sac I and Xba I, according to the manufacturer's recommendations.
Briefly, 5.0 ug the V_{L}-Expression vector (30.5 µl), 50 units of Sac I (Stratagene) and 50 units of Xba I (Stratagene), were admixed to a universal restriction endonuclease buffer consisting of 10 mM Tris-HCl at pH 7.7, 10 mM MgCl₂, 100 mM NaCl and 100 ug/ml BSA to form a digestion admixture. The digestion admixture was maintained at 37C for 2 hours.

The digestion admixture was then adjusted to pH 8.0 by adding a solution of 1.0 m Tris-HCl at pH 8.0 to a final concentration of 0.1M. 2.5 units of calf intestine alkaline phosphatase (Stratagene) was added to this solution and the resulting solution maintained at 37C for 30 minutes. The calf intestine alkaline phosphatase was inactivated by maintaining the solution at 65C for 10 minutes. The V_{L}-Expression vector DNA was then purified by phenol extraction followed by ethanol precipitation. The restriction endonuclease cleaved V_{L}-Expression vector DNA was the re-suspended in 50 µl of a solution containing 10 mM Tris-HCl at pH 8.0 and 1 mM EDTA.

The synthetic DNA insert prepared above was inserted into the restriction endonuclease cleaved V_{L}-Expression vector. Briefly, 1 ug of Sac I and Xba I cleaved V_{L}-Expression vector, 2 µl of synthetic DNA insert (0.5 ug) and 0.5 µl (4 units) of T4 DNA ligase (Stratagene) was admixed to a solution containing 66 mM Tris-HCl at pH 7.6, 5.0 mM MgCl₂, 5.0 mM DTT and 1.0 mM ATP to form a ligation admixture. The ligation admixture was maintained at 37C for 2 hours. The ligation mixture was packaged according to the manufacturer's instructions using Gigapack II Gold packing extract available from Stratagene. The packaged ligation mixture was plated on XL1 blue cells (Stratagene).

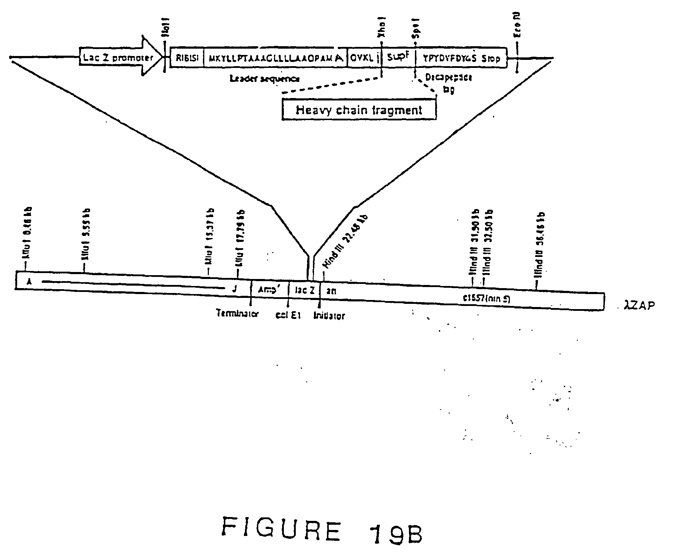

Individual lambda phage plaques were selected for DNA sequencing by screening for plaques that hybridized to polynucleotides contained in the synthetic DNA insert according to the methods described in Molecular Cloning: A Laboratory Manual, Maniatis et al., eds., Cold Spring Harbor, NY (1989). The selectable V_{L} expression vector is shown in Figure 19B.

### D. Construction of a Selectable V_{L} and V_{H}- Expression Vector

The V_{H}-Expression vector prepared in Example 17B is modified so that it does not contain any Xho I or Spe I restriction endonuclease sites. This modification of this vector is accomplished using a set of polynucleotides and methods similar to the methods described in Example 17B.

The V_{L}-Expression vector prepared in Example 17C is modified so that it does not contain any Sac I or Xba I restriction endonuclease sites. This modification of the V_{L}-Expression vector is accomplished using a set of polynucleotides and methods well known in the art and similar to the methods described in Example 17C. The modified V_{L}-Expression vector and the modified **V_{H}-**Expression vector are combined to produce a selectable V_{L} and V_{H} Expression vector. Briefly, the modified V_{H}-Expression vector is digested with the restriction endonucleases Eco RI and Hind III using the conditions recommended by the enzyme manufacturer and is digested with the restriction endonucleases Eco RI and Mlu I. The restriction endonuclease cleaved V_{H} and V_{L} Expression vectors are the ligated together using standard techniques to form the selectable V_{H} and V_{L} Expression vector shown in Figure 20.

The V_{H} and V_{L} Expression vector contains 2 suppressor tRNA genes, one is replaced by the V_{H} DNA homolog and the other is replaced by the V_{L} DNA homolog. Therefore, when the vector contains both a V_{H} and a V_{L} DNA homolog, the vector does not contain a suppressor tRNA gene allowing the V_{H} and V_{L} containing vector to produce phage plaques under the appropriate selection conditions.

### E. Inserting DNA Homologs into the Selectable DNA Expression Vectors

V_{H} coding and/or V_{L} coding DNA homologs prepared in Example 5 are inserted into the V_{H} and V_{L} expression vector, the V_{H} expression vector, or the V_{L} expression vector using the provided restriction endonuclease sites. The V_{H} coding DNA homologs are typically inserted into the provided Xho I and Spe I restriction endonuclease sites (Figure 20) using standard procedures. The V_{L} coding DNA homologs are typically inserted into the provided restriction endonuclease sites (Figure 20). Therefore, depending on the particular expression vector selected, the methods described herein produce an expression vector containing a V_{H} coding DNA homolog alone, a V_{L} coding DNA homolog alone, or a V_{H} and a V_{L} DNA homolog.

The V_{H} coding DNA homologs may be inserted into the expression vector first, followed by the V_{L} DNA homologs. Alternatively, the V_{L} coding homologs may be inserted first followed by the V_{H} coding homologs. Either insertion order allows the random recombination of a library of V_{H} coding DNA homologs with a library V_{L} coding DNA homologs. After the V_{H} homologs have been inserted into the V_{H} + V_{L} expression vector, the expression vector can be grown to produce more of the V_{H} containing expression vector. The V_{L} coding DNA homologs can then be inserted into the V_{H} and V_{L} expression vector. Any of these procedures will allow the production of a large combinatorial library.

### F. Selection of V_{H} and/or V_{L} DNA Homolog Containing Phage

A strong selection system is employed in order to reduce the number of expression vectors present in the final library that do not contain V_{H} and/or V_{L} DNA homologs. The selection system combines the dominant Lambda S gene mutation with the suppressor tRNA that is present in V_{H} and/or V_{L} expression vectors. When the suppressor tRNA is present in the expression vector, the mutant Lambda S protein is produced preventing the lysis of the infected cell and thereby preventing the formation of a phage plaque. When a DNA homolog replaces the suppressor tRNA, the expression vector can produce a phage plaque. In order to detect a V_{H} and/or V_{L} the V_{H} and/or V_{L} expression vector must produce a phage plaque because without plaque production there is not enough V_{H} and V_{L} expressed to detect using either immunologic or binding assays. Therefore, phages not containing a V_{H} and/or V_{L} will not be detected. To accomplish this selection, appropriate host bacterial cells containing the mutant S gene plasmid produced in Example 17A are infected with the desired expression vector library. Only the expression vectors without suppressor tRNA genes, the expression vectors containing DNA homologs, produce phage plaques.

### 18. Generation of a Large Combinatorial Library of the Immunoglobulin Repertoire in Phage

Vectors suitable for expression of V_{H}, V_{L}, F_{V} and Fab sequences are diagrammed in Figures 7 and 9. As previously discussed, the vectors were constructed by modification of Lambda Zap by inserting synthetic oligonucleotides into the multiple cloning site. The vectors were designed to be antisymmetric with respect to the Not I and EcoR I restriction sites which flank the cloning and expression sequences. As described below, this antisymmetry in the placement of restriction sites in a linear vector like bacteriophage is the essential feature of the system which allows a library expressing light chains to be combined with one expressing heavy chains to construct combinatorial Fab expression libraries. Lambda Zap II V_{L}II (Figure 9) is designed to serve as a cloning vector for light chain fragments and Lambda Zap II V_{H} (Figure 7) is designed to serve as a cloning vector for heavy chain sequences in the initial step of library construction. These vectors are engineered to efficiently clone the products of PCR amplification with specific restriction sites incorporated at each end.

### A. PCR Amplification of Antibody Fragments

The PCR amplification of mRNA isolated from spleen cells with oligonucleotides which incorporate restriction sites into the ends of the amplified product can be used to clone and express heavy chain sequences including Fd and kappa chain sequences. The oligonucleotide primers used for these amplifications are presented in Tables 1 and 2. The primers are analogous to those which have been successfully used in Example 5 for amplification of V_{H} sequences. The set of 5' primers for heavy chain amplification were identical to those previously used to amplify V_{H} and those for light chain amplification were chosen on similar principles, Sastry et al., Proc. Natl. Acad. Sci. USA, 8G: 5728 (1989) and Orlandi et al., Proc. Natl. Acad. Sci. USA, 8G:3833 (1989). The unique 3' primers of heavy (IgGl) and light (k) chain sequences were chosen to include the cysteines involved in heavy-light chain disulfide bond formation. At this stage no primer was constructed to amplify lambda light chains since they constitute only a small fraction of murine antibodies. In addition, Fv fragments have been constructed using a 3' primer which is complementary to the mRNA in the J (joining) region (amino acid 128) and a set of unique 5' primers which are complementary to the first strand cDNA in the conserved N-terminal region of the processed protein. Restriction endonuclease recognition sequences are incorporated into the primers to allow for the cloning of the amplified fragment into a lambda phage vector in a predetermined reading frame for expression.

### B. Library Construction

The construction of a combinatorial library was accomplished in two steps. In the first step; separate heavy and light chain libraries were constructed in Lambda Zap II V_{H} and Lambda Zap II V_{L} II respectively. In the second step, these two libraries were combined at the antisymmetric EcoRl sites present in each vector. This resulted in a library of clones each of which potentially co-expresses a heavy and a light chain. The actual combinations are random and do not necessarily reflect the combinations present in the B-cell population in the parent animal. Lambda Zap II V_{H} expression vector has been used to create a library of heavy chain sequences from DNA obtained by PCR amplification of mRNA isolated from the spleen of a 129 Gᵢₓ + mouse previously immunized with p-nitrophenyl phosphonamidate (NPN) antigen 1 according to formula I (Figure 13) conjugated to keyhole limpet hemocyanin (KLH). The NPN-KLH conjugate was prepared by admixture of 250 µl of a solution containing 2.5 mg of NPN according to formula 1 (Figure 13) in dimethylformamide with 750 µl of a solution containing 2 mg of KLH in 0.01 M sodium phosphate buffer (pH 7.2). The two solutions were admixed by slow addition of the NPN solution to the KLH solution while the KLH solution was being agitated by a rotating stirring bar.
Thereafter the admixture was maintained at 4* for 1 hour with the same agitation to allow conjugation to proceed. The conjugated NPN-KLH was isolated from the nonconjugated NPN and KLH by gel filtration through Sephadex G-25. The isolated NPN-KLH conjugate was used in mouse immunizations as described in Example 2.

The spleen mRNA resulting from the above immunizations was isolated and used to create a primary library of V_{H} gene sequences using the Lambda Zap II V_{H} expression vector. The primary library contains 1.3 x 10⁶ pfu and has been screened for the expression of the decapeptide tag to determine the percentage of clones expressing Fd sequences. The sequence for this peptide is only in frame for expression following the cloning of a Fd (or V_{H}) fragment into the vector. At least 80% of the clones in the library express Fd fragments based on immuno-detection of the decapeptide tag.

The light chain library was constructed in the same way as the heavy chain and shown to contain 2.5 x 10⁶ members. Plaque screening, using an anti-kappa chain antibody, indicated that 60% of the library contained expressed light chain inserts. This relatively small percentage of inserts probably resulted from incomplete dephosphorylation of vector after cleavage with Sac I and Xba I.

once obtained, the two libraries were used to construct a combinatorial library by crossing them at the EcoR I site. To accomplish the cross, DNA was first purified from each library. The light chain library was cleaved with MluI restriction endonuclease, the resulting 5' ends dephosphorylated and the product digested with EcoR I. This process cleaved the left arm of the vector into several pieces but the right arm containing the light chain sequences, remained intact. In a parallel fashion, the DNA of heavy chain library was cleaved with HindIII, dephosphorylated and cleaved with EcoR I, destroying the right arm but leaving the left arm containing the heavy chain sequences intact. The DNA's so prepared were then combined and ligated. After ligation only clones which resulted from combination of a right arm of light chain-containing clones and a left arm of heavy chain-containing clones reconstituted a viable phage. After ligation and packaging, 2.5 x 10⁷ clones were obtained. This is the combinatorial Fab expression library that was screened to identify clones having affinity for NPN. To determine the frequency the phage clones which co-express the light and heavy chain fragments, duplicate lifts of the light chain, heavy chain and combinatorial libraries were screened as above for light and heavy chain expression. In this study of approximately 500 recombinant phage approximately 60% coexpressed light and heavy chain proteins.

### C. Antigen Binding

All three libraries, the light chain, the heavy chain and Fab were screened to determine if they contained recombinant phage that expressed antibody fragments binding NPN. In a typical procedure 30,000 phage were plated and duplicate lifts with nitrocellulose screened for binding to NPN coupled to ¹²⁵I labeled BSA (Figure 15). Duplicate screens of 80,000 recombinant phage from the light chain library and a similar number from the heavy chain library did not identify any clones which bound the antigen. In contrast, the screen of a similar number of clones from the Fab expression library identified many phage plaques that bound NPN (Figure 15). This observation indicates that under conditions where many heavy chains in combination with light chains bind to antigen the same heavy or light chains alone do not. Therefore, in the case of NPN, it is believed that there are many heavy and light chains that only bind antigen when they are combined with specific light and heavy chains respectively.

To assess the ability to screen large numbers of clones and obtain a more quantitative estimate of the frequency of antigen binding clones in the combinatorial library, one million phage plaques were screened and approximately 100 clones which bound to antigen were identified. For six clones which were believed to bind NPN, a region of the plate containing the positive and approximately 20 surrounding bacteriophage plaques was "cored", replated, and screened with duplicate lifts (Figure 15). As expected, approximately one in twenty of the phage specifically bind to antigen. "Cores" of regions of the plated phage believed to be negative did not give positives on replating.

To determine the specificity of the antigen-antibody interaction, antigen binding was competed with free unlabeled antigen as shown in Figure 16. Competition studies showed that individual clones could be distinguished on the basis of antigen affinity. The concentration of free antigen required for complete inhibition of binding varied between 10-100 x 10⁹ M suggesting that the expressed Fab fragments had binding constants in the nanomolar range.

### D. Composition of the Clones and Their Expressed Products

In preparation for characterization of the protein products able to bind NPN as described in Example 18C, a plasmid containing the heavy and light chain genes was excised from the appropriate "cored" bacteriophage plaque using M13mp8 helper phage. Mapping of the excised plasmid demonstrated a restriction pattern consistent with incorporation of heavy and light chain sequences. The protein products of one of the clones was analyzed by ELISA and Western blotting to establish the composition of the NPN binding protein. A bacterial supernate following IPTG induction was concentrated and subjected to gel filtration. Fractions in the molecular weight range 40-60 kD were pooled, concentrated and subjected to a further gel filtration separation. As illustrated in Figure 17, ELISA analysis of the eluting fractions demonstrated that NPN binding was associated with a protein of molecular weight about 50 kD which immunological detection showed contained both heavy and light chains. A Western blot (not shown) of a concentrated bacterial supernate preparation under nonreducing conditions was developed with anti-decapeptide antibody. This revealed a protein band of molecular weight of 50 kD. Taken together these results are consistent with NPN binding being a function of Fab fragments in which heavy and light chains are covalently linked.

### E. Comparison of the Properties of the In Vivo Repertoire Versus the Phage Combinatorial Library

In this example a relatively restricted library was prepared because only a limited number of primers were used for PCR amplification of Fd sequences. The library is expected to contain only clones expressing kappa/gammal sequences. However, this is not an inherent limitation of the method since additional primers can be added to amplify any antibody class or subclass. Despite this restriction we were able to isolate a large number of antigen binding clones.

A central issue arising from this work is how a phage library prepared as described herein compares with the in vivo antibody repertoire in terms of size, characteristics of diversity, and ease of access.

The size of the mammalian antibody repertoire is difficult to judge but a figure of the order of 10⁶-10⁸ different antigen specificities is often quoted. With some of the reservations discussed below, a phage library of this size or larger can readily be constructed by a modification of the current method. In fact once an initial combinatorial library has been constructed, heavy and light chains can be shuffled to obtain libraries of exceptionally large numbers.

In principle, the diversity characteristics of the naive (unimmunized) in vivo repertoire and corresponding phage library are expected to be similar in that both involve a random combination of heavy and light chains. However, different factors will act to restrict the diversity expressed by an in vivo repertoire and phage library. For example a physiological modification such as tolerance will restrict the expression of certain antigenic specificities from the in vivo repertoire but these specificities may still appear in the phage library. On the other hand, bias in the cloning process may introduce restrictions into the diversity of the phage library. For example the representation of mRNA for sequences expressed by stimulated B-cells can be expected to predominate over those of unstimulated cells because of higher levels of expression. Different source tissues (e.g., peripheral blood, bone marrow or regional lymph nodes) and different PCR primers (e.g., ones expected to amplify different antibody classes) may result in libraries with different diversity characteristics.

Another difference between in vivo repertoire and phage library is that antibodies isolated from the former may have benefited from affinity maturation due to somatic mutations after combination of heavy and light chains whereas the latter randomly combines the matured heavy and light chains. Given a large enough phage library derived from a particular in vivo repertoire, the original matured heavy and light chains will be recombined. However, since one of the potential benefits of this new technology is to obviate the need for immunization by the generation of a single highly diverse "generic" phage library, it would be useful to have methods to optimize sequences to compensate for the absence of somatic mutation and clonal selection. Three procedures are made readily available through the methods of the present invention. First, saturation mutagenesis may be performed on the CDR's and the resulting Fabs can be assayed for increased function. Second, a heavy or a light chain of a clone which binds antigen can be recombined with the entire light or heavy chain libraries respectively in a procedure identical to the one used to construct the combinatorial library. Third, iterative cycles of the two above procedures can be performed to further optimize the affinity or catalytic properties of the immunoglobulin. It should be noted that the latter two procedures are not permitted in B-cell clonal selection which suggests that the methods described here may actually increase the ability to identify optimal sequences.

Access is the third area where it is of interest to compare the in vivo antibody repertoire and phage library. In practical terms the phage library is much easier to access. The screening methods allow one to survey at least 50,000 clones per plate so that 10⁶ antibodies can be readily examined in a day. This factor alone should encourage the replacement of hybridoma technology with the methods described here. The most powerful screening methods utilize selection which may be accomplished by incorporating selectable markers into the antigen such as leaving groups necessary for replication of auxotrophic bacterial strains or toxic substituents susceptible to catalytic inactivation. There are also further advantages related to the fact that the in vivo antibody repertoire can only be accessed via immunization which is a selection on the basis of binding affinity. The phage library is not similarly restricted. For example, the only general method to identify antibodies with catalytic properties has been by pre-selection on the basis of affinity of the antibody to a transition state analogue. No such restrictions apply to the in vitro library where catalysis can, in principle, be assayed directly. The ability to directly assay large numbers of antibodies for function may allow selection for catalysts in reactions where a mechanism is not well defined or synthesis of the transition state analog is difficult. Assaying for catalysis directly eliminates the bias of the screening procedure for reaction mechanisms pejorative to a synthetic analog and therefore simultaneous exploration of multiple reaction pathways for a given chemical transformation are possible.

The methods disclosed herein describe generation of Fab fragments which are clearly different in a number of important respects from intact (whole) antibodies. There is undoubtedly a loss of affinity in having monovalent Fab antigen binders but this can be compensated by selection of suitably tight binders. For a number of applications such as diagnostics and biosensors it may be preferable to have monovalent Fab fragments. For applications requiring Fc effector functions, the technology already exists for extending the heavy chain gene and expressing the glycosylated whole antibody in mammalian cells.

The ideas presented here address the bottle neck in the identification and evaluation of antibodies. It is now possible to construct and screen at least three orders of magnitude more clones with mono-specificity than previously possible. The potential applications of the method should span basic research and applied sciences.

## Claims

1. A method of producing a receptor-coding nucleic acid coding for a ligand binding antibody heavy chain or light chain variable region having an affinity for a target ligand of greater than 10⁸ M⁻¹, which method comprises:
(a) synthesizing a conserved V_{H} or V_{L} receptor-coding gene library containing at least 10³ different V_{H} or V_{L} receptor-coding DNA homologs by:
(i) separating the strands of a repertoire of conserved V_{H} or V_{L} receptor-coding genes, said repertoire comprising double-stranded nucleic acids each containing a V_{H} or V_{L} receptor-coding strand annealed to a complementary strand;
(ii) treating said separated strands, under conditions suitable for polymerase chain reaction amplification, with first and second polynucleotide synthesis primers, each of said first primers having a nucleotide sequence capable of hybridizing to a sequence conserved among said V_{H} or V_{L} receptor-coding strands, and each of said second primers having a nucleotide sequence capable of hybridizing to a sequence conserved among said complementary strands, said primers being capable of priming the amplification of a plurality of different V_{H} or V_{L} receptor-coding DNA homologs from said V_{H} or V_{L} receptor-coding gene repertoire, said treating producing said conserved receptor-coding gene library; and
(b) segregating from said library a receptor-coding nucleic acid coding for a ligand binding antibody heavy chain or light chain variable region having an affinity for a target ligand of greater than 10⁸ M⁻¹.

2. The method of claim 1 wherein said conserved receptor-coding nucleic acid codes for a V_{H}, said conserved receptor-coding genes are V_{H}-coding genes, and said receptor-coding DNA homologs are V_{H}-coding DNA homologs.

3. The method of claim 2 wherein said first polynucleotide synthesis primer hybridizes to an immunoglobulin J_{H} or framework region nucleotide sequence.

4. The method of claim 2 wherein said second polynucleotide synthesis primer hybridizes to a framework, leader or promoter region of a V_{H} immunoglobulin gene.

5. The method of claim 1 wherein said conserved receptor-coding nucleic acid codes for a V_{L}, said conserved receptor-coding genes are V_{L}-coding genes, and said receptor-coding DNA homologs are V_{L}-coding DNA homologs.

6. The method of claim 5 wherein said first polynucleotide synthesis primer hybridizes to an immunoglobulin J_{L} or framework region nucleotide sequence.

7. The method of claim 5 wherein said second polynucleotide synthesis primer hybridizes to a framework, leader or promoter region of a V_{L} immunoglobulin gene.

8. The method of claim 2 or claim 5 wherein said segregating comprises:
(a) operatively linking for expression each of a plurality of said different V_{H}- or V_{L}-coding DNA homologs to an expression vector, thereby forming a plurality of different V_{H} or V_{L} expression vectors;
(b) transforming a population of host cells compatible with said expression vector with a plurality of said different V_{H} or V_{L} expression vectors to produce a transformed population of host cells whose members contain said V_{H} or V_{L} expression vectors;
(c) culturing said transformed population under conditions for expressing the receptors coded for by said V_{H}- or V_{L}-coding DNA homologs;
(d) assaying the members of said transformed population for expression of a receptor capable of binding a preselected ligand, thereby identifying transformants containing said V_{H}- or V_{L}-coding DNA homolog; and
(e) segregating an identified transformant of step (d) from said population, thereby producing said conserved V_{H}- or V_{L}-coding nucleic acid.

9. The method of claim 8 wherein said receptor contains a preselected epitope coded for by either of said primers or said expression vector.

10. The method of claim 8 wherein said expression vector is an episome, phage or plasmid comprised of a selectable marker gene.

11. The method of claim 1 wherein said synthesizing is performed using a plurality of different first primers.

12. The method of claim 8 wherein said expression vector molecules are linear DNA expression vector molecules.

13. The method of claim 12 wherein said linear DNA expression vector molecules are phage vector molecules.

14. The method of claim 13 wherein said phage vector molecules are Lambda Zap II V_{H} molecules, as depicted in Figure 7.

15. The method of claim 3 or claim 6 wherein said first polynucleotide synthesis primer encodes a predetermined restriction endonuclease recognition site.

16. The method of claim 4 or claim 7 wherein said second polynucleotide synthesis primer encodes a predetermined restriction endonuclease recognition site.

17. The method of any one of claims 1 to 16 wherein a plurality of the V_{H} or V_{L} receptor-coding nucleic acids code for conserved sequences from FR1 and FR4 shared by the encoded variable regions.

18. The method of any one of claims 1 to 17 wherein the library is provided by a population of B cells.

19. The method of claim 18 wherein the B cells are rearranged B cells.

20. The method of claim 18 or claim 19 wherein the population of B cells is sourced from an animal that has been immunized with the target ligand.

21. The method of claim 18 or claim 19 wherein the population of B cells is sourced from an animal that has been repeatedly immunized with the target ligand.

22. The method of claim 18 or claim 19 wherein the population of B cells is sourced from an animal that has not recently been immunized with the target ligand.

23. The method of any one of claims 1 to 22 wherein the library is provided by a population of cells from different individuals.

24. The method of any one of claims 1 to 23 wherein the library is provided as an expression library.

25. A method of producing a ligand binding antibody heavy chain or light chain variable region having an affinity for a target ligand of greater than 10⁸ M⁻¹ comprising:
(a) operatively linking for expression a gene isolated according to claim 1 or 8 to a suitable expression vector to form a V_{H}- or V_{L}-expression vector;
(b) transforming a host cell compatible with said expression vector to produce a transformant;
(c) culturing said transformant under conditions for expressing the ligand binding antibody heavy chain or light chain variable region coded for by said V_{H}- or V_{L}-coding DNA homolog, thereby producing said ligand binding antibody heavy chain or light chain variable region in said culture; and
(d) recovering from said culture said ligand binding antibody heavy chain or light chain variable region.

26. A method according to claim 25 wherein the ligand binding antibody heavy chain or light chain variable region is mutated to improve binding affinity.

27. A method according to claim 25 wherein the sequence of the ligand binding antibody heavy chain or light chain variable region is further modified.

28. A method according to claim 27 wherein a synthetic polynucleotide is employed to vary one or more amino acids in a hypervariable region.

29. A method according to claim 25 wherein the sequence of the ligand binding antibody heavy chain or light chain variable region is further modified to vary its idiotype.

30. A ligand binding antibody heavy chain or light chain variable region having an affinity for a target ligand of greater than 10⁸ M⁻¹ produced by the method of any one of claims 25 to 29.

31. A ligand binding antibody heavy chain or light chain variable region according to claim 30 comprising a C-terminal cysteine.

32. A ligand binding antibody heavy chain or light chain variable region according to claim 30 or claim 31 wherein the region consists of fewer than 120 amino acids and greater than 60 amino acids.

33. A ligand binding antibody heavy chain or light chain variable region according to any one of claims 30 to 32 wherein the region comprises an intrachain disulfide bond connecting FR1 and FR3.

34. A ligand binding polypeptide comprising at least one antibody variable region as defined in any one of claims 30 to 33, each variable region having a ligand binding site.

35. A ligand binding polypeptide according to claim 34 comprising substantially all, or a portion of the heavy or light chain constant region.

36. A complex of a ligand binding antibody heavy chain or light chain variable region according to any one of claims 30 to 33 with the target ligand.

37. A multimer of ligand binding variable regions according to any one of claims 30 to 33.

38. The multimer of claim 37 wherein the multimer is a homo-multimer.

39. A method according to any one of claims 1 to 29, a ligand binding antibody variable region according to any one of claims 30-33, a ligand binding polypeptide according to claim 34 or claim 35, a complex according to claim 36 or a multimer according to claim 37 or claim 38 wherein the first or second primer "referred to in claims 1-8" is selected from a primer as set out in Table 1 or Table 2.

## Patentansprüche

1. Verfahren zur Herstellung einer Rezeptor-kodierenden Nucleinsäure, die für eine Liganden-bindende variable Region einer Antikörper-Schwer- oder -Leichtkette kodiert, die eine Affinität für einen Target-Liganden aufweist, die größer als 10⁸ M⁻¹ ist, wobei das Verfahren Folgendes umfasst:
(a) Synthetisieren einer konservierten V_{H}- oder V_{L}-Rezeptor-kodierenden Genbibliothek, die zumindest 10³ unterschiedliche V_{H}- oder V_{L}-Rezeptor-kodierende DNA-Homologe enthält, mittels:
(i) Trennen der Stränge eines Repertoires an konservierten V_{H}- oder V_{L}-Rezeptor-kodierenden Genen, wobei das Repertoire doppelsträngige Nucleinsäuren umfasst, die jeweils einen V_{H}- oder V_{L}-Rezeptor-kodierenden Strang enthalten, die an einen komplementären Strang anelliert sind;
(ii) Behandeln der getrennten Stränge unter Bedingungen, die zur Polymerasekettenreaktionsamplifikation geeignet sind, mit ersten und zweiten Polynucleotid-Syntheseprimern, wobei jeder der ersten Primer eine Nucleotidsequenz aufweist, die in der Lage ist, an eine Sequenz zu hybridisieren, die unter den V_{H}- oder V_{L}-Rezeptor-kodierenden Strängen konserviert ist, und jeder der zweiten Primer eine Nucleotidsequenz aufweist, die in der Lage ist, an eine Sequenz zu hybridisieren, die unter den komplementären Strängen konserviert ist, wobei die Primer in der Lage sind, die Amplifikation einer Vielzahl an unterschiedlichen V_{H}- oder V_{L}-Rezeptor-kodierenden DNA-Homologen aus dem V_{H}- oder V_{L}-Rezeptor-kodierenden Genrepertoire zu primen, wobei die Behandlung die konservierte Rezeptor-kodierende Genbibliothek produziert;
und
(b) Trennen einer Rezeptor-kodierenden Nucleinsäure, die für eine Liganden-bindende variable Region einer Antikörper-Schwer- oder -Leichtkette mit einer Affinität für einen Target-Liganden, die größer als 10⁸ M⁻¹ ist, kodiert, von der Bibliothek.

2. Verfahren nach Anspruch 1, worin die konservierte Rezeptor-kodierende Nucleinsäure für ein V_{H} kodiert, wobei die konservierten Rezeptor-kodierenden Gene V_{H}-kodierende Gene sind und die Rezeptor-kodierenden DNA-Homologe V_{H}-kodierende DNA-Homologe sind.

3. Verfahren nach Anspruch 2, worin der erste Polynucleotidsyntheseprimer an ein Immunglobulin J_{H} oder an eine Gerüstregion-Nucleotidsequenz hybridisiert.

4. Verfahren nach Anspruch 2, worin der zweite Polynucleotidsyntheseprimer an eine Gerüst-, Leader- oder Promotorregion eines V_{H}-Immunglobulingens hybridisiert.

5. Verfahren nach Anspruch 1, worin die konservierte Rezeptor-kodierende Nucleinsäure für ein V_{L} kodiert, wobei die konservierten Rezeptor-kodierenden Gene V_{L}-kodierenden Gene sind und die Rezeptor-kodierenden DNA-Homologe V_{L}-kodierende DNA-Homologe sind.

6. Verfahren nach Anspruch 5, worin der erste Polynucleotidsyntheseprimer an eine Immunglobulin-J_{L}- oder Gerüstregion-Nucleotidsequenz hybridisiert.

7. Verfahren nach Anspruch 5, worin der zweite Polynucleotidsyntheseprimer an eine Gerüst-, Leader- oder Promotorregion eines V_{L}-Immunglobulingens hybridisiert.

8. Verfahren nach Anspruch 2 oder Anspruch 5, worin das Trennen Folgendes umfasst:
(a) operatives Binden eines jeden von einer Vielzahl der unterschiedlichen V_{H}- oder V_{L}-kodierenden DNA-Homologe an einen Expressionsvektor zu Expression, wodurch eine Vielzahl an unterschiedlichen V_{H}- oder V_{L}-Expressionsvektoren gebildet wird;
(b) Transformieren einer Population an Wirtszellen, die mit dem Expressionsvektor verträglich sind, mit einer Vielzahl an unterschiedlichen V_{H}- oder V_{L}-Expressionsvektoren, um eine transformierte Population an Wirtszellen herzustellen, deren Mitglieder die V_{H}- oder V_{L}-Expressionsvektoren enthalten;
(c) Züchten der transformierten Population unter Bedingungen zur Expression der Rezeptoren, für welche die V_{H}- oder V_{L}-kodierenden DNA-Homologe kodieren;
(d) Testen der Mitglieder der transformierten Population zur Expression eines Rezeptors, der in der Lage ist, einen vorselektierten Liganden zu binden, wodurch Transformanten identifiziert werden, die das V_{H}- oder V_{L}-kodierenden DNA-Homolog enthalten; und
(e) Trennen eines identifizierten Transformanten von Schritt (d) von der Population, wodurch die konservierte V_{H}- oder V_{L}- kodierende Nucleinsäure hergestellt wird.

9. Verfahren nach Anspruch 8, worin der Rezeptor ein vorselektiertes Epitop enthält, für das entweder einer der Primer oder der Expressionvektor kodiert.

10. Verfahren nach Anspruch 8, worin der Expressionsvektor ein Episom, ein Phage oder ein Plasmid ist, die aus einem selektierbaren Markergen bestehen.

11. Verfahren nach Anspruch 1, worin das Synthetisieren unter Verwendung einer Vielzahl an unterschiedlichen ersten Primern durchgeführt wird.

12. Verfahren nach Anspruch 8, worin die Expressionsvektormoleküle lineare DNA-Expressionsvektormoleküle sind.

13. Verfahren nach Anspruch 12, worin die linearen DNA-Expressionsvektormoleküle Phagen-Vektormoleküle sind.

14. Verfahren nach Anspruch 13, worin die Phagenvektormoleküle Lambda-Zap-II-V_{H}-Moleküle, wie in Fig. 7 dargestellt, sind.

15. Verfahren nach Anspruch 3 oder nach Anspruch 6, worin der erste Polynucleotidsyntheseprimer für eine vorbestimmte Restriktionsendonuklease-Erkennungsstelle kodiert.

16. Verfahren nach Anspruch 4 oder nach Anspruch 7, worin der zweite Polynucleotidsyntheseprimer für eine vorbestimmte Restriktionsendonuclease-Erkennungsstelle kodiert.

17. Verfahren nach einem der Ansprüche 1 bis 16, worin eine Vielzahl der V_{H}- oder V_{L}-Rezeptor-kodierenden Nucleinsäuren für konservierte Sequenzen aus FR1 und FR4 kodieren, die den kodierten variablen Regionen gemeinsam sind.

18. Verfahren nach einem der Ansprüche 1 bis 17, worin die Bibliothek von einer Population von B-Zellen bereitgestellt ist.

19. Verfahren nach Anspruch 18, worin die B-Zellen umgeordnete B-Zellen sind.

20. Verfahren nach Anspruch 18 oder nach Anspruch 19, worin die Population von B-Zellen aus einem Tier stammt, das mit dem Target-Liganden immunisiert wurde.

21. Verfahren nach Anspruch 18 oder nach Anspruch 19, worin die Population von B-Zellen aus einem Tier stammt, das wiederholt mit dem Target-Liganden immunisiert wurde.

22. Verfahren nach Anspruch 18 oder Anspruch 19, worin die Population von B-Zellen aus einem Tier stammt, das in letzter Zeit nicht mit dem Target-Liganden immunisiert wurde.

23. Verfahren nach einem der Ansprüche 1 bis 22, worin die Bibliothek von einer Population von Zellen von unterschiedlichen Individuen bereitgestellt ist.

24. Verfahren nach einem der Ansprüche 1 bis 23, worin die Bibliothek als eine Expressionsbibliothek bereitgestellt ist.

25. Verfahren zum Herstellen einer Liganden-bindenden variablen Region einer Antikörper-Schwer- oder -Leichtkette, die eine Affinität für einen Target-Liganden aufweist, die größer ist als 10⁸ M⁻¹, umfassend:
(a) operatives Binden eines Gens, das nach Anspruch 1 oder Anspruch 8 isoliert worden ist, an einen geeigneten Expressionsvektor zur Expression, um einen V_{H}- oder V_{L}-Expressionsvektor zu bilden;
(b) Transformieren einer Wirtszelle, die mit dem Expressionsvektor kompatibel ist, um einen Transformanten zu produzieren;
(c) Züchten des Transformanten unter Bedingungen zur Expression der Liganden-bindenden variablen Region einer Antikörper-Schwer- oder -Leichtkette, für welche das V_{H}- oder V_{L}-kodierende DNA-Homolog kodiert, wodurch die Liganden-bindende variable Region einer Antikörper-Schwer- oder -Leichtkette in der Kultur hergestellt wird; und
(d) Gewinnen der Liganden-bindenden variablen Region einer Antikörper-Schwer- oder -Leichtkette aus der Kultur.

26. Verfahren nach Anspruch 25, worin die Liganden-bindende variable Region einer Antikörper-Schwer- oder -Leichtkette mutiert ist, um Bindungsaffinität zu verbessern.

27. Verfahren nach Anspruch 25, worin die Sequenz der Liganden-bindenden variablen Region einer Antikörper-Schwer- oder -Leichtkette weiter modifiziert ist.

28. Verfahren nach Anspruch 27, worin ein synthetisches Polynucleotid verwendet wird, um eine oder mehrere Aminosäuren in einer hypervariable Region zu variieren.

29. Verfahren nach Anspruch 25, worin die Sequenz der Liganden-bindenden variablen Region einer Antikörper-Schwer- oder -Leichtkette weiter modifiziert ist, um ihren Idiotyp zu variieren.

30. Liganden-bindende variable Region einer Antikörper-Schwer- oder -Leichtkette mit einer Affinität für einen Target-Liganden, die größer ist als 10⁸ M⁻¹, die mit Hilfe des Verfahrens nach einem der Ansprüche 25 bis 29 hergestellt wurde.

31. Liganden-bindende variable Region einer Antikörper-Schwer- oder -Leichtkette nach Anspruch 30, die ein C-terminales Cystein umfasst.

32. Liganden-bindende variable Region einer Antikörper-Schwer- oder -Leichtkette nach Anspruch 30 oder Anspruch 31, worin die Region aus weniger als 120 Aminosäuren besteht und größer ist als 60 Aminosäuren.

33. Liganden-bindende variable Region einer Antikörper-Schwer- oder -Leichtkette nach einem der Ansprüche 30 bis 32, worin die Region eine Intraketten-Disulfidbrücke umfasst, die FR1 und FR3 verbindet.

34. Liganden-bindendes Polypeptid, das zumindest eine variable Region eines Antikörpers, wie in einem der Ansprüche 30 bis 33 beschreiben, umfasst, wobei jede variable Region eine Liganden-bindende Stelle aufweist.

35. Liganden-bindendes Polypeptid nach Anspruch 34, das im Wesentlichen die gesamte oder einen Teil der konstanten Region der Schwer- oder Leichtkette umfasst.

36. Komplex einer Liganden-bindenden variablen Region einer Antikörper-Schwer- oder -Leichtkette nach einem der Ansprüche 30 bis 33 mit dem Target-Liganden.

37. Multimer der Liganden-bindenden variablen Regionen nach einem der Ansprüche 30 bis 33.

38. Multimer nach Anspruch 37, worin das Multimer ein Homo-Multimer ist.

39. Verfahren nach einem der Ansprüche 1 bis 29, variable Region eines Liganden-bindenden Antikörpers nach einem der Ansprüche 30-33, Liganden-bindendes Polypeptid nach Anspruch 34 oder Anspruch 35, Komplex nach Anspruch 36 oder Multimer nach Anspruch 37 oder Anspruch 38, worin der erste oder zweite Primer, auf die in den Ansprüchen 1-8 verwiesen wird, aus einem Primer, wie in Tabelle 1 oder Tabelle 2 dargestellt, ausgewählt ist.

## Revendications

1. Procédé pour produire un acide nucléique codant pour un récepteur, l'acide nucléique codant pour une région variable de chaîne lourde ou de chaîne légère d'un anticorps se liant à un ligand, ayant une affinité pour un ligand cible supérieure à 10⁸ M⁻¹, lequel procédé consiste à:
(a) synthétiser un gène conservé codant pour un récepteur V_{H} ou V_{L} contenant au moins 10³ homologues d'ADN différents codant pour un récepteur V_{H} ou V_{L}:
(i) en séparant les brins d'un répertoire de gènes conservés codant pour un récepteur V_{H} ou V_{L}, ledit répertoire comprenant des acides nucléiques double brin contenant chacun un brin codant pour un récepteur V_{H} ou V_{L} annelé à un brin complémentaire;
(ii) en traitant lesdits brins séparés, dans des conditions appropriées pour une amplification par réaction en chaîne par polymérase, avec des premières et deuxièmes amorces de synthèse d'un polynucléotide, chacune desdites premières amorces ayant une séquence de nucléotides capable de s'hybrider à une séquence conservée parmi lesdits brins codant un récepteur V_{H} ou V_{L}, et chacune desdites deuxièmes amorces ayant une séquence de nucléotides capable de s'hybrider à une séquence conservée parmi lesdits brins complémentaires, lesdites amorces étant capables d'amorcer l'amplification d'une pluralité d'homologues d'ADN différents codant pour un récepteur V_{H} ou V_{L} issus dudit répertoire de gènes codant pour un récepteur V_{H} ou V_{L}, ledit traitement produisant ladite banque de gènes conservés codant pour un récepteur; et
(b) isoler à partir de ladite banque un acide nucléique codant pour un récepteur, l'acide nucléique codant pour une région variable de chaîne lourde ou de chaîne légère d'un anticorps se liant à un ligand, et ayant une affinité pour un ligand cible supérieure à 10⁸ M⁻¹.

2. Procédé selon la revendication 1, dans lequel ledit acide nucléique conservé codant pour un récepteur code pour une V_{H}, lesdits gènes conservés codant pour un récepteur étant des gènes codant pour une V_{H}, et lesdits homologues d'ADN codant pour un récepteur étant des homologues d'ADN codant pour une V_{H}.

3. Procédé selon la revendication 2, dans lequel ladite première amorce de synthèse d'un polynucléotide s'hybride à une séquence de nucléotides d'une région J_{H} ou de charpente d'une immunoglobuline.

4. Procédé selon la revendication 2, dans lequel ladite deuxième amorce de synthèse d'un polynucléotide s'hybride à une région charpente, leader ou promotrice d'un gène d'immunoglobuline V_{H}.

5. Procédé selon la revendication 1, dans lequel ledit acide nucléique conservé codant pour un récepteur code pour une V_{L}, lesdits gènes conservés codant pour un récepteur étant des gènes codant pour une V_{L}, et lesdits homologues d'ADN codant pour un récepteur étant des homologues d'ADN codant pour une V_{L}.

6. Procédé selon la revendication 5, dans lequel ladite première amorce de synthèse d'un polynucléotide s'hybride à une séquence de nucléotides d'une région J_{L} ou de charpente d'une immunoglobuline.

7. Procédé selon la revendication 5, dans lequel ladite deuxième amorce de synthèse d'un polynucléotide s'hybride à une région charpente, leader ou promotrice d'un gène d'immunoglobuline V_{L}.

8. Procédé selon la revendication 2 ou la revendication 5, dans lequel chaque isolation consiste à:
(a) lier de manière fonctionnelle pour obtenir une expression chacun d'une pluralité desdits homologues d'ADN différents codant pour une V_{H} ou une V_{L} à un vecteur d'expression, ce qui forme ainsi une pluralité de vecteurs d'expression d'une V_{H} ou d'une V_{L} différents;
(b) transformer une population de cellules hôtes, compatibles avec ledit vecteur d'expression, avec une pluralité desdits vecteurs d'expression d'une V_{H} ou d'une V_{L} différents afin de produire une population transformée de cellules hôtes dont les membres contiennent lesdits vecteurs d'expression d'une V_{H} ou d'une V_{L};
(c) cultiver ladite population transformée dans des conditions permettant d'exprimer les récepteurs codés par lesdits homologues d'ADN codant pour une V_{H} ou une V_{L};
(d) analyser les membres de ladite population transformée pour identifier l'expression d'un récepteur capable de se lier à un ligand présélectionné, ce qui permet ainsi d'identifier des transformants contenant ledit homologue d'ADN codant pour une V_{H} ou une V_{L}; et
(e) isoler un transformant identifié à l'étape (d) à partir de ladite population, ce qui permet ainsi de produire ledit acide nucléique conservé codant pour une V_{H} ou une V_{L}.

9. Procédé selon la revendication 8, dans lequel ledit récepteur contient un épitope présélectionné qui est codé par l'une quelconque desdites amorces ou par ledit vecteur d'expression.

10. Procédé selon la revendication 8, dans lequel ledit vecteur d'expression est un épisome, un phage ou un plasmide constitué d'un gène marqueur de sélection.

11. Procédé selon la revendication 1, dans lequel ladite synthèse est réalisée en utilisant une pluralité de premières amorces différentes.

12. Procédé selon la revendication 8, dans lequel lesdites molécules de vecteur d'expression sont des molécules de vecteur d'expression d'ADN linéaire.

13. Procédé selon la revendication 12, dans lequel lesdites molécules de vecteur d'expression d'ADN linéaire sont des molécules de vecteur phage.

14. Procédé selon la revendication 13, dans lequel lesdites molécules de vecteur phage sont des molécules Lambda Zap II V_{H}, tel que représenté sur la Figure 7.

15. Procédé selon la revendication 3 ou la revendication 6, dans lequel ladite première amorce de synthèse d'un polynucléotide code pour un site de reconnaissance d'une endonucléase de restriction prédéterminé.

16. Procédé selon la revendication 4 ou la revendication 7, dans lequel ladite deuxième amorce de synthèse d'un polynucléotide code pour un site de reconnaissance d'une endonucléase de restriction prédéterminé.

17. Procédé selon l'une quelconque des revendications 1 à 16, dans lequel une pluralité des acides nucléiques codant pour un récepteur V_{H} ou V_{L} code pour des séquences conservées issues de FR1 et de FR4 partagées par les régions variables codées.

18. Procédé selon l'une quelconque des revendications 1 à 17, dans lequel la banque est fournie par une population de cellules B.

19. Procédé selon la revendication 18, dans lequel les cellules B sont des cellules B réarrangées.

20. Procédé selon la revendication 18 ou la revendication 19, dans lequel la population de cellules B provient d'un animal qui a été immunisé avec le ligand cible.

21. Procédé selon la revendication 18 ou la revendication 19, dans lequel la population de cellules B provient d'un animal qui a été immunisé de façon répétée avec le ligand cible.

22. Procédé selon la revendication 18 ou la revendication 19, dans lequel la population de cellules B provient d'un animal qui n'a pas été récemment immunisé avec le ligand cible.

23. Procédé selon l'une quelconque des revendications 1 à 22, dans lequel la banque est fournie par une population de cellules issues d'individus différents.

24. Procédé selon l'une quelconque des revendications 1 à 23, dans lequel la banque est fournie en tant que banque d'expression.

25. Procédé pour produire une région variable de chaîne lourde ou de chaîne légère d'un anticorps se liant à un ligand, ayant une affinité pour un ligand cible supérieure à 10⁸ M⁻¹, consistant à:
(a) lier de manière fonctionnelle pour obtenir une expression un gène isolé selon la revendication 1 ou 8 à un vecteur d'expression approprié pour former un vecteur d'expression d'une V_{H} ou d'une V_{L};
(b) transformer une cellule hôte compatible avec ledit vecteur d'expression afin de produire un transformant;
(c) cultiver ledit transformant dans des conditions permettant d'exprimer la région variable de chaîne lourde ou de chaîne légère d'un anticorps se liant à un ligand, codée par ledit homologue d'ADN codant pour une V_{H} ou une V_{L}, ce qui permet ainsi de produire ladite région variable de chaîne lourde ou de chaîne légère d'un anticorps se liant à un ligand dans ladite culture; et
(d) récupérer à partir de ladite culture ladite région variable de chaîne lourde ou de chaîne légère d'un anticorps se liant à un ligand.

26. Procédé selon la revendication 25, dans lequel la région variable de chaîne lourde ou de chaîne légère d'un anticorps se liant à un ligand est mutée pour améliorer l'affinité de liaison.

27. Procédé selon la revendication 25, dans lequel la séquence de la région variable de chaîne lourde ou de chaîne légère d'un anticorps se liant à un ligand est en outre modifiée.

28. Procédé selon la revendication 27, dans lequel un polynucléotide synthétique est employé pour faire varier un ou plusieurs acides aminés dans une région hypervariable.

29. Procédé selon la revendication 25, dans lequel la séquence de la région variable de chaîne lourde ou de chaîne légère d'un anticorps se liant à un ligand est en outre modifiée pour faire varier son idiotype.

30. Région variable de chaîne lourde ou de chaîne légère d'un anticorps se liant à un ligand ayant une affinité pour un ligand cible supérieure à 10⁸ M⁻¹, produite par le procédé selon l'une quelconque des revendications 25 à 29.

31. Région variable de chaîne lourde ou de chaîne légère d'un anticorps se liant à un ligand selon la revendication 30, comprenant une cystéine C-terminale.

32. Région variable de chaîne lourde ou de chaîne légère d'un anticorps se liant à un ligand selon la revendication 30 ou la revendication 31, où la région consiste en moins de 120 acides aminés et en plus de 60 acides aminés.

33. Région variable de chaîne lourde ou de chaîne légère d'un anticorps se liant à un ligand selon l'une quelconque des revendications 30 à 32, où la région comprend une liaison disulfure intra-chaîne connectant FR1 et FR3.

34. Polypeptide se liant à un ligand comprenant au moins une région variable d'anticorps telle que définie dans l'une quelconque des revendications 30 à 33, chaque région variable ayant un site de liaison à un ligand.

35. Polypeptide se liant à un ligand selon la revendication 34, comprenant essentiellement l'intégralité ou une partie de la région constante de chaîne lourde ou légère.

36. Complexe entre une région variable de chaîne lourde ou de chaîne légère d'un anticorps se liant à un ligand selon l'une quelconque des revendications 30 à 33 et le ligand cible.

37. Multimère de régions variables se liant à un ligand selon l'une quelconque des revendications 30 à 33.

38. Multimère selon la revendication 37, où le multimère est un homo-multimère.

39. Procédé selon l'une quelconque des revendications 1 à 29, région variable de chaîne lourde ou de chaîne légère d'un anticorps se liant à un ligand selon l'une quelconque des revendications 30-33, polypeptide se liant à un ligand selon la revendication 34 ou la revendication 35, complexe selon la revendication 36 ou multimère selon la revendication 37 ou la revendication 38, où la première et la deuxième amorce citées dans les revendications 1-8 sont sélectionnées parmi une amorce telle que définie dans le Tableau 1 ou le Tableau 2.
